# EUROPEAN PATENT SPECIFICATION

(11) **EP 1 175 411 B1**
(45) Date of publication and mention of the grant of the patent: **20.02.2008**
(21) Application number: 00930266.2
(22) Date of filing: 01.05.2000
(51) Int. Cl.: C07D 265/18, C07D 413/04, A61K 31/536, A61P 15/00, A61P 35/00

(54) **CYCLOTHIOCARBAMATE DERIVATIVES AS PROGESTERONE RECEPTOR MODULATORS**
CYCLOTHIOCARBAMATDERIVATE ALS PROGESTERON-REZEPTORMODULATOREN
DERIVES DE CYCLOTHIOCARBAMATE TENANT LIEU DE MODULATEURS DU RECEPTEUR DE PROGESTERONE

(30) Priority: 04.05.1999 US 183013 P; 19.04.2000 US 552354
(43) Date of publication of application: 30.01.2002
(73) Proprietor: Wyeth, Madison, New Jersey 07940 (US)
(72) Inventor: ZHANG, Puwen, Audubon, PA 19403 (US); FENSOME, Andrew, Wayne, PA 19087 (US); TEREFENKO, Eugene, A., Quakertown, PA 18951 (US); ZHI, Lin, San Diego, CA 92129 (US); JONES, Todd, K., Solana Beach, CA 92075 (US); COLLINS, MARK, A., Norristown, PA 19401 (US); TEGLEY, Christopher, M., Thousand Oaks, CA 91360 (US); WROBEL, Jay, E., Lawrenceville, NJ 08648 (US); EDWARDS, James, P., San Diego, CA 92129 (US)
(74) Representative: Manaton, Ross Timothy
(86) International application number: PCT/US2000/011749
(87) International publication number: WO 2000/066570

(56) References cited:
- EP-A- 0 510 235
- WO-A-95/20389
- WO-A-96/19458
- WO-A-98/14436

## Description

### Field of the Invention

This invention relates to compounds which are agonists of the progesterone receptor, their preparation and utility. The invention provides for the use of these compounds in the preparation of a medicament for use as a contraceptive, for use in the treatment of dysfunctional bleeding, uterine leiomyomata, endometriosis, polycystic ovary syndrome and fibroids and of carcinomas and adenocarcinomas of the endometrium, ovary, breast, colon, and prostate, and also for use in hormone replacement therapy.

### Background of the Invention

Intracellular receptors (IR) form a class of structurally related gene regulators known as "ligand dependent transcription factors" (R. M. Evans, Science, 240, 889, 1988). The steroid receptor family is a subset of the IR family, including progesterone receptor (PR), estrogen receptor (ER), androgen receptor (AR), glucocorticoid receptor (GR), and mineralocorticoid receptor (MR).

The natural hormone, or ligand, for the PR is the steroid progesterone, but synthetic compounds, such as medroxyprogesterone acetate or levonorgestrel, have been made which also serve as ligands. Once a ligand is present in the fluid surrounding a cell, it passes through the membrane via passive diffusion, and binds to the IR to create a receptor/ligand complex. This complex binds to specific gene promoters present in the cell's DNA. Once bound to the DNA the complex modulates the production of mRNA and protein encoded by that gene.

A compound that binds to an IR and mimics the action of the natural hormone is termed an agonist, whilst a compound which inhibits the effect of the hormone is an antagonist.

PR agonists (natural and synthetic) are known to play an important role in the health of women. PR agonists are used in birth control formulations, typically in the presence of an ER agonist, alternatively they may be used in conjunction with a PR antagonist. ER agonists are used to treat the symptoms of menopause, but have been associated with a proliferative effect on the uterus which can lead to an increased risk of uterine cancers. Co-administration of a PR agonist reduces/ablates that risk.

The compounds of this invention have been shown to act as competitive inhibitors of progesterone binding to the PR and act as agonists in functional models, both *in-vitro* and *in-vivo.* These compounds may be used for contraception, in the treatment of fibroids, endometriosis, breast, uterine, ovarian and prostate cancer, and post menopausal hormone replacement therapy.

Jones, *et al* describe in U.S. Patent No. 5,688,810 the PR antagonist dihydroquinoline **1.**

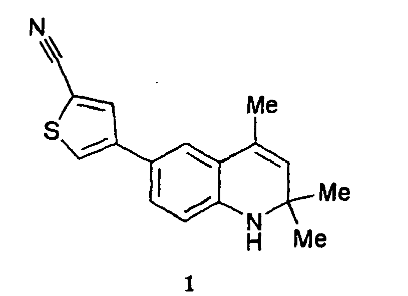

Jones, *et al,* described the enol ether **2** (U.S. Patent No. 5,693,646) as a PR ligand.

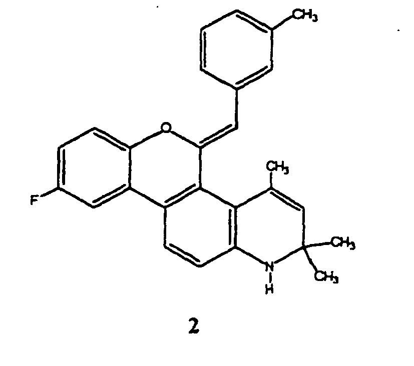

Jones, *et al,* described compound **3** (U.S. Patent No. 5,696,127) as a PR ligand.

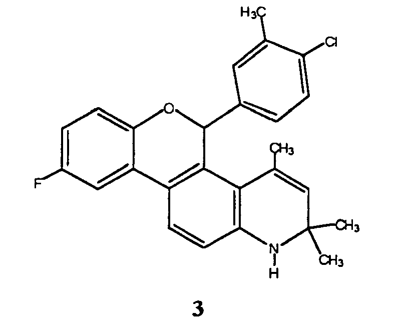

Zhi, *et al,* described lactones **4, 5** and **6** as PR antagonists (J. Med. Chem., 41, 291, 1998).

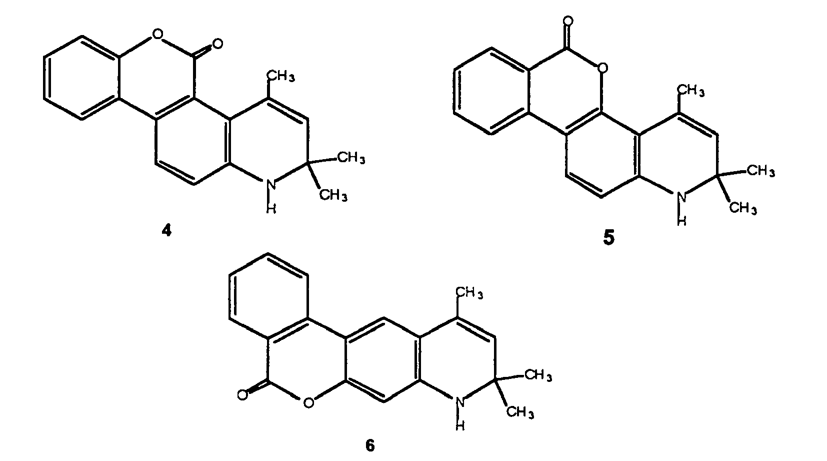

Zhi, *et al,* described the ether **7** as a PR antagonist (J. Med. Chem, 41, 291, 1998).

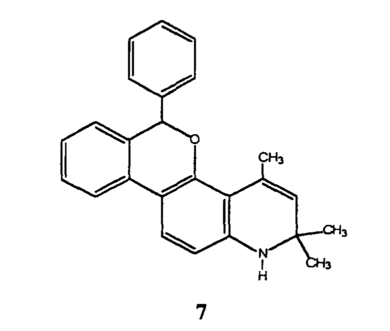

Combs, *et al.,* disclosed the amide **8** as a ligand for the PR (J. Med. Chem., 38, 4880, 1995).

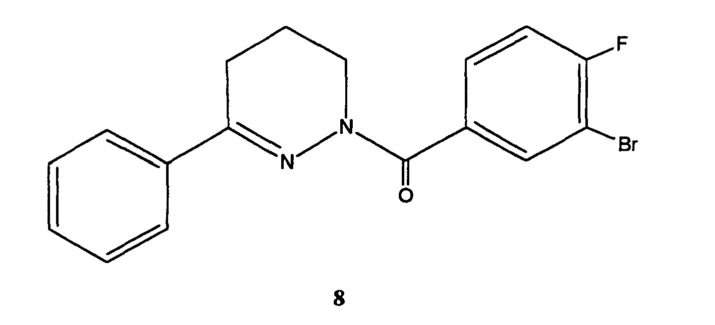

Perlman, *et. al.,* described the vitamin D analog **9** as a PR ligand (Tet. Letters, 35,2295, 1994).

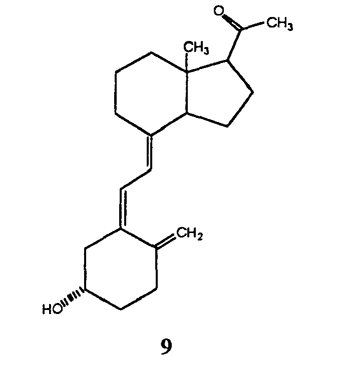

Hamann, *et al,* described the PR antagonist **10** (Ann. N.Y. Acad. Sci., 761, 383, 1995).

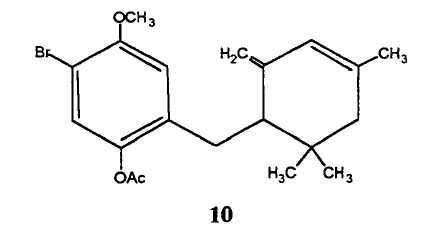

Chen, *et al,* described the PR antagonist **11** (Chen, et al, POI-37, 16th Int. Cong. Het. Chem., Montana, 1997).

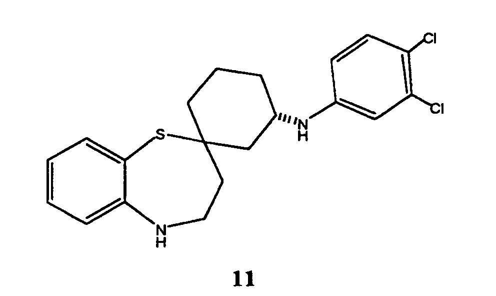

Kurihari, *et. al.,* described the PR ligand **12** *(*J. Antibiotics, 50, 360, 1997).

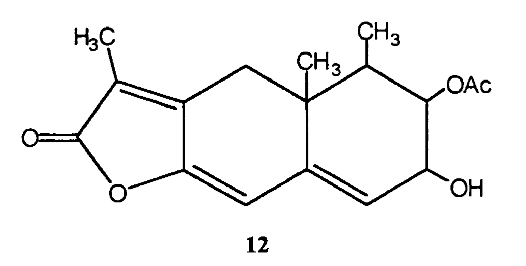

Sakata et al. (JP 07159917, CA 123:301431) teach that certain benzoxazin-2-thione compounds such as compound **A** can be used as photographic materials. Kim et al. disclose that some imidazole substituted benzothiazines, such as compound **B,** can be used as cardiotonics (U.S. Patent No. 5,171,851 and EP 510235). More recently, Young et al. (WO95/20389) and Christ et al. (WO98/14436) claimed benzoxazin-2-thiones such as compound **C** as inhibitors of HIV reverse transcriptase.

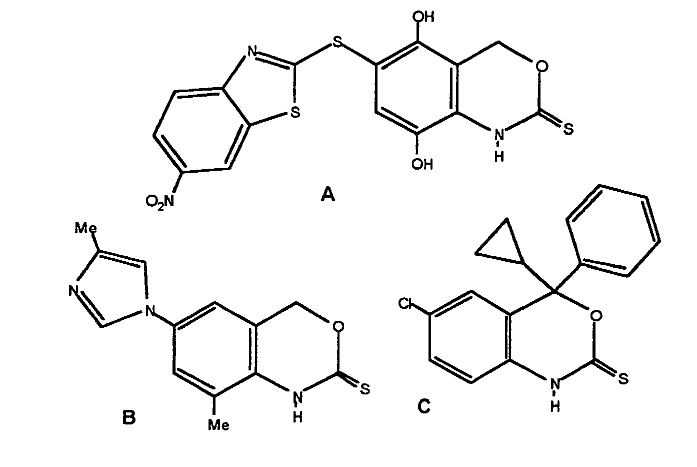

Pflegel et al. (Pharmazie, 37(10), 714-717(1982)) disclosed quinazolin-2-thiones such as compound **D** in their study of polarography of heterocyclics, but disclosed no activity for compound **D**.

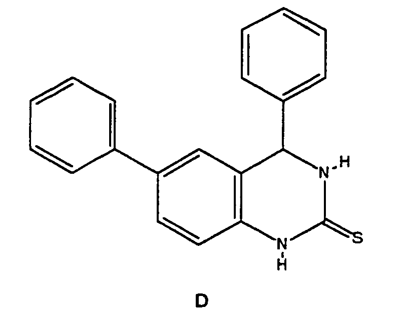

### Description of the Invention

The present invention provides compounds selected from the group consisting of (I), (II), (III), (IV) and (V):
(I) a compound of the formula : 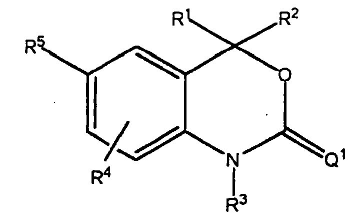 wherein:
   R₁ and R₂ are independent substituents selected from the group consisting of H, C₁ to C₆ alkyl, substituted C₁ to C₆ alkyl, C₂ to C₆ alkenyl, substituted C₂ to C₆ alkenyl, C₂ to C₆ alkynyl, substituted C₂ to C₆ alkynyl, C₃ to C₈ cycloalkyl, substituted C₃ to C₈ cycloalkyl, aryl, substituted aryl, heterocyclic, substituted heterocyclic, COR^{A}, and NR^{B}COR^{A};
   or R¹ and R² are fused to form a ring selected from the group consisting of a), b) and c), each ring being optionally substituted by from 1 to 3 substituents selected from the group consisting of H and C₁ to C₃ alkyl:
      a) a carbon-based 3 to 8 membered saturated spirocyclic ring;
      b) a carbon-based 3 to 8 membered spirocyclic ring having in its backbone one or more carbon-carbon
      c) a 3 to 8 membered spirocyclic ring containing double bonds; and in its backbone one to three heteroatoms selected from the group consisting of O, S and N;
   R^{A} is selected from the group consisting of H, C₁ to C₃ alkyl, substituted C₁ to C₃ alkyl, aryl, substituted aryl, C₁ to C₃ alkoxy, substituted C₁ to C₃ alkoxy, amino, C₁ to C₃ aminoalkyl, and substituted C₁ to C₃ aminoalkyl;
   R^{B} is H, C₁ to C₃ alkyl, or substituted C₁ to C₃ alkyl;
   R₃ is H, OH, NH₂, C₁ to C₆ alkyl, substituted C₁ to C₆ alkyl, C₃ to C₆ alkenyl, substituted C₃ to C₆ alkenyl, alkynyl, substituted alkynyl, or COR^{C};
   R^{C} is selected from the group consisting of H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, and substituted C₁ to C₃ aminoalkyl;
   R⁴ is selected from the group consisting of H, halogen, CN, NO₂, C₁ to C₆ alkyl, substituted C₁ to C₆ alkyl, C₁ to C₆ alkoxy, substituted C₁ to C₆ alkoxy, C₁ to C₆ aminoalkyl, and substituted C₁ to C₆ aminoalkyl;
   R⁵ is selected from the group consisting of (i) and (ii):
      (i) a substituted benzene ring containing the substituents X, Y and Z as shown below: 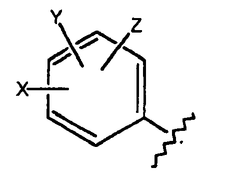 X is selected from the group consisting of H, halogen, CN, C₁ to C₃ alkyl, substituted C₁ to C₃ alkyl, C₁ to C₃ alkoxy, substituted C₁ to C₃ alkoxy, C₁ to C₃ thioalkoxy, substituted C₁ to C₃ thioalkoxy, C₁ to C₃ aminoalkyl, substituted C₁ to C₃ aminoalkyl, NO₂, C₁ to C₃ perfluoroalkyl, 5 or 6 membered heterocyclic ring containing in its backbone 1 to 3 heteroatoms, COR^{D}, OCOR^{D}, and NR^{E}COR^{D};
         R^{D} is H, C₁ to C₃ alkyl, substituted C₁ to C₃ alkyl aryl, substituted aryl, C₁ to C₃ alkoxy, substituted C₁ to C₃ alkoxy, C₁ to C₃ aminoalkyl, or substituted C₁ to C₃ aminoalkyl;
         R^{E} is H, C₁ to C₃ alkyl, or substituted C₁ to C₃ alkyl;
         Y and Z are independent substituents selected from the group consisting of H, halogen, CN, NO₂, C₁ to C₃ alkoxy, C₁ to C₃ alkyl, and C₁ to C₃ thioalkoxy;
         wherein not all of X, Y and Z are H; and
      (ii) a five or six membered heterocyclic ring having in its backbone 1, 2, or 3 ring heteroatoms selected from the group consisting of O, S, S(O), S(O₂) and NR⁶ and containing one or two independent substituents selected from the group consisting of H, halogen, CN, NO₂ and C₁ to C₃ alkyl, C₁ to C₃ alkoxy, C₁ to C₃ aminoalkyl, COR^{F}, and NR^{G}COR^{F};
         R^{F} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
         R^{G} is H, C₁ to C₃ alkyl, or substituted C₁ to C₃ alkyl;
         R⁶ is H, or C₁ to C₃ alkyl;
         Q¹ is S, NR⁷, or CR⁸R⁹;
         R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, or substituted heterocyclic, SO₂CF₃, OR¹¹ and NR¹¹R¹²;
         R⁸ and R⁹ are independent substituents selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂, CN, and CO₂R¹⁰;
         R¹⁰ is C₁ to C₃ alkyl;
         or CR⁸R⁹ is a six membered ring as shown by the structure below: 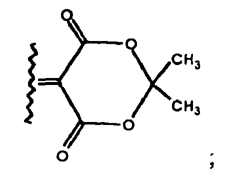 R¹¹ and R¹² are independently selected from the group consisting of H, C₁ to C₆ alkyl, substituted C₁ to C₆ alkyl, aryl, substituted aryl, heteroaryl, substituted heteroaryl, acyl and sulfonyl;
(II) a compound of the formula : 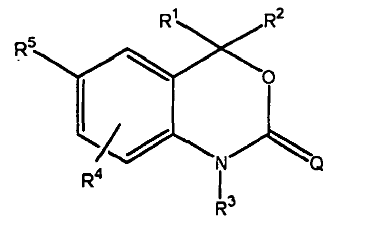 wherein:
   R¹ is H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, COR^{A}, or NR^{B}COR^{A};
   R² is H, C₁ to C₆ alkyl, substituted C₁ to C₆ alkyl, C₂ to C₆ alkenyl, substituted C₂ to C₆ alkenyl, C₃ to C₈ cycloalkyl, substituted C₃ to C₈ cycloalkyl, aryl, substituted aryl, heterocyclic, substituted heterocyclic, COR^{A}, or NR^{B}COR^{A};
   or R¹ and R² are fused to form a spirocyclic ring selected from the group consisting of a), b) and c), each ring being optionally substituted by from 1 to 3 substituents selected from the group consisting of H and C₁ to C₃ alkyl:
      a) a carbon-based 3 to 8 membered saturated spirocyclic ring;
      b) a carbon-based 3 to 8 membered spirocyclic ring having in its backbone one or more carbon- carbon double bonds; and
      c) a 3 to 8 membered spirocyclic ring containing in its backbone one to three heteroatoms selected from the group consisting of O, S and N;
   R^{A} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
   R^{B} is H, C₁ to C₃ alkyl, or substituted C₁ to C₃ alkyl;
   R³ is H, OH, NH₂, C₁ to C₆ alkyl, substituted C₁ to C₆ alkyl, C₃ to C₆ alkenyl, substituted C₃ to C₆ alkenyl, alkynyl, substituted alkynyl, or COR^{c};
   R^{c} is H, C₁ to C₄ alkyl, substituted C₁ to C₄ alkyl, aryl, substituted aryl, C₁ to C₄ alkoxy, substituted C₁ to C₄ alkoxy, C₁ to C₄ aminoalkyl, or substituted C₁ to C₄ aminoalkyl;
   R⁴ is H, halogen, CN, NO₂, C₁ to C₆ alkyl, substituted C₁ to C₆ alkyl, C₁ to C₆ alkoxy, substituted C₁ to C₆ alkoxy, C₁ to C₆ aminoalkyl, or substituted C₁ to C₆ aminoalkyl;
   R⁵ is (i) or (ii): (i) substituted benzene ring containing the substituents X, Y and Z as shown below; 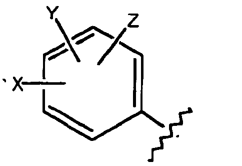
   X is selected from the group including H, halogen, CN, C₁ to C₃ alkyl, substituted C₁ to C₃ alkyl, C₁ to C₃ alkoxy, substituted C₁ to C₃ alkoxy, C₁ to C₃ thioalkoxy, substituted C₁ to C₃ thioalkoxy, C₁ to C₃ aminoalkyl, substituted C₁ to C₃ aminoalkyl, NO₂, C₁ to C₃ perfluoroalkyl, 5 membered heterocyclic ring containing in its backbone 1 to 3 heteroatoms, COR^{D}, OCOR^{D}, and NR^{E}COR^{D};
   R^{D} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
   R^{E} is H, C₁ to C₃ alkyl, or substituted C₁ to C₃ alkyl;
   Y and Z are independent substituents selected from the group consisting of H, halogen, CN,
   NO₂, C₁ to C₃ alkoxy, C₁ to C₃ alkyl, and C₁ to C₃ thioalkoxy; wherein not all of X, Y and Z are H; or
      (ii) a five or six membered ring having in its backbone 1, 2, or 3 heteroatoms selected from the group consisting of O, S, SO, SO₂ and NR⁶ and containing one or two independent substituents selected from the group consisting of H, halogen, CN, NO₂, C₁ to C₃ alkyl and C₁ to C₃ alkoxy,
         R⁶ is H or C₁ to C₃ alkyl,
         Q¹ is S, NR⁷ or CR⁸R⁹ ;
         R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic and SO₂CF₃.
         R⁸ and R⁹ are independent substituent selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂, CN and CO₂R¹⁰;
         R¹⁰ is C₁ to C₃ alkyl;
      or CR⁸R⁹ is a six membered ring as shown by the structure below ; 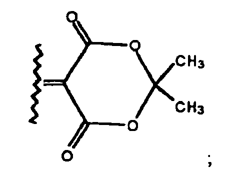
(III) a compound of the formula: 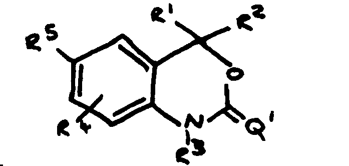 wherein:
   R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
   R³ is H, OH, NH₂, C₁ to C₆ alkyl, substituted C₁ to C₆ alkyl, or COR^{C};
   R^{C} is H, C₁ to C₄ alkyl, or C₁ to C₄ alkoxy;
   R⁴ is H, halogen NO₂, C₁ to C₃ alkyl, or substituted C₁ to C₃ alkyl;
   R⁵ is (i) or (ii) : (i) a substituted benzene ring of the structure: 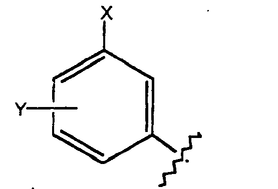
   wherein:
   X is selected from the group consisting of halogen, CN, C₁ to C₃ alkoxy, C₁ to C₃ alkyl, NO₂, C₁ to C₃ perfluoroalkyl, 5 membered heterocyclic ring containing in its backbone 1 to 3 heteroatoms, and C₁ to C₃ thioalkoxy;
   Y is selected from the group consisting of H, halogen, CN, NO₂, C₁ to C₃ alkoxy, C₁ to C₄ alkyl, and C₁ to C₃ thioalkoxy;
      or
      (ii) a five membered ring of the structure: 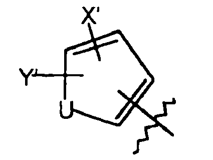 wherein :
         U is O, S, or NR⁶;
         R⁶ is H, C₁ to C₃ alkyl, or C₁ to C₄ CO₂alkyl;
         X' is selected from the group consisting of halogen, CN, NO₂, C₁ to C₃ alkyl or C₁ to C₃ alkoxy;
         Y' is selected from the group consisting of H halogen, CN, NO₂, C₁ to C₃ alkoxy, C₁ to C₄ alkyl and C₁ to C₃ thioalkoxy;
         Q¹ is S, NR⁷, or CR⁸R⁹;
         R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃;
         R⁸ and R⁹ are independent substituents selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂, CN and CO₂R¹⁰;
         R¹⁰ is C₁ to C₃ alkyl;
         or CR⁸R⁹ a six membered ring as shown by the structure below 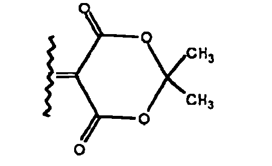
(IV) a compound of the formula: 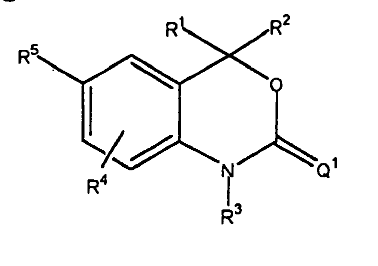 wherein:
   R¹ and R² are independently selected from the group consisting of C₁ to C₃ alkyl and substituted C₁ to C₃ alkyl, or R¹ and R² are fused to form a carbon-based 3 to 6 membered saturated spirocyclic ring;
   R³ is H;
   R⁴ is H, halogen, NO₂, C₁ to C₃ alkyl; or substituted C₁ to C₃ alkyl;
   R⁵ is the 6-membered ring of the structure: 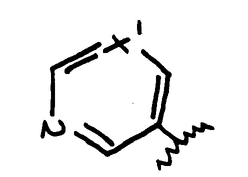
   wherein :
   X¹ is N; and
   Q¹ is S, NR⁷, or CR⁸R⁹; and
   with the proviso for each of compounds (I) to (IV) that when Q¹ is S and R³ is H, both R¹ and R² are not H;
   with the proviso for each of compounds (I) to (IV) that when Q¹ is S; R³ is H; R⁵ is a benzene ring (i) substituted with one or two substituents or R⁵ is the 5 or 6 membered ring (ii) having in its backbone 1, 2, or 3 heteroatoms selected from the group consisting of O, S, and NR⁶, and one of R¹ or R² is C₂ to C₄ alkenyl, substituted C₂ to C₄ alkenyl, C₂ to C₄ alkynyl, or substituted C₂ to C₄ alkynyl, the other of R² or R¹ is not C₁ to C₆ alkyl, substituted C₁ to C₆ alkyl, C₃ to C₅ cycloalkyl, substituted C₃ to C₅ cycloalkyl, C₂ to C₄ alkenyl, substituted C₂ to C₄ alkenyl, C₂ to C₄ alkynyl, nor substituted C₂ to C₄ alkynyl;
   with the proviso for each of compounds (I) to (IV) that when Q¹ is S; R³ is H; R⁵ is a benzene ring (i) substituted with one or two substituents or R⁵ is the 5 or 6 membered ring (ii) having in its backbone 1, 2, or 3 heteroatoms selected from the group consisting of O, S, and NR⁶, and one of R¹ or R² is C₁ to C₆ alkyl or substituted C₁ to C₆ alkyl, the other of R² or R¹ is not C₃ to C₈ cycloalkyl nor substituted C₃ to C₈ cycloalkyl;
   with the proviso that for each of compounds (I) to (IV) the term "substituted alkyl" does not include perfluorinated alkyl;
(V) 6-[3-Fluoro-5-(trifluoromethyl)phenyl]-4,4-dimethyl-1,4-dihydro-2H-3,1-benzoxazine-2-thione;
   or a pharmaceutically acceptable salt thereof.

The compounds of the present invention may contain an asymmetric carbon atom and some of the compounds of the present invention may contain one or more asymmetric centers and may thus give rise to optical isomers and diastereomers. While shown without respect to the stereochemistry of the formula, the present invention includes such optical isomers and diastereomers; as well as the racemic and resolved, enantiomerically pure R and S stereoisomers; as well as other mixtures of the R and S stereoisomers and pharmaceutically acceptable salts thereof.

The term "alkyl" is used herein to refer to both straight- and branched-chain saturated aliphatic hydrocarbon groups having one to eight carbon atoms, preferably one to six carbon atoms; "alkenyl" is intended to include both straight- and branched-chain alkyl group with at least one carbon-carbon double bond and two to eight carbon atoms, preferably one to six carbon atoms; "alkynyl" group is intended to cover both straight- and branched-chain alkyl group with at least one carbon-carbon triple bond and two to eight carbon atoms, preferably two to six carbon atoms. The terms "substituted alkyl", "substituted alkenyl", and "substituted alkynyl" refer to alkyl, alkenyl, and alkynyl as just described having one or more substituents from the group including halogen, CN, OH, NO₂, amino, aryl, heterocyclic, substituted aryl, substituted heterocyclic, alkoxy, aryloxy, substituted alkyloxy, alkylcarbonyl, alkylcarboxy, alkylamino, arylthio. These substituents may be attached to any carbon of alkyl, alkenyl, or alkynyl group provided that the attachment constitutes a stable chemical moiety. The term "aryl" is used herein to refer to an aromatic system which may be a single ring or multiple aromatic rings fused or linked together as such that at least one part of the fused or linked rings forms the conjugated aromatic system The aryl groups include, but are not limited to, phenyl, naphthyl, biphenyl, anthryl, tetrahydronaphthyl, phenanthryl. The term "substituted aryl" refers to aryl as just defined having one to four substituents from the group including halogen, CN, OH, NO₂, amino, alkyl, cycloalkyl, alkenyl, alkynyl, alkoxy, aryloxy, substituted alkyloxy, alkylcarbonyl, alkylcarboxy, alkylamino, or arylthio. The term "heterocyclic" is used herein to describe a stable 4- to 7-membered monocyclic or a stable multicyclic heterocyclic ring which is saturated, partially unsaturated, or unsaturated, and which consists of carbon atoms and from one to four heteroatoms selected from the group including N, O, and S atoms. The N and S atoms may be oxidized. The heterocyclic ring also includes any multicyclic ring in which any of above defined heterocyclic rings is fused to an aryl ring. The heterocyclic ring may be attached at any heteroatom or carbon atom provided the resultant structure is chemically stable. Such heterocyclic groups include, for example, tetrahydrofuran, piperidinyl, piperazinyl, 2-oxopiperidinyl, azepinyl, pyrrolidinyl, imidazolyl, pyridyl, pyrazinyl, pyrimidinyl, pyridazinyl, oxazolyl, isoxazolyl, morpholinyl, indolyl, quinolinyl, thienyl, furyl, benzofuranyl, benzothienyl, thiamorpholinyl, thiamorpholinyl sulfoxide, and isoquinolinyl. The term "substituted heterocyclic" is used herein to describe the heterocyclic just defined having one or more substituents selected from the group which includes halogen, CN, OH, NO₂, amino, alkyl, substituted alkyl, cycloalkyl, alkenyl, substituted alkenyl, alkynyl, alkoxy, aryloxy, substituted alkyloxy, alkylcarbonyl, alkylcarboxy, alkylamino, or arylthio. The term "alkoxy" is used herein to refer to the OR group, where R is alkyl or substituted alkyl. The term "aryloxy" is used herein to refer to the OR group, where R is aryl or substituted aryl. The term "alkylcarbonyl" is used herein to refer to the RCO group, where R is alkyl or substituted alkyl. The term "alkylcarboxy" is used herein to refer to the COOR group, where R is alkyl or substituted alkyl. The term "aminoalkyl" refers to both secondary and tertiary amines wherein the alkyl or substituted alkyl groups, containing one to eight carbon atoms, which may be either same or different and the point of attachment is on the nitrogen atom The term "halogen" refers to Cl, Br, F, and I elements.

The compounds of the invention can be prepared following the Schemes illustrated below:

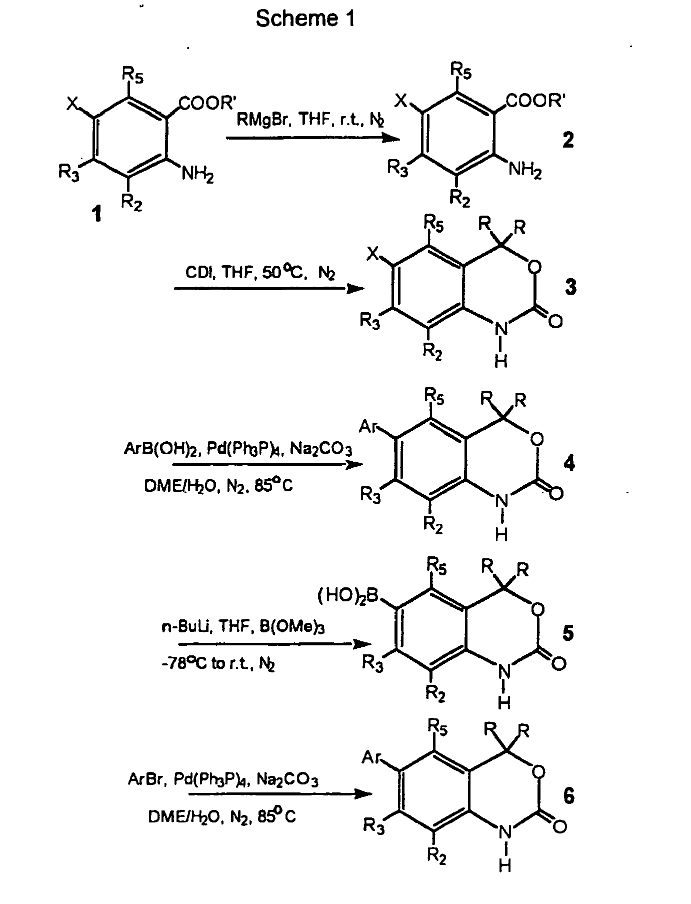

As demonstrated in Scheme I, the compounds of the invention are generally prepared by employing the suitable coupling reaction as a final step. An appropriately substituted ortho-amino benzoic acid or its derivatives such as ethyl ester (X = Br, I, Cl, or a latent coupling precursor such as alkoxy group which can be converted into a OTf group suitable in the coupling reaction) was treated with a suitable organo metallic reagent, e.g. Grignard reagent, in appropriate nonprotic solvents which include, but are not limited to, THF or ether to give ortho-amino carbinol 2 under an inert atmosphere such as argon or nitrogen at -78 °C to room temperature. Ring closure of carbinol **2** to yield benzoxazin-2-ones **3** is commonly effected by a condensing agent such as carbonyldiimidazole, phosgene, dimethylcarbonate, or diethylcarbonate in a suitable nonprotic solvent such as THF at temperatures ranging from room temperature to 65 °C. The arylation of benzoxazin-2-ones **3** to yield **4** can be effected by various coupling reactions including Suzuki, Stille reactions. These reactions are commonly performed in the presence of transition metallic catalyst, e.g., palladium or nickel complex often with phosphino ligands, e.g., Ph₃P, dppf, dppe or palladium acetate. Under this catalytic condition, an appropriately substituted nucleophilic reagent, e.g., aryl boronic acid, arylstannane, or aryl zinc compound, is coupled with benzoxazinones **3** to give **4.** If a base is needed in the reaction, the commonly used bases include, but are not limited to, sodium bicarbonate, sodium carbonate, potassium phosphate, barium carbonate, or potassium acetate. The most commonly used solvents in these reactions include benzene, DMF, isopropanol, ethanol, DME, ether, acetone, or a mixture of above solvents and water. The coupling reaction is generally executed under an inert atmosphere such as nitrogen or argon at temperatures ranging from room temperature to 95 °C.

Benzoxazinones **3** can be converted into a nucleophile such as boronic acid which can be coupled with an appropriate electrophile, e.g., aryl bromide or aryl iodide, to yield **4** employing the coupling reaction condition as described above. The transformation of **3** into **5** can be effected by treating **3** with an organo metallic reagent, e.g., *n*-BuLi, in a nonprotic solvent such as THF or ether followed by quenching the reaction solution with a suitable electrophile, such as trimethyl borate, triisopropyl borate, or zinc chloride at temperatures ranging from -78 °C to room temperature under an inert atmosphere such as argon or nitrogen.

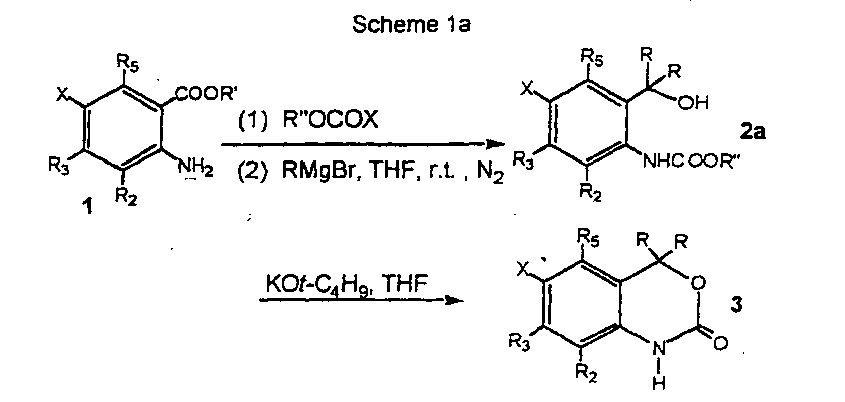

Scheme Ia illustrates an alternative approach leading to the benzoxazinones 3. Thus, an appropriate aniline 1 is protected with a suitable alkoxy carbonyl protective group including but not limited to allenoxy carbonyl, *t*-butoxy carbonyl, benzoxy carbonyl, ethoxy carbonyl, or methoxy carbonyl in a suitable solvent such as THF, acetonitrile, with or without presence of a base either as a catalyst or as an acid scavenger. The protected aniline is then treated with a suitable organo metallic reagent such as organo lithium agent or Grignard reagent in the same fashion as to prepare compound 2 to give the carbinol 2a. The treatment of **2a** with a suitable base such as potassium *t*-butoxide, *n*-butyl lithium, potassium hydroxide in an appropriate solvent such as toluene, THF, alcohol under an inert atmosphere such as nitrogen or argon at the temperature ranging from room temperature to the boiling point of the relevant solvent to afford benzoxazinones **3**.

Scheme II describes the procedures to prepare benzoxazinones bearing two different substituents at position-4. The Weinreb amide **8** can be prepared from an appropriately substituted isatoic anhydride 7 when treated with *N-, O-*dimethylhydroxyl-amine hydrochloride salt in a protic solvent such as ethanol, isopropanol at reflux under an inert atmosphere such as argon or nitrogen. Coupling of amide **8** with an aryl electrophile such as aryl boronic acid or arylstannane to give 9 can be effected by employing a typical coupling reaction such as Suzuki, Stille coupling procedure in a similar fashion as described for the preparation of benzoxazinones **4.** Treatment of Weinreb amide **9** with organo metallic compounds, e.g., alkyllithium, alkynyllithium, aryllithium, or their Grignard counterpart in a nonprotic solvent such as THF or ether under an inert atmosphere such as argon or nitrogen at -78 °C to room temperature affords amino ketone **10.** Conversion of ketone **10** to carbinol **11** can be effected by treatment of **10** with an organo metallic reagent such as alkyl, alkynyl, or aryl Grignard compound in a nonprotic solvent such as THF or ether under an inert atmosphere such as argon or nitrogen at -78 °C to room temperature. Conversion of ketone 10 to carbinol **11** can also be effected by reduction of ketone group of **10** to the carbinol moiety of **11** using an appropriate reducing reagent such as lithium aluminum hydride, sodium borohydride in a suitable solvent such as THF, ether, or anhydrous alcohol under an inert atmosphere in the temperature range from 0 °C to the boiling point of the solvent. Ring closure of carbinol **11** to produce the compounds of the invention can be accomplished with condensing agents such as carbonyldiimidazole, phosgene, dimethylcarbonate, or diethylcarbonate in a suitable nonprotic solvent such as THF at temperatures ranging from room temperature to 65 °C.

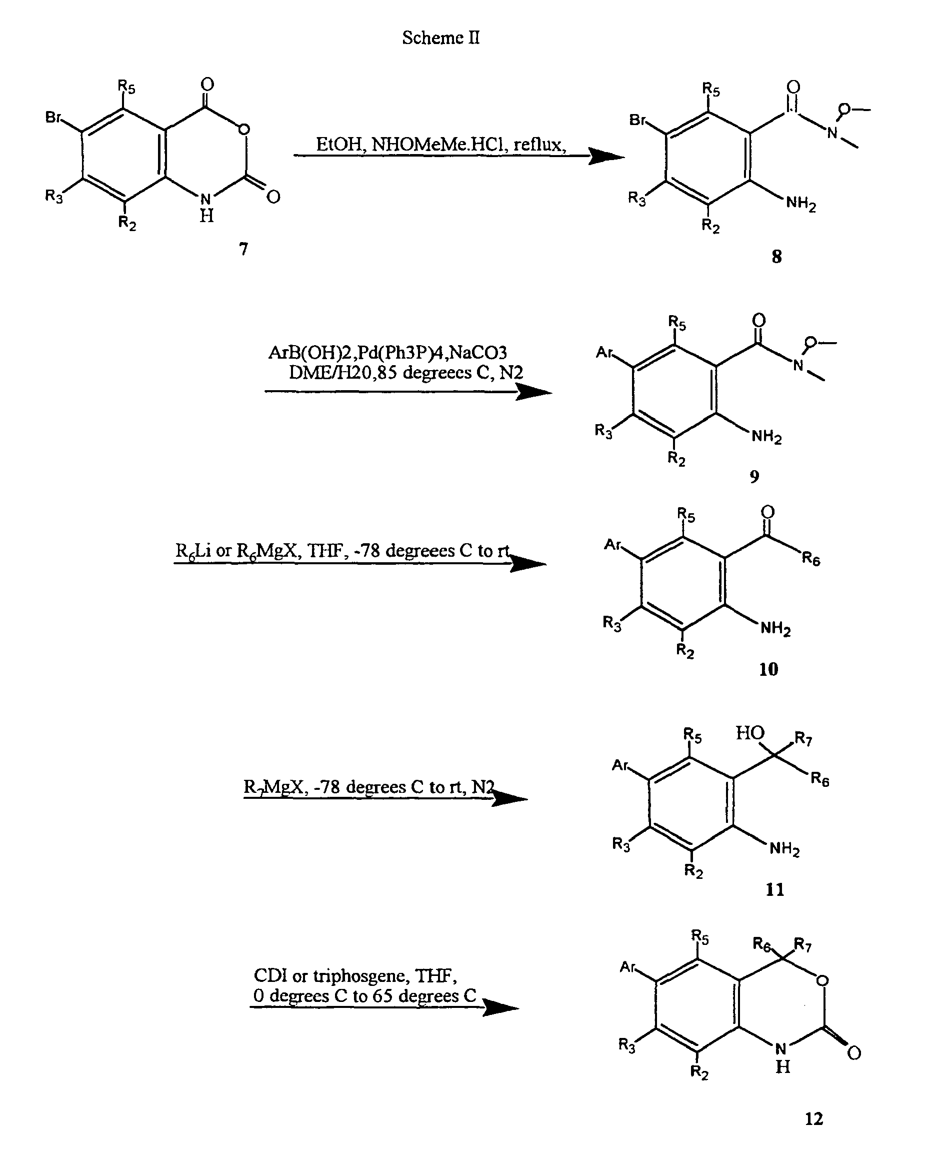

Alternatively, ortho-amino ketone **10** can be prepared by treatment of ortho-amino benzonitrile **14** with an organo metallic compound such as organo lithium reagent or Gringard reagent in a suitable solvent such as THF or ether under an inert atmosphere such as argon or nitrogen at temperatures ranging from -78 °C to room temperature as illustrated in Scheme III. Benzonitrile **14** can be readily prepared from an appropriately substituted benzonitrile such as bromobenzonitrile **13** using a suitable coupling reaction such as Stille or Suzuki protocol carried out in a similar fashion as described for the preparation of the Weinreb amide **9.** 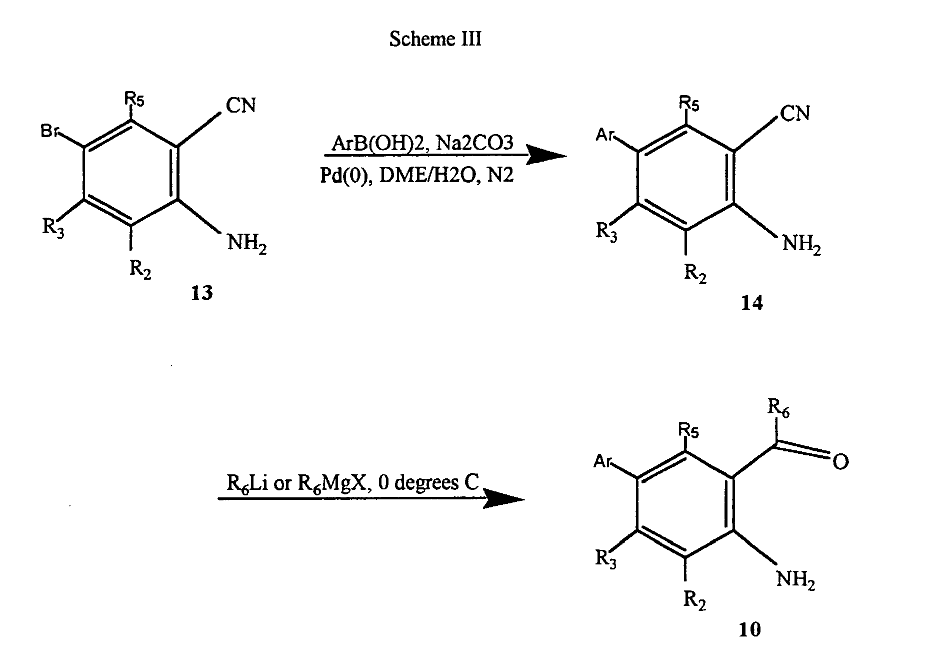 Scheme IV depicts an approach to prepare benzoxazinones with a low perfluoroalkyl substituent at position-4, e.g. R₆ is trifluoromethyl group. An appropriately substituted chloroaniline **15** was protected with a suitable protective group such as pivaloyl chloride or di-*tert*-butyl pyrocarbonate to give protected aniline **16** in a suitable solvent such as acetonitrile, acetone, THF, methylene chloride, or a mixture of solvent such as methylene chloride and water under an inert atmosphere such as argon or nitrogen at temperatures ranging from 0 °C to 70 °C. A suitable base such as sodium carbonate, sodium bicarbonate, or potassium carbonate may be needed when the reaction produces an acid as a side-product such as hydrochloride. Treatment of 16 with an appropriate alkyllithium such as *n*-butyllithium or s-butyllithium followed by reaction with a low perfluorocarboxy derivatives, e.g., trifluoroacetyl chloride, 1-(trifluoroacetyl)- imidazole, or ethyl trifluoroacetate in a nonprotic solvent such as ether or THF under an inert atmosphere such as argon or nitrogen at -78 °C to ambient temperature gives the protective ortho-amino ketones. Subsequent removal of the protecting group can be effected by reaction of protected amino ketones with a suitable acid such as TFA, 3N aqueous hydrochloride solution in a suitable solvent such as methylene chloride or water at 0 °C to boiling point of the solvent to afford ortho-amino ketone **17**.

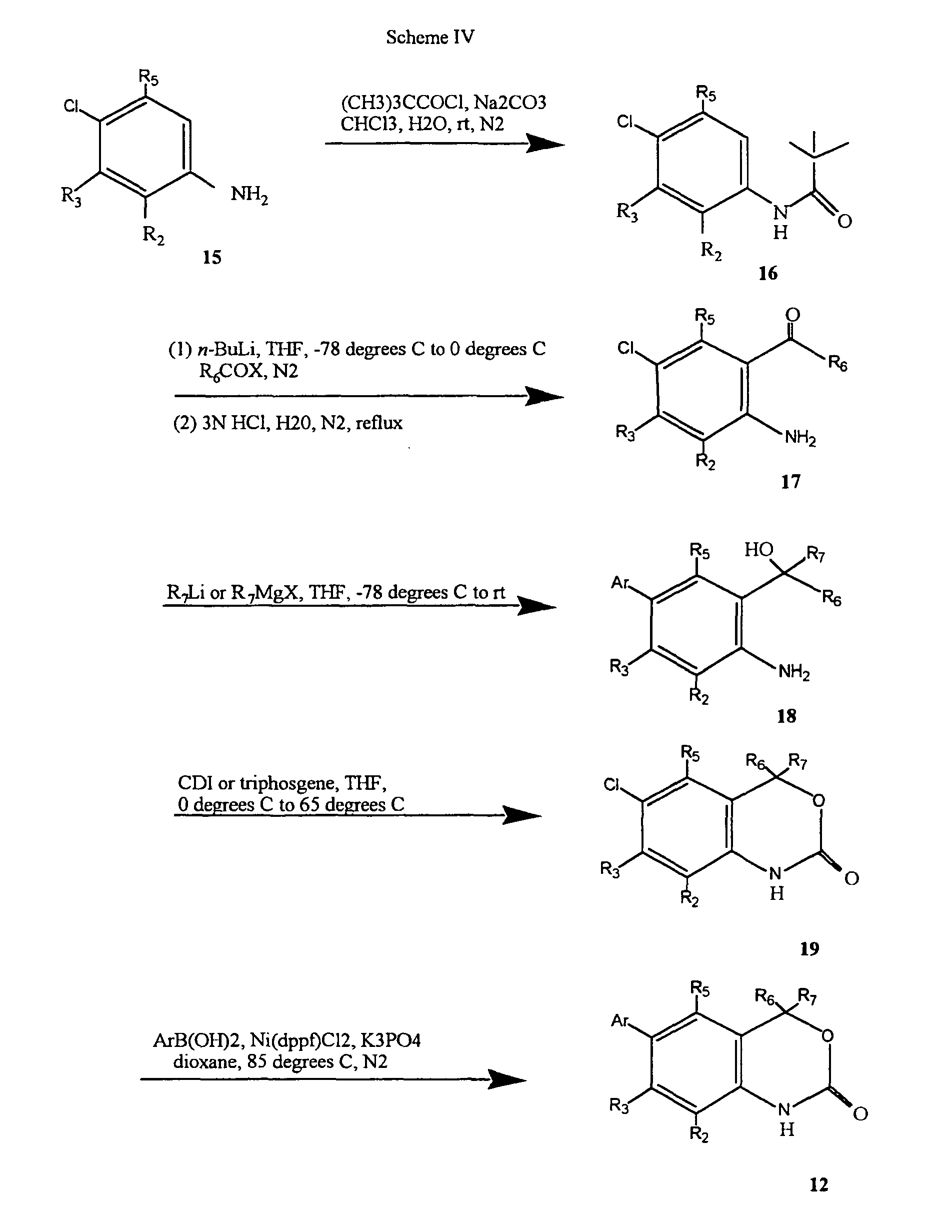

The preparation of 6-chlorobenzoxazinones **19** from **17** can be accomplished in the same fashion as described for the synthesis of benzoxazinone **12** from ketone **10.** Coupling of **19** with an aryl group to yield **12** can be effected by a nickel complex catalyzed coupling reaction. The palladium catalysts proved not to be an efficient catalyst in this coupling process. The coupling reaction of **19** with an appropriate aryl boronic acid can be accomplished in the presence of a suitable base such as potassium phosphate and a catalyst of nickel (0 or II) complex, e.g. a nickel complex of 1,2-bis(diphenylphosphino)ethane, 1,1'-bis(diphenylphosphino)ferrocene, or triphenylphosphine. The most commonly used solvents in the reaction include dioxane or THF. The coupling reaction is generally executed under an inert atmosphere such as nitrogen or argon at temperatures ranging from ambient temperature to 95 °C.

As described in Scheme V the conversion of benzoxazin-2-ones **3** or **12** into benzoxazin-2-thiones **20** or **21** can be accomplished by treatment of **3** or **12** with a suitable sulfur reagent such as Lawesson's reagent in a nonprotic solvent such as o-xylene, chlorobenzene, or toluene under an inert atmosphere such as argon or nitrogen at reflux.

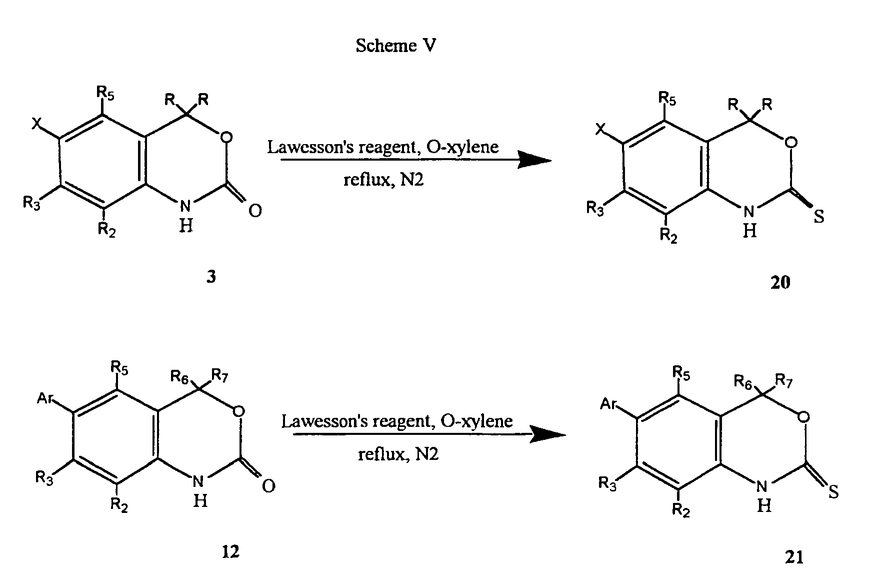

Schemes VI and VII describe the synthesis of other benzoxazinone bioisosteres. Using a similar procedure reported by Kondo et al. (Kondo, et al. J. Med. Chem. 33(7), 2012-2015 (1990)) compound **22** can be formed by treatment of amino carbinol **11** with an appropriate ketene-*S*, *S*-acetals (at least one of R₁₆ or R₁₇ is an electron withdrawing group) in a suitable solvent such as toluene or anhydrous ethanol under an inert atmosphere such as nitrogen or argon at reflux. In a similar fashion, compound **23** can be formed by reaction of amino carbinol **11** with an appropriate imino-S, S-acetals or imino-acetals (R₁₈ is an electron withdrawing group) employing a procedure similar to that of Evers, et al. (Evers, et al. I. Prakt. Chem. 333(5), 699-710 (1991)) or Haake et al. (Haake et al. Synthesis-Stuttgart 9, 753-758 (1991)) in a suitable solvent such as ethanol under an inert atmosphere such as argon or nitrogen at reflux. Other procedures (e.g. Wrobel et al. J. Med. Chem. 32, 2493(1989)) potentially leading to compounds **22** or **23** from **20** or **21** is illustrated in scheme VIIa. Thus, compound **20** or **21** is alkylated with an appropriate alkylating agent such as the Meerwein reagent in a suitable solvent such as methylene chloride. This is then followed by a nucleophilic replacement of an appropriate nucleophile such as carbon anion or a amine base to give compounds **22** or **23,** which may produce either tautomeric form of compounds **22** or **23.**

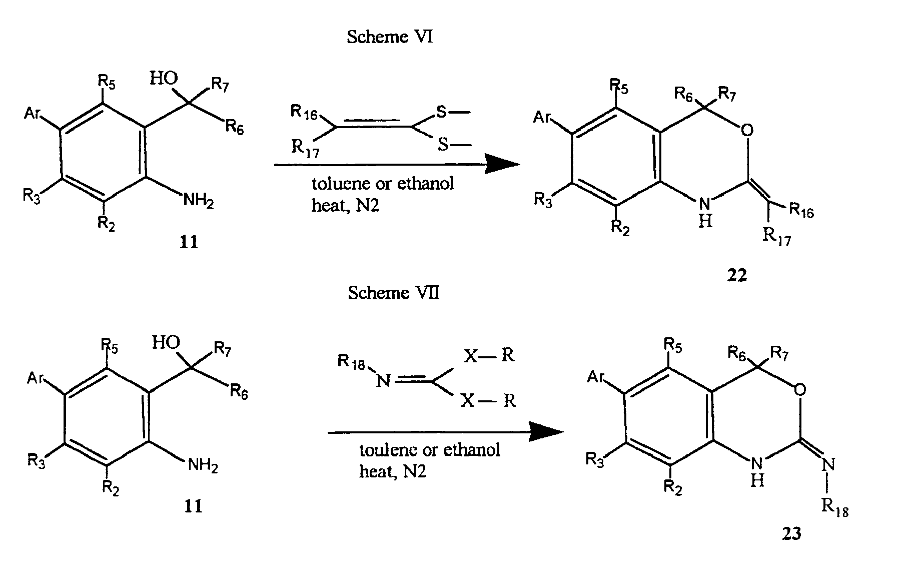

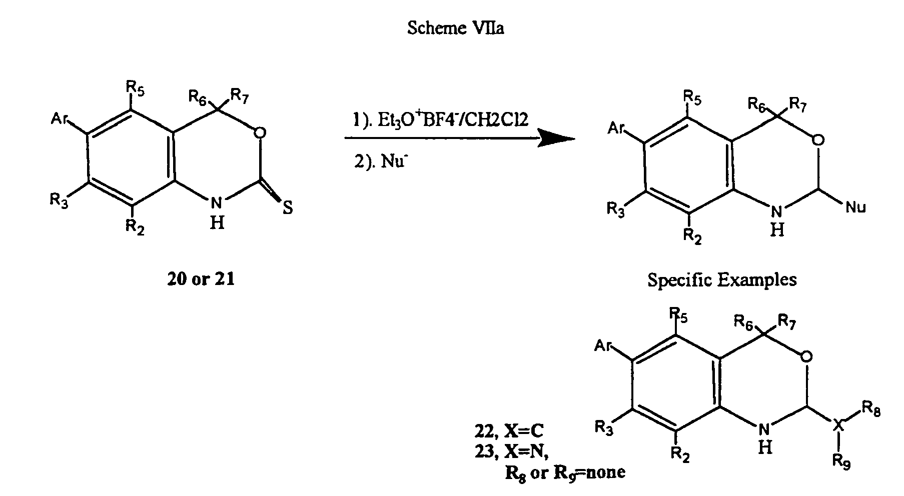

As illustrated in Scheme VIII, the compound **21** can be further derivatized at position-1 via numerous approaches leading to a variety of the novel cyclothiocarbamate derivatives including 1-alkyl, substituted 1-alkyl, 1-carbonyl, substituted 1-carbonyl, 1-carboxy, substituted 1-carboxy derivatives. For example, alkyl or substituted alkyl derivatives **24** can be formed by treatment of thiocarbamate **12** or **6** with a suitable base such as sodium hydride in suitable solvent such as DMF under an inert atmosphere, such as argon or nitrogen, followed by addition of an appropriate electrophile such as alkyl or substituted alkyl bromide, iodide, or triflate. Such a transformation of **21** at position-1 can also be effected using a biphasic condition as indicated in Scheme VIII in which alkylation is executed using a biphasic catalyst such as tributylammonium bromide in a suitable solvent such as acetonitrile. A further example of such a modification includes, but is not limited to, heating 21 with triethyl orthoformate to afford 1-substituted derivatives **24.** (Scheme VIII)

The acylation or carboxylation of the compound **21** at position-1 to give compound **25** can be readily effected by treatment of **12** or **6** with a suitable acylating or carboxylating reagent such as di-*t*-butyl dicarbonate in the presence of a suitable basic catalyst such as DMAP in a suitable solvent such as acetonitrile under an inert atmosphere such as argon or nitrogen. The amination of position-1 of compound **21** to give compound **26** can be furnished using a suitable aminating reagent such as chloroamine in the presence of a suitable base such as sodium hydride in a suitable solvent such as THF or diethyl ether following the literature procedure (Metlesics et al. J. Org. Chem. 30, 1311(1965)).

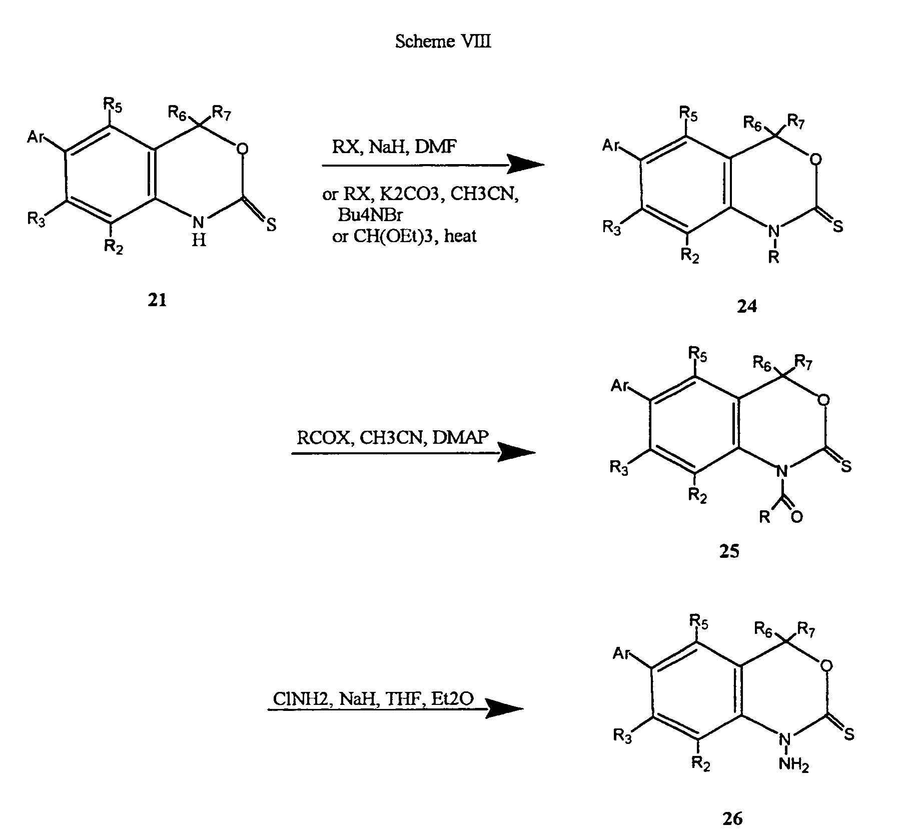

The compounds of the present invention can be used in the form of salts derived from pharmaceutically or physiologically acceptable acids or bases. These salts include, but are not limited to, the following salts with inorganic acids such as hydrochloric acid, sulfuric acid, nitric acid, phosphoric acid and, as the case may be, such organic acids as acetic acid, oxalic acid, succinic acid, and maleic acid. Other salts include salts with alkali metals or alkaline earth metals, such as sodium, potassium, calcium or magnesium in the form of esters, carbamates and other conventional "pro-drug" forms, which, when administered in such form, convert to the active moiety *in vivo.*

This invention includes pharmaceutical compositions and use of the compounds of the invention in the preparation of medicaments for use as agonists of the progesterone receptor.

The progesterone receptor agonists of this invention, used alone or in combination, can be utilized in methods of contraception and in the treatment of dysfunctional bleeding, uterine leiomyomata, endometriosis, polycystic ovary syndrome, and of carcinomas and adenocarcinomas of the endometrium, ovary, breast, colon, and prostate and also for use in hormone replacement therapy. Additional uses of the invention include stimulation of food intake.

When the compounds are employed for the above utilities, they may be combined with one or more pharmaceutically acceptable carriers or excipients, for example, solvents and diluents, and may be administered orally in such forms as tablets, capsules, dispersible powders, granules, or suspensions containing, for example, from about 0.05 to 5% of suspending agent, syrups containing, for example, from about 10 to 50% of sugar, and elixirs containing, for example, from about 20 to 50% ethanol, or parenterally in the form of sterile injectable solutions or suspensions containing from about 0.05 to 5% suspending agent in an isotonic medium Such pharmaceutical preparations may contain, for example, from about 25 to about 90% of the active ingredient in combination with the carrier, more usually between about 5% and 60% by weight.

The effective dosage of active ingredient employed may vary depending on the particular compound employed, the mode of administration and the severity of the condition being treated. However, in general satisfactory results are obtained when the compounds of the invention are used in the preparation of a medicament and are administered at a daily dosage of from about 0.5 to about 500 mg/kg of animal body weight, preferably given in divided doses two to four times a day, or in a sustained release form For most large mammals, the total daily dosage is from about 1 to 100 mg, preferably from about 2 to 80 mg. Dosage forms suitable for internal use comprise from about 0.5 to 500 mg of the active compound in intimate admixture with a solid or liquid pharmaceutically acceptable carrier. This dosage regimen may be adjusted to provide the optimal therapeutic response. For example, several divided doses may be administered daily or the dose may be proportionally reduced as indicated by the exigencies of the therapeutic situation.

The compounds used in the preparation of a medicament may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as com, peanut and sesame oils, as are appropriate to the nature of the active ingredient and the particular form of administration desired. Adjuvents customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the standpoint of ease of preparation and administration are solid compositions, particularly tablets and hard-filled or liquid-filled capsules. Oral administration of the compounds is preferred.

The compounds used in the preparation of a medicament may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid, polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringe ability exits. It must be stable under conditions of manufacture and storage and must be preserved against the contaminating action of microorganisms such as bacterial and fungi. The carrier can be a solvent or dispersion medium containing, for example, water, ethanol (e.g., glycerol, propylene glycol and liquid polyethylene glycol), suitable mixtures thereof, and vegetable oil.

The following examples illustrate preparation of compounds of the invention.

### EXAMPLE 1

### 2-(2-Amino-5-bromophenyl)propan-2-ol

A solution of 2-amino-5-bromobenzoic acid (10g, 46 mmol) in dry THF (200 mL) was treated at -78 °C under nitrogen with a solution of methylmagnesium bromide in ether (3.0 M, 90 mL, 270 mmol). The reaction mixture was slowly warmed to ambient temperature, kept stirring for 48 hours under nitrogen and then poured into a cold 0.5 N aqueous hydrochloride solution (300 mL). The mixture was neutralized with aqueous 1 N sodium hydroxide solution and ethyl acetate (300 mL) was added. The organic layer was separated and aqueous layer was extracted with ethyl acetate (3x100 mL). The combined organic layers were washed with brine and dried (MgSO₄). After removal of solvent *in vacuo,* the residue was purified by a silica gel flash chromatography (hexane:ethyl acetate/3:2) to give 2-(2-amino-5-bromophenyl)propan-2-ol as an off-white solid (6g, 57%): mp 62-63 °C; ¹H-NMR (CDCl₃) δ 7.19 (d, 1H, *J =* 2.3 Hz), 7.12 (dd, 1H, *J =* 8.4, 2.3 Hz), 6.51 (d, 1H, *J =* 8.4 Hz), 4.70 (s, 2H), 1.82 (s, 1H), 1.65 (s, 6H).

### EXAMPLE 2

### 6-Bromo-4,4-dimethyl-1,4-dihydro-benzo[d][1,3]oxazin-2-one

To a solution of 2-(2-amino-5-bromophenyl)propan-2-ol (18g, 78 mmol) in dry THF (150 mL) was added 1,1'-carbonyldiimidazole (15.5g, 94 mmol) under nitrogen. The reaction solution was heated at 50 °C overnight. The solvent was removed *in vacuo* and the residue was dissolved in ethyl acetate (100 mL). The solution was washed with 1N aqueous hydrochloride solution (2x40 mL), brine (20 mL), and dried with MgSO₄. After removal of solvent *in vacuo,* 6-bromo-4,4-dimethyl-1,4-dihydro-benzo[d][1,3]-oxazin-2-one was obtained as a white solid (20 g, 100%): mp 199-200 °C; ¹H-NMR (DMSO-d₆) δ 10.32 (s, 1H, D₂O exchangeable), 7.48 (d, 1H, *J =* 2.1 Hz), 7.43 (dd, 1H, *J=* 8.5, 2.1 Hz), 6.84 (d, 1H, *J =* 8.4 Hz), 1.61 (s, 6H).

### EXAMPLE 3

### (1,4-Dihydro-4,4-dimethyl-2-oxo-2H-3,1-benzoxazin-6-yl)boronic acid

To a solution of 6-bromo-4,4-dimethyl-1,4-dihydro-benzo[d][1,3]oxazin-2-one (2g, 7.8 mmol) in anhydrous THF (60 mL) was added a solution of *n*-BuLi in hexane (10 M, 2.4 mL, 24 mmol) at -78 °C under nitrogen. After stirred at -78 °C for 30 minutes, a slurry was obtained and treated with triisopropyl borate (6.5 mL, 28 mmol). The reaction solution was slowly warmed to ambient temperature and quenched with 1N aqueous hydrochloric acid solution (60 mL). Ethyl acetate (100 mL) was added and organic layer was separated, and aqueous layer was extracted with ethyl acetate (3x60 mL). The combined organic layer was washed with brine and dried with MgSO₄. The solvent was *removed in vacuo* and the residue was purified by a silica gel flash chromatography (ethyl acetate:hexane/2:1) to afford (1,4-dihydro-4,4-dimethyl-2-oxo-2H-3,1-benzoxazin-6-yl)boronic acid as a white solid (1.4g, 81%): mp 249-250 °C; ¹H-NMR (DMSO-d₆) δ 10.21 (s, 1H, D₂O exchangeable), 7.90-7.95 (br s, 2H, D₂O exchangeable), 7.67 (m, 2H), 6.79 (d, 1H, *J* = 7.8 Hz), 1.61 (s, 6H); MS (ESI) *m*/*z* 222([M+H]⁺, 87%).

### EXAMPLE 4

### 6-(3-Chlorophenyl)-4,4-dimethyl-1,4-dihydrobenzo[d][1,3]oxazin-2-one

### (Procedure A)

A mixture of 6-bromo-4,4-dimethyl-1,4-dihydro-benzo[d][1,3]oxazin-2-one (1.5g, 5.9 mmol), 3-chlorophenyl boronic acid (1.83g, 11.7 mmol), tetrakis(triphenylphosphine)-palladium (0) (0.35g, 0.3 mmol), and sodium carbonate (2.48g, 23.4 mmol) in a mixture of DME and water (40 mL/10 mL) was degassed to remove the oxygen and then heated at 85 °C under a blanket of nitrogen for 3 hours. The reaction mixture was cooled to ambient temperature and quenched with a saturated aqueous ammonium chloride solution (20 mL). Ethyl acetate (50 mL) was added and the organic layer was separated. The aqueous layer was extracted with ethyl acetate (3x15 mL). The combined organic layers were washed with brine and dried with MgSO₄. The solvent was removed *in vacuo* and the residue was purified by silica gel flash chromatography (hexane:ethyl acetate/2:1) to afford 6-(3-chlorophenyl)-4,4-dimethyl-1,4-dihydrobenzo[d][1,3]oxazin-Z-one as a yellowish solid (1.4g, 82%): mp 158-159 °C; ¹H-NMR (DMSO-d₆) δ 10.31 (s, 1H, D₂O exchangeable), 7.75 (s, 1H), 7.61 (m, 3H), 7.46 (t, 1H, J= 7.9 Hz), 7.39 (dd, 1H, *J*= 7.0, 1.1 Hz), 6.96 (d, 1H, *J*= 8.6 Hz), 1.68 (s, 6H); Anal. Calc. For C₁₆H₁₄ClNO₂·0.1 H₂O: C, 66.37, H, 4.94, N, 4.84. Found: C, 66.14, H, 4.61, N, 4.71.

### EXAMPLE 5

### 6-(3-Bromo-5-fluorophenyl)-4,4-dimethyl-1,4-dihydrobenzo[d][1,3]oxazin-2-one

### (Procedure B)

A mixture of (1,4-dihydro-4,4-dimethyl-2-oxo-2H-3,1-benzoxazin-6-yl)boronic acid (2.22g, 10 mmol), 1,3-dibromo-5-fluorobenzene (3.05g, 12 mmol), tetrakis(triphenylphosphine)palladium (0) (0.6g, 0.52 mmol), and sodium carbonate (2.2g, 21 mmol) in a mixture of DME and water (70 mL/15 mL) was degassed to remove the oxygen and then heated at 85 °C under a blanket of nitrogen for 3 hours. The reaction mixture was cooled to ambient temperature and quenched with a saturated aqueous ammonium chloride solution (20 mL). Ethyl acetate (100 mL) was added and organic layer was separated. The aqueous layer was extracted with ethyl acetate (3x30 mL). The combined organic layers were washed with brine and dried with MgSO₄. The solvent was removed *in vacuo* and the residue was purified by a silica gel flash chromatography (hexane:ethyl acetate/1:1) to give 6-(3-bromo-5-fluorophenyl)-4,4-dimethyl-1,4-dihydrobenzo[d][1,3]oxazin-2-one as a white solid (1.4g, 40%): mp 182-183 °C; ¹H-NMR (DMSO-d₆) δ 10.36 (s, 1H, D₂O exchangeable), 7.78 (s, 1H), 7.58-7.65 (m, 3H), 7.49 (dd, 1H, *J =* 8.3, 1.8 Hz), 6.96 (d, 1H, *J =* 8.5 Hz), 1.69 (s, 6H); ¹⁹F-NMR (DMSO-d₆) δ -112.46 (m, 1F); MS (CI) *m*/*z* 352([M+H]⁺, 78%), 350([M+H]⁺, 75%). Anal. Calc. For C₁₆H₁₃BrFNO₂: C, 54.88, H, 3.74, N, 4.00. Found: C, 54.83, H, 3.82, N, 3.95.

### EXAMPLE 6

### 3-(4,4-Dimethyl-2-oxo-1,4-dihydro-2H-benzo [d] [1,3] oxazin-6-yl)-5-fluorobenzonitrile

A mixture of 6-(3-bromo-5-fluorophenyl)-4,4-dimethyl-2H-benz[d][1,3]oxazin-2-one (1g, 2.8 mmol), zinc cyanide (0.2g, 1.7 mmol), and tetrakis(triphenylphosphine)-palladium (0) (0.2g, 0.17 mmol) in dry DMF (20 mL) was degassed to remove oxygen and was then heated at 85 °C under a blanket of nitrogen for 6.5 hours. The reaction solution was cooled to room temperature and poured onto a cold saturated aqueous ammonium chloride solution (100 mL). The white precipitate appeared and was collected on a filter. The white solid was washed with distilled water (3x20 mL) and dissolved in a mixture of ethyl acetate (10 mL) and methanol (10 mL). The solution was applied on a pad of silica gel and eluted with a mixture of ethyl acetate and hexane (1:1). After solvent was removed, 3-(4,4-dimethyl-2-oxo-1,4-dihydro-2H-benzo[d][1,3]oxazin-6-yl)-5-fluorobenzonitrile was obtained as a white solid (0.7g, 84%): mp 253-254 °C; ¹H-NMR (DMSO-d₆) δ 10.4 (s, 1H, D₂O exchangeable), 8.13 (s, 1H), 7.92 (m, 1H), 7.82 (m, 1H), 7.73 (m, 2H), 6.98 (d, 1H, *J*= 8.2 Hz), 1.68 (s, 6H); ¹⁹F-NMR (DMSO-d₆) δ -112.25 (m, 1F); MS (EI) *m*/*z* 296(M⁺, 65%); Anal. Calc. For C₁₇H₁₃FN₂O₂: C, 68.91, H, 4.42, N, 9.45. Found: C, 68.85, H, 4.58, N, 9.14.

### EXAMPLE 7

### 4-(4,4-Dimethyl-2-oxo-1,4-dihydro-2H-benzo[d][1,3]oxazin-6-yl)-thiophene-2-carbonitrile

The product was prepared, from (1,4-dihydro-4,4-dimethyl-2-oxo-2H-3,1-benzoxazin-6-yl)boronic acid and 4-bromo-2-thiophenecarbonitrile according to the procedure outlined in Example 5, as a yellowish solid: mp 230-231 °C (decomposed); ¹H-NMR (CDCl₃) δ 8.32 (s, 1H, D₂O exchangeable), 7.83 (d, 1H, *J* = 1.5 Hz), 7.61 (d, 1H, *J =* 1.4 Hz), 7.43 (dd, 1H, *J=* 8.2, 1.9 Hz), 7.29 (d, 1H, *J =* 1.8 Hz), 6.85 (d, 1H, *J=* 8.2 Hz), 1. 78 (s, 6H); MS (EI) *mlz* 283 ([M-H]⁻, 100%); Anal. Calc. For C₁₅H₁₂N₂O₂S·0.2 H₂O: C, 62.57, H, 4.34, N, 9.73. Found: C, 62.48, H, 4.31, N, 9.64.

### EXAMPLE 8

### 6-(3-Chlorophenyl)-4,4-dimethyl-1,4-dihydro-benzo[d][1,3]oxazin-2-thione

A mixture of 6-(3-chlorophenyl)-4,4-dimethyl-1,4-dihydro-benzo[d]-[1,3]oxazin-2-one (0.15g, 0.5 mmol) and Lawesson's reagent (0.24g, 0.6 mmol) in anhydrous o-xylene was heated at reflux under nitrogen for 3 hours. The solvent was removed and the residue was purified by a flash chromatography (silica gel, hexane: ethyl acetate/6:1) to afford the title compound as a white solid (80 mg, 52%): mp 183-184 °C; ¹H-NMR (DMSO-d₆) δ 12.25 (s, 1H, D₂O exchangeable), 7.78 (t, 1H*, J =* 1.7 Hz), 7.63-7.70 (m, 3H), 7.49 (t, 1H, *J =* 7.8 Hz), 7.42 (d, 1H, *J=* 8.1 Hz), 7.12 (d, 8.8 Hz), 1.72 (s, 6H); MS (EI) *m*/*z* 303 (M⁺, 100%), 305 (M⁺, 32%); Anal. Calc. For C₁₆H₁₄ClNOS: C, 63.26, H, 4.64, N, 4.61. Found: C, 63.37, H, 4.62, N, 4.54.

### EXAMPLE 9

### 4-(4,4-Dimethyl-2-thioxo-1,4-dihydro-2H-benzo[d][1,3]oxazin-6-yl)-thiophene-2-carbonitrile

A mixture of 4-(4,4-dimethyl-2-oxo-1,4-dihydro-2H-benzo[d][1,3]oxazin-6-yl)-thiophene-2-carbonitrile (0.23g, 0.8 mmol) and Lawesson's reagent (0.38g, 0.96 mmol) in anhydrous o-xylene was heated to reflux under nitrogen for 3 hours. The solvent was removed *in vacuo* and the residue was purified by a flash chromatography (silica gel, hexane:ethyl acetate/3:1) to afford the title compound as a yellow solid (85 mg, 35%): mp 242-243 °C; ¹H-NMR (DMSO-d₆) δ 12.22 (s, 1H, D₂O exchangeable), 8.50 (d, 1H*, J=* 1.2 Hz), 8.37 (d, 1H, *J=* 1.0 Hz), 7.71 (m, 2H), 7.09 (d, 1H, *J=* 8.0 Hz), 1.69 (s, 6H); MS (APCI) *m*/*z* 301 ([M+H]⁺, 100%); Anal. Calc. For C₁₅H₁₂N₂OS₂: C, 59.97, H, 4.03, N, 9.33. Found: C, 59.67, H, 3.85, N, 9.14.

### EXAMPLE 10

### 6-Bromo-4,4-dimethyl-1,4-dihydro-benzo[d][1,3]oxazin-2-thione

The product was prepared, from 6-bromo-4,4-dimethyl-1,4-dihydro-benzo[d][1,3]oxazin-2-one and Lawesson's reagent using the procedure of Example 9, as a white solid: mp 221-222.5 °C; ¹H-NMR (CDCl₃) δ 9.38 (s, 1H, D₂O exchangeable), 7.42 (dd, 1H, *J=* 8.5, 2.1 Hz), 7.29 (d, 1H, *J=* 2.0 Hz), 6.76 (d, 1H, *J* = 8.4 Hz), 1.76 (s, 6H); MS (EI) *m*/*z* 272 ([M+H]⁺, 94%), 274 ([M+H]⁺, 100%).

### EXAMPLE 11

### 3-(4,4-Dimethyl-2-thioxo-1,4-dihydro-2H-benzo[d][1,3]oxazin-6-yl)-5-fluorobenzonitrile

The product was prepared, from 3-(4,4-dimethyl-2-oxo-1,4-dihydro-2H-benzo[d][1,3]oxazin-6-yl)-5-fluorobenzonitrile and Lawesson's reagent using the procedure of Example 9, as a yellow solid: 248-249 °C; ¹H-NMR (DMSO-d₆) δ 12.3 (s, 1H), 8.15 (bs, 1H), 8.02 (d, 1H, *J* = 10.48 Hz), 7.85-7.78 (m, 3H), 7.13 (d, 1H, *J*= 8.92 Hz), 1.71 (s, 6H); MS (APCI) *m*/*z* 313([M+H]⁺, 100%); Anal. Calc. For C₁₇H₁₃FN₂OS: C, 65.37, H, 4.19, N,8.97. Found: C, 65.26, H, 4.31, N, 8.61.

### EXAMPLE 12

### 3-(4,4-Dimethyl-2-oxo-1,4-dihydro-2H-benzo[d][1,3]oxazin-6-yl)-benzonitrile

A mixture of (1,4-dihydro-4,4-dimethyl-2-oxo-2H-3,1-benzoxazin-6-yl)boronic acid (2.22g, 10 mmol), 3-bromobenzonitrile (2.18 g, 12 mmol), tetrakis(triphenylphosphine)palladium (0) (0.6g, 0.52 mmol), and sodium carbonate (2.2g, 21 mmol) in a mixture of DME and water (70 mL/15 mL) was degassed to remove the oxygen and then heated at 85 °C under a blanket of nitrogen for 3 hours. The reaction mixture was cooled to ambient temperature and quenched with a saturated aqueous ammonium chloride solution (20 mL). Ethyl acetate (100 mL) was added and the organic layer was separated. The aqueous layer was extracted with ethyl acetate (3x30 mL). The combined organic layers were washed with brine and dried with MgSO₄. The solvent was *removed in vacuo* and the residue was purified by a silica gel flash chromatography (hexane:ethyl acetate/1:1) to give 3-(4,4-dimethyl-2-oxo-1,4-dihydro-2H-benzo[d][1,3]oxazin-6-yl)-benzonitrile as an off-white solid (0.7g, 25%): mp 236-237 °C; ¹H-NMR (DMSO-d₆) δ 10.34 (s, 1H, D₂O exchangeable), 8.21 (s, 1H), 8.02 (d, 1H, *J* = 8.1 Hz), 7. 79 (d, 1H, *J* = 7.7 Hz), 7.60-7.70 (m, 3H), 6.98 (d, 1H, *J*= 8.2 Hz), 1.71 (s, 6H); Anal. Calc. For C₁₇H₁₄N₂O₂·0.1 H₂O: C, 72.89, H, 5.11, N, 10.00. Found: C, 72.75, H, 5.05, N, 9.65.

### EXAMPLE 13

### 3-(4,4-Dimethyl-2-thioxo-1,4-dihydro-2H-benzo[d][1,3]oxazin-6-yl)-benzonitrile

A mixture of 3-(4,4-dimethyl-2-oxo-1,4-dihydro-2H-benzo[d][1,3]oxazin-6-yl)-benzonitrile (1 g, 3.6 mmol) and Lawesson's reagent (1.8 g, 4.3 mmol) in *o*-xylene (30 mL) was heated at reflux overnight. The reaction mixture was cooled to room temperature, poured into ethyl acetate (50 mL) and washed with 1 N HCl (2x20 mL). The organic layer was dried over sodium sulfate and concentrated. The residue was purified *via* flash chromatography (silica gel, 20% ethyl acetate/hexane) to give 3-(4,4-dimethyl-2-thioxo-1,4-dihydro-2H-benzo[d][1,3]oxazin-6-yl)-benzonitrile (0.21 g, 20%) as a white solid: mp 236-237°C; ¹H-NMR (DMSO-d*₆*) δ 12.3 (s, 1H), 8.24 (s, 1H), 8.05 (d, 1H, *J= 8.07* Hz), 7.82 (d, 1H, *J= 7.68* Hz), 7.74-7.64 (m, 3H), 7.14 (d, 1H, *J* = 8.78 Hz), 1.71 (s, 6H); MS (APCI) m/z 295 ([M + H]⁺, 100%); Anal. Calc. For C₁₇H₁₄N₂OS: C, 69.36, H, 4.79, N, 9.52. Found: C, 68.35, H, 4.91, N, 9.07

### EXAMPLE 14

### Potency in the relevant assays

The compounds of this invention were tested in the relevant assay as described below and their potency are in the range of 0.01 nM to 5 µM in the *in vitro* assays and 0.001 to 300 mg/kg in the *in vivo* assays. The selected examples are listed in Table 1 below.

**Table 1**

| | | | | | |
|---|---|---|---|---|---|
| 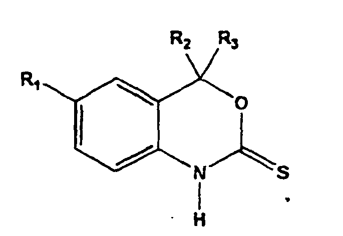 | | | | | |

| Compound | R₁ | R₂ | R₃ | hPR CV-1 EC₅₀ (nM) | Ovulation Inhibition ED₁₀₀ (mg/kg) |
|---|---|---|---|---|---|
| 1 | 3-chlorophenyl | Me | Me | 0.65 | ND* |
| | | | | | |
| 2 | 4-(2-cyanothiophene) | Me | Me | 0.3 | 1 |
| | | | | | |
| 3 | 3-cyano-5-fluoro-phenyl | Me | Me | 5.1 | ND |
| | | | | | |
| 4 | 3-cyanophenyl | Me | Me | 0.4** | |

| | | | | | |
|---|---|---|---|---|---|
| * ND, not determined; ** alkaline phosphatase data. | | | | | |

### (1) T47D cell proliferation assay

The objective of this assay is the determination of progestational and antiprogestational potency by using a cell proliferation assay in T47D cells. A compound's effect on DNA synthesis in T47D cells is measured. The materials and methods used in this assay are as follows.
a. Growth medium: DMEM:F12 (1:1) (GIBCO, BRL) supplemented with 10% (v/v) fetal bovine serum (not heat-inactivated), 100U/ml penicillin, 100mg/ml streptomycin, and 2 mM GlutaMax (GIBCO, BRL).
b. Treatment medium Minimum Essential Medium (MEM) (#51200-038GIBCO, BRL) phenol red-free supplemented with 0.5% charcoal stripped fetal bovine serum, 100U/ml penicillin, 200 mg/ml streptomycin, and 2 mM GlutaMax (GIBCO, BRL).
c. Cell culture
   Stock T47 D cells are maintained in growth medium For BrdU incorporation assay, cells are plated in 96-well plates (Falcon, Becton Dickinson Labware) at 10,000 cells/well in growth medium After overnight incubation, the medium is changed to treatment medium and cells are cultured for an additional 24 hr before treatment Stock compounds are dissolved in appropriate vehicle (100% ethanol or 50% ethanol/50% DMSO), subsequently diluted in treatment medium and added to the cells. Progestin and antiprogestin reference compounds are run in full dose-response curves. The final concentration of vehicle is 0.1 %. In control wells, cells receive vehicle only. Antiprogestins are tested in the presence of 0.03 nM trimegestone, the reference progestin agonist. Twenty-four hours after treatment, the medium is discarded and cells are labeled with 10 mM BrdU (Amersham Life Science, Arlington Heights, IL) in treatment medium for 4 hr.
d. Cell Proliferation Assay
   At the end of BrdU labeling, the medium is removed and BrdU incorporation is measured using a cell proliferation ELISA kit (#RPN 250, Amersham Life Science) according to manufacturer's instructions. Briefly, cells are fixed in an ethanol containing fixative for 30 min, followed by incubation in a blocking buffer for 30 min to reduce background. Peroxidase-labeled anti-BrdU antibody is added to the wells and incubated for 60 min. The cells are rinsed three times with PBS and incubated with 3,3'5,5'-tetramethylbenzidine (TMB) substrate for 10-20 min depending upon the potency of tested compounds. Then 25 µl of 1 M sulfuric acid is added to each well to stop color reaction and optical density is read in a plate reader at 450 nm within 5 min.
e. Analysis of Results:
   Square root-transformed data are used for analysis of variance and nonlinear dose response curve fitting for both agonist and antagonist modes. Huber weighting is used to downweight the effects of outliers. EC₅₀ or IC₅₀ values are calculated from the retransformed values. JMP software (SAS Institute, Inc.) is used for both one-way analysis of variance and non-linear dose response analyses in both single dose and dose response studies.
f. Reference Compounds:
   Trimegestone and medroxyprogesterone acetate (MPA) are reference progestins and RU486 is the reference antiprogestin. All reference compounds are run in full dose-response curves and the EC₅₀ or IC₅₀ values are calculated.

**Table 2. Estimated EC₅₀, standard error (SE), and 95% confidence intervals (CI) for individual studies**

| | EC₅₀ | | | 95% CI | |
|---|---|---|---|---|---|
| Compound | Exp | (nM) | SE | lower | upper |
| Trimegestone | 1 | 0.017 | 0.003 | 0.007 | 0.040 |
| | 2 | 0.014 | 0.001 | 0.011 | 0.017 |
| | 3 | 0.019 | 0.001 | 0.016 | 0.024 |
| | | | | | |
| MPA | 1 | 0.019 | 0.001 | 0.013 | 0.027 |
| | 2 | 0.017 | 0.001 | 0.011 | 0.024 |

**Table 3. Estimated IC₅₀, standard error, and 95% confident interval for the antiprogestin, RU486**

| | IC₅₀ | | | 95% CI | |
|---|---|---|---|---|---|
| Compound | Exp | (nM) | SE | lower | upper |
| RU486 | 1 | 0.011 | 0.001 | 0.008 | 0.014 |
| | 2 | 0.016 | 0.001 | 0.014 | 0.020 |
| | 3 | 0.018 | 0.001 | 0.014 | 0.022 |

EC₅₀: Concentration of a compound that gives half-maximal increase in BrdU incorporation with SE; IC₅₀: Concentration of a compound that gives half-maximal decrease in 0.1 trimegestone induced BrdU incorporation with SE

### (2) Rat decidualization assay The objective of this procedure is used to evaluate the effect of progestins and antiprogestins on rat uterine decidualization and compare the relative potencies of various test compounds. The materials and methods used in this assay are as follows.

a. Methods: Test compounds are dissolved in 100% ethanol and mixed with corn oil (vehicle). Stock solutions of the test compounds in oil (Mazola™) are then prepared by heating (-80 °C) the mixture to evaporate ethanol. Test compounds are subsequently diluted with 100% corn oil or 10% ethanol in corn oil prior to the treatment of animals. No difference in decidual response was found when these two vehicles were compared.
b. Animals (RACUC protocol #5002)
   Ovariectomized mature female Sprague-Dawley rats (~60-day old and 230g) are obtained from Taconic (Taconic Farms, NY) following surgery. Ovariectomy is performed at least 10 days prior to treatment to reduce circulating sex steroids. Animals are housed under 12 hr light/dark cycle and given standard rat chow and water ad libitum.
c. Treatment
   Rats are weighed and randomly assigned to groups of 4 or 5 before treatment. Test compounds in 0.2 ml vehicle are administered by subcutaneous injection in the nape of the neck or by gavage using 0.5 ml. The animals are treated once daily for seven days. For testing antiprogestins, animals are given the test compounds and a EC₅₀ dose of progesterone (5.6 mg/kg) during the first three days of treatment. Following decidual stimulation, animals continue to receive progesterone until necropsy four days later.
d. Dosing
   Doses are prepared based upon mg/kg mean group body weight. In all studies, a control group receiving vehicle is included. Determination of dose-response curves is carried out using doses with half log increases (e.g. 0.1, 0.3, 1.0, 3.0 mg/kg).
e. Decidual induction
   Approximately 24 hr after the third injection, decidualization is induced in one of the uterine horns by scratching the antimesometrial luminal epithelium with a blunt 21 G needle. The contralateral horn is not scratched and serves as an unstimulated control. Approximately 24 hr following the final treatment, rats are sacrificed by CO₂ asphyxiation and body weight measured. Uteri are removed and trimmed of fat. Decidualized (D-horn) and control (C-horn) uterine horns are weighed separately.
f. Analysis of Results:
   The increase in weight of the decidualized uterine horn is calculated by D-hom/C-hom and logarithmic transformation is used to maximize normality and homogeneity of variance. The Huber M-estimator is used to down weight the outlying transformed observations for both dose-response curve fitting and one-way analysis of variance. JMP software (SAS Institute, Inc.) is used for both one-way ANOVA and non-linear dose-response analyses.
g. Reference Compounds:
   All progestin reference compounds were run in full dose-response curves and the EC₅₀ for uterine wet weight were calculated.

**Table 4. Estimated EC₅₀, standard error (SE), and 95% confidence intervals for individual studies**

| | EC₅₀ | | | 95% CI | |
|---|---|---|---|---|---|
| Compound | Exp | (mg/kg, s.c.) | SE | lower | upper |
| Progesterone | 1 | 5.50 | 0.77 | 4.21 | 7.20 |
| | 2 | 6.21 | 1.12 | 4.41 | 8.76 |
| | | | | | |
| 3-Ketodesogestrel | 1 | 0.11 | 0.02 | 0.07 | 0.16 |
| | 2 | 0.10 | 0.05 | 0.11 | 0.25 |
| | 3 | 0.06 | 0.03 | 0.03 | 0.14 |
| | | | | | |
| Levonorgestrel | 1 | 0.08 | 0.03 | 0.04 | 0.16 |
| | 2 | 0.12 | 0.02 | 0.09 | 0.17 |
| | 3 | 0.09 | 0.02 | 0.06 | 0.13 |
| | 4 | 0.09 | 0.02 | 0.06 | 0.14 |
| | | | | | |
| MPA | 1 | 0.42 | 0.03 | 0.29 | 0.60 |
| | 2 | 0.39 | 0.05 | 0.22 | 0.67 |
| | 3 | 0.39 | 0.04 | 0.25 | 0.61 |

**Table 5. Estimated average EC₅₀, standard error, and 95% confidence intervals for dose-response curves of 3 reference compounds**

| | EC50 | | 95% CI | |
|---|---|---|---|---|
| Compound | (mg/kg, s.c.) | SE | lower | upper |
| Progesterone | 5.62 | 0.62 | 4.55 | 7.00 |
| 3-Ketodesogestrel | 0.10 | 0.02 | 0.07 | 0.14 |
| Levonorgestrel | 0.10 | 0.01 | 0.08 | 0.12 |

**Table 6. Estimated IC₅₀, standard error, and 95% confident interval for the antiprogestin, RU 486**

| | IC₅₀ | | | 95% CI | |
|---|---|---|---|---|---|
| Compound | Exp. | (mg/kg, p.o.) | SE | lower | upper |
| RU 486 | 1 | 0.21 | 0.07 | 0.05 | 0.96 |
| | 2 | 0.14 | 0.02 | 0.08 | 0.27 |

Concentration: Compound concentration in assay(default-mg/kg body weight)
Route of administration: Route the compound is administered to the animals
Body weight: Mean total animal body weight (default-kg)
D-horn: Wet weight of decidualized uterine horn (default-mg)
C-hom: Wet weight of control uterine horn (default-mg)
Decidual response: [(D-C)/C]x100%
Progestational activity: Compounds that induce decidualization significantly (p<0.05) compared to vehicle control are considered active
Antiprogestational activity: Compounds that decrease EC₅₀ progesterone induced decidualization significantly (p<0.05)
EC₅₀ for uterine weight: Concentration of compound that gives half-maximal increase in decidual response (default-mg/kg)
IC₅₀ for uterine weight: Concentration of compound that gives half-maximal decrease in EC₅₀ progesterone induced decidual response (default-mg/kg)

### (3) PRE-luciferase assay in CV-1 cells

The object of this assay is to determine a compound's progestational or antiprogestational potency based on its effect on PRE-luciferase reporter activity in CV-1 cells co-transfected with human PR and PRE-luciferase plasmids. The materials methods used in the assay are as follows.
a. Medium: The growth medium was as follows: DMEM (BioWhittaker) containing 10% (v/v) fetal bovine serum (heat inactivated), 0.1 mM MEM non-essential amino acids, 100U/ml penicillin, 100mg/ml streptomycin, and 2 mM GlutaMax (GIBCO, BRL). The experimental medium was as follows: DMEM (BioWhittaker), phenol red-free, containing 10% (v/v) charcoal-stripped fetal bovine serum (heat-inactivated), 0.1 mM MEM non-essential amino acids, 100U/ml penicillin, 100mg/ml streptomycin, and 2 mM GlutaMax (GIBCO, BRL).
b. Cell culture, transfection, treatment, and luciferase assay
   Stock CV-1 cells are maintained in growth medium. Co-transfection is done using 1.2x10⁷ cells, 5 mg pLEM plasmid with hPR-B inserted at Sph1 and BamH1 sites, 10 mg pGL3 plasmid with two PREs upstream of the luciferase sequence, and 50 mg sonicated calf thymus DNA as carrier DNA in 250 ml. Electroporation is carried out at 260 V and 1,000 mF in a Biorad Gene Pulser II. After electroporation, cells are resuspended in growth medium and plated in 96-well plate at 40,000 cells/well in 200 µl. Following overnight incubation, the medium is changed to experimental medium Cells are then treated with reference or test compounds in experimental medium. Compounds are tested for antiprogestational activity in the presence of 3 nM progesterone. Twenty-four hr. after treatment, the medium is discarded, cells are washed three times with D-PBS (GIBCO, BRL). Fifty µl of cell lysis buffer (Promega, Madison, WI) is added to each well and the plates are shaken for 15 min in a Titer Plate Shaker (Lab Line Instrument, Inc.). Luciferase activity is measured using luciferase reagents from Promega.
c. Analysis of Results:
   Each treatment consists of at least 4 replicates. Log transformed data are used for analysis of variance and nonlinear dose response curve fitting for both agonist and antagonist modes. Huber weighting is used to downweight the effects of outliers. EC₅₀ or IC₅₀ values are calculated from the retransformed values. JMP software (SAS Institute, Inc.) is used for both one-way analysis of variance and non-linear response analyses.
d. Reference Compounds:
   Progesterone and trimegestone are reference progestins and RU486 is the reference antiprogestin. All reference compounds are run in full dose-response curves and the EC₅₀ or IC₅₀ values are calculated.

**Table 8. Estimated EC₅₀, standard error (SE), and 95% confidence intervals (CI) for reference progestins from three individual studies**

| | EC50 | | | 95% CI | |
|---|---|---|---|---|---|
| Compound | Exp. | (nM) | SE | lower | upper |
| Progesterone | 1 | 0.616 | 0.026 | 0.509 | 0.746 |
| | 2 | 0.402 | 0.019 | 0.323 | 0.501 |
| | 3 | 0.486 | 0.028 | 0.371 | 0.637 |
| | | | | | |
| Trimegestone | 1 | 0.0075 | 0.0002 | 0.0066 | 0.0085 |
| | 2 | 0.0081 | 0.0003 | 0.0070 | 0.0094 |
| | 3 | 0.0067 | 0.0003 | 0.0055 | 0.0082 |

**Table 9. Estimated IC₅₀, standard error (SE), and 95% confident interval (CI) for the antiprogestin, RU486 from three individual studies**

| | IC 50 | | | 95% CI | |
|---|---|---|---|---|---|
| Compound | Exp. | (nM) | SE | lower | upper |
| RU486 | 1 | 0.028 | 0.002 | 0.019 | 0.042 |
| | 2 | 0.037 | 0.002 | 0.029 | 0.048 |
| | 3 | 0.019 | 0.001 | 0.013 | 0.027 |

Progestational activity: Compounds that increase PRE-luciferase activity significantly (p<0.05) compared to vehicle control are considered active.
Antiprogestational activity: Compounds that decrease 3 nM progesterone induced
PRE-luciferase activity significantly (p<0.05). EC₅₀: Concentration of a compound that gives half-maximal increase PRE-luciferase activity (default-nM) with SE.
IC₅₀: Concentration of a compound that gives half-maximal decrease in 3 nM progesterone induced PRE-luciferase activity (default-nM) with SE.

### EXAMPLE 15

### 6-(3-fluorophenyl)-4-methyl-1,4-dihydro-2H-3,1-benzoxazine-2-thione

4-Amino-3'-fluoro[1,1'-biphenyl]-3-carbonitrile was prepared from 3-fluorophenyl boronic acid and 2-amino-5-bromobenzonitrile according to the procedure of Example 4. A solution of 4-amino-3'-fluoro[1,1'-biphenyl]-3-carbonitrile (6.65g, 31.3 mmol) in anhydrous THF (100 mL) was treated drop wise at room temperature under nitrogen with methylmagnesium bromide (3.0 M in ether, 21 mL, 63 mmol). The reaction mixture was then heated at gentle reflux for 1.5 hours, cooled to room temperature, and treated with 3N aqueous hydrogen chloride solution (30 mL). The resulted mixture was heated at reflux for 3 hours, cooled to ambient temperature, and adjusted to a pH of 5-6 by the addition of a saturated aqueous sodium carbonate solution. Ethyl acetate (100 mL) was added, the organic layer was separated and the aqueous layer was extracted with ethyl acetate (3x50 mL). The combined organic layers were dried (MgSO₄) and evaporated. The residue was purified by a silica gel chromatography (hexane:ethyl acetate/3:1) to afford 1-(4-arnino-3'-fluoro[1,1'-biphenyl]-3-yl)ethanone (3.1g, 43%): mp 156-157 °C.

A solution of 1-(4-amino-3'-fluoro[1,1'-biphenyl]-3-yl)ethanone (3g, 13 mmol) in anhydrous methanol (60 mL) was then treated at room temperature under nitrogen with sodium borohydride in a portion wise manner. After addition, the reaction mixture was stirred for 4 hours, treated with a saturated solution of aqueous ammonium sulfate (50 mL) and ethyl acetate (100 mL). The organic layer was separated, dried (MgSO₄), and evaporated *in vacuo.* The residue was purified on a silica gel chromatography (hexane:ethyl acetate/3:1) to yield 1-(4-amino-3'-fluoro[1,1'-biphenyl]-3-yl)ethanol as a white solid (2g, 67%): mp 136-137 °C.

A mixture of the above alcohol (0.2g, 0.87 mmol) and triphosgene in anhydrous THF (20 mL) was stirred at room temperature under nitrogen. After 15 minutes, the mixture was treated with a saturated aqueous sodium bicarbonate solution (30 mL) and ethyl acetate (40 mL). The organic layer was separated, dried (MgSO₄), and evaporated to give 6-(3-fluorophenyl)-4-methyl-1,4-dihydro-2H-3,1-benzoxazin-2-one as a white solid (0.18g, 81%): mp 160-161 °C ; ¹H-NMR (DMSO-*d₆*) δ 10.31 (s, 1H), 7.62 (dd, 1H, *J=* 8.2, 1.9 Hz), 7.57 (s, 1H), 7.44-7.53 (m, 3H), 7.13-7.20 (m 1H), 6.97 (d, 1H, *J=* 8.2 Hz), 5.57 (q, 1H, *J=* 6.6 Hz), 1.63 (d, 3H, *J=* 6.6 Hz). MS (ESI) *m*/*z* 256 [M - H]⁻.

A solution of 6-(3-fluorophenyl)-4-methyl-1,4-dihydro-2H-3,1-benzoxazin-2-one (0.15g, 0.58 mmol) in toluene was treated with Lawesson's reagent according to the procedure in example 9 to yield 6-(3-fluorophenyl)-4-methyl-1,4-dihydro-2H-3,1-benzoxazin-2-thione (0.08g, 50%) as an off-white solid (0.08g, 50%): mp 173-174 °C; ¹H-NMR (DMSO-*d₆*) δ 12.27 (s, 1H), 7.70 (dd, 1H, *J =* 8.2, 2.0 Hz), 7.62 (s, 1H), 7.46-7.56 (m, 3H), 7.15-7.22 (m, 1H), 7.11 (d, 1H, *J=* 8.3 Hz), 5.64 (q, 1H, *J* = 6.6 Hz), 1.67 (d, 3H, *J=* 6.6 Hz); MS (ESI) *mlz* 272 [M - H]⁻.

### EXAMPLE 16

### 5-(4,4-Dimethyl-2-thioxo-1,4-dihydro-2H-3,1-benzoxazin-6-yl)-4-methylthiophene-2-carbonitrile

5-(4, 4-Dimethyl-2-oxo-1,4-dihydro-2H-benzo[d][1,3]oxazin-6-yl)-4-methylthiophene-2-carbonitrile was prepared, according to the procedure in Example 5 using (1,4-dihydro-4,4-dimethyl-2-oxo-2H-3,1-benzoxazin-6-yl)boronic acid and 5-bromo-4-methyl-2-thiophenecarbonitrile, as an off-white solid: mp 195-200 °C; ¹H-NMR (DMSO-d₆) δ 10.2 (s, 1H), 8.32 (s, 1H), 7.41-7.44 (m, 2H), 7.01 (d, 1H, *J* = 8.8 Hz), 2.28 (S, 3H), 1.64 (S, 6H); MS (APCI) *m*/*z* 299 [M+H]⁺; Anal. Calc. For C₁₆H₁₄N₂O₂S; C, 64.41; H, 4.75; N, 8.89. Found: C, 64.64; H, 4.62; N, 9.39.

To a solution of 5-(4,4-dimethyl-2-oxo-1,4-dihydro-2H-3,1-benzoxazin-6-yl)-4-methylthiophene-2-carbonitrile (5g, 16.7 mmol) in anhydrous toluene was added, at room temperature under a blanket of nitrogen, Lawesson's reagent (6g, 14.8 mmol). The reaction mixture was heated at reflux for 2 hrs, allowed to cool to room temperature, and solvent was removed *in vacuo.* The residue was purified by a silica gel column chromatography (THF:hexane/1:3) to yield the title compound as a yellowish solid (2.4g, 46%): mp 211-212 °C; ¹H-NMR (DMSO-d₆) δ 12.3 (s, 1H), 7.88 (s, 1H), 7.46-7.52 (m, 2H), 7.16 (d, 1H, *J* = 8.3 Hz), 2.30 (S, 3H), 1.68 (S, 6H); MS (ESI) *m*/*z* 313 [M-H]-; Anal. Calc. For C₁₆H₁₄N₂OS₂; C, 61.12; H, 4.49; N, 8.91. Found: C, 60.91; H, 4.48; N, 8.66.

### EXAMPLE 17

### tert-Butyl 2-cyano-5-(4,4-dimethyl-2-thioxo-1,4-dihydro-2H-3,1-benzoxazin-6-yl)-1H-pyrrole-1-carboxylate

A solution of 6-bromo-4,4-dimethyl-1,4-dihydro-benzo[d][1,3]oxazin-2-one (5.0 g, 20 mmol) and tetrakis(triphenylphosphine)palladium(0) (580 mg, 0.5 mmol) in toluene (200 mL) was stirred under a flow of nitrogen for 25 min. To the solution was added sequentially 1-*tert*-butoxycarbonylpyrrole-2-boronic acid (8.24 g, 39 mmol) in absolute ethanol (50 mL) and potassium carbonate (5.39 g, 39 mmol) in water (50 mL). The mixture was heated to 80 °C for 16 h and allowed to cool. The reaction mixture was poured into an aqueous saturated sodium bicarbonate solution (200 mL) and extracted with ethyl acetate (3 x 200 mL). The organic layers were combined, washed with water (200 mL) and brine (100 mL) and dried over magnesium sulfate. The solution was filtered, concentrated *in vacuo,* and the residue was purified by flash column chromatography on silica gel (30% ethyl acetate/hexane) to give 2-(4,4-dimethyl-2-oxo-1,4-dihydro-2H-benzo [d] [1,3] oxazin-6-yl)-pyrrole-1-carboxylic acid tert-butyl ester (4.0 g, 58%) as a tan solid, mp 172-173 °C.

To a solution of 2-(4,4-dimethyl-2-oxo-1,4-dihydro-2H-benzo [d] [1,3] oxazin-6-yl)-pyrrole-1-carboxylic acid tert-butyl ester (2.0 g, 5.8 mmol) in THF (anhydrous, 50 mL) at -78 °C was added chlorosulfonyl isocyanate (0.66 mL, 6.7 mmol). After 90 min, DMF (9 mL, 116 mmol) was added and the reaction was allowed to warm to room temperature. The reaction mixture was poured into water (50 mL) and the product was extracted with ethyl acetate (2 x 50 mL). The organic layers were combined, washed with brine (50 mL), dried over magnesium sulfate, filtered and concentrated *in vacuo.* Purification via flash column chromatography on silica gel (30% ethyl acetate/hexane) gave 2-(4,4-dimethyl-2-oxo-1,4-dihydro-2H-benzo [d] [1,3] oxazin-6-yl)-5-cyano-pyrrole-1-carboxylic acid tert-butyl ester (1.1 g, 52%) as a white powder, mp 165-167 °C; ¹H NMR (DMSO-d₆) δ 1.36 (s, 9H), 1.61 (s, 6 H), 6.44 (d, 1H, *J* = 3.7 Hz), 6.92 (d, 1 H, *J* = 8.2 Hz), 7.27-7.32 (m, 2H), 7.36 (`d', 1 H, *J*= 1.5 Hz), 10.36 (s, 1 H); MS (EI) *m*/*z* 367 [M]⁺.

To 2-(4,4-dimethyl-2-oxo-1,4-dihydro-2H-benzo [d] [1,3] oxazin-6-yl)-5-cyano-pyrrole-1-carboxylic acid tert-butyl ester (1.3 g, 35 mmol, 1 eq) in toluene (130 mL) was added Lawesson's reagent (1.58 g, 3.9 mmol, 1.1 eq) and the reaction mixture was heated to 80 °C for 2 h. The solvent was removed *in vacuo* and the residue was dissolved in acetone/dichloromethane and adsorbed onto silica gel. Purification by flash column chromatography (10% ethyl acetate/hexane) gave the product (0.51 g, 38%) as yellow crystals. ¹H NMR (DMSO-d₆) δ1.35 (s, 9 H), 1.64 (s, 6 H), 6.47 (d, 1 H*, J =* 3.6 Hz), 7.07 (d, 1 H, *J* = 8.1 Hz), 7.32 (d, 1 H, *J =* 3.6 Hz), 7.37 (dd, 1 H, *J*= 1.8, 8.1 Hz), 7.43 (d, 1 H, *J* = 1.8 Hz), 12.28 (s, 1 H); MS (ESI) [M-H]⁻ = 382; Anal. Calcd. For C₂₀H₂₁N₃O₃S: C, 62.64; H, 5.52; N, 10.96. Found: C, 62.53; H, 5.6; N, 10.87.

### EXAMPLE 18

### 5-(4,4-Dimethyl-2-thioxo-1,4-dihydro-2H-3,1-benzoxazin-6-yl)-1H-pyrrole-2-carbonitrile

To a solution of tert-butyl 2-cyano-5-(4,4-dimethyl-2-thioxo-1,4-dihydro-2H-3,1-benzoxazin-6-yl)-1H-pyrrole-1-carboxylate (0.5 g, 1.3 mmol, 1 eq) in THF (5 mL) was added NaOEt (0.27 g, 3.9 mmol, 3 eq) in EtOH (5 mL) and the reaction was heated to 80°C for 2 h. The solvents were *removed in vacuo* and the residue partitioned between ethyl acetate (100 mL) and water (100 mL). The layers were separated and the aqueous layer was extracted with ethyl acetate (100 mL). The organic layers were combined, washed with brine (50 mL), dried over magnesium sulfate, filtered and concentrated *in vacuo.* Purification by flash column chromatography (10% ethyl acetate/hexane) gave the title compound (0.27g, 73%) as a brown powder, mp 261-262 °C. ¹H NMR (DMSO-*d₆*) δ 1.68 (s, 6 H), 6.72 - 6.73 (m, 1 H), 6.99 - 7.01 (m, 1 H), 7.06 (d, 1 H, *J*= 7.9 Hz), 7.66 - 7.70 (m, 2 H), 12.26 (s, 1 H), 12.62 (s, 1 H); MS (ESI) [M-H]⁻ = 282; Anal. Calcd. For C₁₅H₁₃N₃OS: C, 63.58; H, 4.62; N, 14.83. Found: C, 63.25; H, 4.78; N, 15.11.

### EXAMPLE 19

### [6-(4,4-dimethyl-2-thioxo-1,4-dihydro-2H-3,1-benzoxazin-6-yl)pyridin-2 yl]acetonitrile

[6-(4,4-dimethyl-2-oxo-1,4-dihydro-2H-3,1-benzoxazin-6-yl)pyridin-2-yl]acetonitrile was prepared, according to the procedure of Example 5 using (1,4-dihydro-4,4-dimethyl-2-oxo-2H-3,1-benzoxazin-6-yl)boronic acid and (6-bromo-2-pyridyl)acetonitrile (*J. Org. Chem.* 1988, *53*, 786-790), as an off-white solid: mp 210-212.5 °C; ¹H NMR (DMSO-*d₆*) δ1.68 (s, 6 H), 4.27 (s, 2 H), 7.00 (d, 1 H, *J =* 8.3 Hz), 7.34 (d, 1 H, *J =* 7.1 Hz), 7.89 - 7.96 (m, 2 H), 8.00 - 8.05 (m, 2 H), 10.42 (s, 1H); MS (ESI) [M-H]⁻ = 292; Anal. Calcd. For C₁₇H₁₅N₃O₂: C, 69.61; H, 5.15; N, 14.33. Found: C, 68.49; H, 5.19; N, 13.74.

To [6-(4,4-dimethyl-2-oxo-1,4-dihydro-2H-3,1-benzoxazin-6-yl)pyridin-2-yl]acetonitrile (80 mg, 0.27 mmol, 1 eq) in *p*-xylene (10 mL) was added Lawesson's reagent (55 mg, 0.14 mmol, 0.5 eq) and the reaction was heated to reflux for 2 hours. The reaction was cooled to room temperature and adsorbed onto silica gel. Purification by flash column chromatography (50% ethyl acetate/hexane) on silica gel gave the title compound (40 mg, 48%) as an off-white solid: mp 215-217 °C; ¹H NMR (*d₆*-DMSO, 300 MHz) δ 1.71 (s, 6 H), 4.28 (s, 2 H), 7.15 (d, 1 H, *J* = 8.3 Hz), 7.36 (d, 1H, *J* = 7.3 Hz), 7.89 - 7.99 (m, 2 H), 8.04 - 8.11 (m, 2 H), 12.32 (s, 1 H); MS (ESI) [M-H]⁻ = 308; Anal. calcd. for C₁₇H₁₅N₃OS: C, 66.00; H, 4.89; N, 13.58. Found: C, 64.43; H, 4.65; N, 12.95.

### EXAMPLE 20

### 5-(4,4-Dimethyl-2-thioxo-1,4-dihydro-2H-3,1-benzoxazin-6-yl-1-methyl-1H-pyrrole-2-carbonitrile

2-(4,4-Dimethyl-2-oxo-1,4-dihydro-2H-benzo [d] [1,3] oxazin-6-yl)-5-cyanopyrrole-1-carboxylic acid *tert*-butyl ester (1 g, 2.7 mmol) was placed in a 25 mL round bottomed flask stoppered with a rubber septum and equipped with nitrogen inlet and a needle to allow gaseous outflow. A vigorous flow of nitrogen was maintained as the flask was placed in an oil bath and heated to 160 °C. After 20 min at this temperature, the flask was removed from the oil bath and allowed to cool. The yellow residue was washed into a larger flask with dichloromethane/ethyl acetate and adsorbed onto a small amount of silica gel. Purification by flash column chromatography on silica gel (40% ethyl acetate/hexane) gave of 5-(4,4-dimethyl-2-oxo-1,4-dihydro-2H-3,1-benzoxazin-6-yl)-1H-pyrrole-2-carbonitrile (340 mg, 47%) as a yellow powder: mp 241-242 °C; ¹H NMR (d₆-DMSO, 300 MHz) δ 1.65 (s, 6 H), 6.67 (d, 1 H, *J*= 3.9 Hz), 6.91 (d, 1 H, *J* = 8.3 Hz), 6.98 (d, 1 H, *J* = 3.9 Hz), 7.61 (dd, 1 H, *J* = 1.8, 8.3 Hz), 7.65 ('d', 1 H, *J* = 1.6 Hz), 10.32 (s, I H), 12.54 (bs, 1 H); MS (EI) *m*/*z* 267 M⁺; Anal. Calcd. For C₁₅H₁₃N₃O₂: C, 67.41; H, 4.90; N, 15.72. Found: C, 67.19; H, 4.96; N, 15.35.

To a solution of 5-(4,4-dimethyl-2-oxo-1,4-dihydro-2H-3,1-benzoxazin-6-yl)-1H-pyrrole-2-carbonitrile (1 eq, 71 mg, 0.27 mmol) in dimethylformamide (0.5 mL) was added potassium carbonate (5 eq, 0.18 g, 0.1.35 mmol). After 10 min, iodomethane (3 eq, 05 mL, 0.81 mmol) was added and the suspension was stirred for 2 hours, poured into water (5 mL) and the product was extracted with ethyl acetate (3 x 5 mL). The layers were then separated, the aqueous layer extracted with ethyl acetate (3 x 10 mL) and the combined organic layer was washed with brine, dried over MgSO₄ and purified by flash column chromatography on silica gel eluting with 30% ethyl acetate/hexane to give 5-(4,4-danethyl-2-oxo-1,4-dihydro-2H-3,1-benaoxazin-6-yl)-1-methyl-1H-pyrrole-2-carbonitrile (30 mg, 41%) as a white solid; MS (ES) *m*/*z* 280 (M-H)⁻; Anal. Calcd For C₁₆H₁₅N₃O₂: C, 68.3, H, 5.37, N, 14.9. Found, C, 68.4, H, 5.51, N, 14.6.

To a solution of 5-(4,4-dimethyl-2-oxo-1,4-dihydro-2H-3,1-benzoxazin-6-yl)-1-methyl-1H-pyrrole-2-carbonitrile (6.0 g, 22 mmol, 1 eq) in toluene (600 mL) was added Lawesson's reagent (5.9 g, 15 mmol, 0.65 eq) and the reaction was heated to 80 °C for 2 hours. The reaction was cooled to room temperature, poured into water (200 mL) and extracted with ethyl acetate (2 x 200 mL). The organic layers were combined, washed with brine (50 mL), dried over magnesium sulfate, filtered and concentrated *in vacuo.* Purification by flash column chromatography (5-10% ethyl acetate/hexane) gave the title compound (2.0 g, 31 %) as a slightly yellow solid: mp 225-228 °C. ¹H NMR (d₆-DMSO, 300 MHz) δ1.67 (s, 6 H), 3.72 (s, 3 H), 6.37 (dd, 1 H, *J* = 0.8,4.1 Hz), 7.04 (dd, 1 H, *J* = 0.8, 4.1 Hz), 7.13 (m, 1 H), 7.47 (m, 2 H), 12.30 (s, 1 H); MS (ESI) [M-H]⁻ = 296; Anal. calcd. for C₁₆H₁₅N₃OS: C, 64.62; H, 5.08; N, 14.13. Found: C, 64.7; H, 5.12; N, 14.17.

### EXAMPLE 21

### 5-(4.4-dimethyl-2-thioxo-1,4-dihydro-2H-3,1-benzoxazin-6-yl)-1H-pyrrole-2-carbothiamide

To 4,4-dimethyl-6-(5-cyano-1H-pyrrol-2-yl)-1,4-dihydrobenzo [d][1,3]oxazin-2-one (6.0 g, 22.5 mmol) in *p*-xylene (100 mL) was added Lawesson's reagent (5.9 g, 14.6 mmol, 0.65 eq) and the reaction was heated to reflux for 3 hours. The reaction was cooled to room temperature, concentrated *in vacuo,* and adsorbed onto silica gel. Purification by flash column chromatography (30% ethyl acetate/hexane) on silica gel gave the title compound (1.2 g, 17%) as a yellow powder: ¹H NMR (DMSO-*d*₆). □1.69 (s, 6 H), 6.65 (dd, 1H*, J =* 2.2, 3.8 Hz), 6.98 (dd, 1 H, *J =* 2.2, 3.8 Hz), 7.03 (d, 1H, *J*= 8.2 Hz), 7.69 (dd, 1 H, *J*= 1.6, 8.2 Hz), 7.81 (d, I H, *J*= 1.6 Hz), 8.92 (s, 1H), 9.09 (s, 1H), 11.19 (s, 1H), 12.22 (s, 1H); MS (ESI) [M+H]⁺ = 318, [M-H]⁻ = 316; Anal. calcd. for C₁₅H₁₅N₃OS₂: C, 56.76; H, 4.76; N, 13.24. Found: C, 56.78; H, 4.87; N, 12.54.

### EXAMPLE 22

### 5-(4,4-Dimethyl-2-thioxo-1,4-dihydro-2H-benzo[d] [1,3] oxazin-6-yl) thiophene-3-carbonitrile

5-(4,4-Dimethyl-2-oxo-1,4-dihydro-2H-benzo[d] [1,3]oxazin-6-yl)-thiophene-3-carbonitrile was prepared, according to the procedure in Example 5 using (1,4-dihydro-4,4-dimethyl-2-oxo-2H-3,1-benzoxin-6-yl)boronic acid and 2-bromo-4-thiophenecarbonitrile, as an off-white solid: mp 255-260 °C; ¹H-NMR (DMSO-d₆) δ 10.36 (s, 1H), 8.48(d, 1H, *J*= 1.1 Hz),7.88-7.87 (d, 1H *J*= 1.3 Hz), 7.63 (d, 1H *J*= 1.9 Hz),7.56-7.54 (dd, 1H, *J =* 8.0, 2.0 Hz), 6.93 (d, 1H, *J =* 8.1 Hz),1.64 (s, 6H); MS(-ESI) m/z 283 (M-H)⁻.

The title compound was prepared in a manner similar to Example 16 using 5-(4,4-dimethyl -2-oxo- 1,4-dihydro-2H-benzo [d] [1,3] oxazin-6-yl) thiophene-3-carbonitrile and Lawesson's reagent. The product was obtained in the form of yellow crystals: mp. 258-259 °C.¹H NMR (DMSO-d₆) δ 12.3 (s,1 H),8.54 (d, 1H, *J*= 0.9 Hz),7.96 (s, 1H), 7.69-7.62 (m, 2H), 7.11-7.08 (d, 1H , *J* = 8.3 Hz), 1. 69 (s, 6H); MS (ESI) *mlz* 299 [M-H]; Anal. Calcd. For C₁₅H₁₂N₂OS₂ ½ H₂O: C, 58.0; H, 4.24; N, 9.05. Found C, 58.33; H, 3.85; N, 8.39.

### EXAMPLE 23

### 5-(4,4-dimethyl-2-thioxo-1,4-dihydro-2H-3,1-benzoxazin-6-yl)-1-ethyl-1H-pyrrole-2-carbonitrile

To a solution of 5-(4,4-dimethyl-2-oxo-1,4-dihydro-2H-3,1-benzoxazin-6-yl)-1H-pyrrole-2-carbonitrile (1.3 g, 5 mmol) in dimethylformamide (25 ml) was added potassium carbonate (1 g, 7.5 mmol), and iodomethane (0.4 ml, 5.1 mmol), and the mixture was stirred at room temperature for 3 hours. The mixture was triturated with ethyl acetate/water, and the ethyl acetate layer was separated, dried over magnesium sulfate, and concentrated *in vacuo.* The residue was recrystallized from ethyl acetate/hexane to afford 5-(4,4-dimetltyl-2-oxo-1,4-dihydro-2H-3,1-benzoxazin-6-yl)-1-ethyl-1H-pyrrole-2-carbonitrile (0.4 g, 27%): mp. 200-202 °C; ¹H-NMR (DMSO-d₆) δ 1.25 (t, *J =* 7.2 Hz, 3H), 1.64 (s, 6H), 4.07 (q, *J =* 7.2 Hz, 2H), 6.29 (d, *J =* 4.1, Hz, 1H), 7.0 (d, *J*= 8Hz, 1H), 7.05 (d, *J*=4.1 Hz, 1H),7.34 (m, 2H), 10.42 (s, 1H); MS (ESI) *m*/z 2 94 (M-H)⁻.

The title compound was prepared according to the procedure for Example 16 from 5-(4,4-dimethyl-2-oxo-1,4-dihydro-2H-3, 1-benzoxazin-6-yl)-1-ethyl-1H-pyrrele-2-carbonitrile and Lawesson's reagent The product was obtained in the form of white needles: mp 212-213 °C; ¹H-NMR (DMSO-d₆)δ 1.25 (t, 3H, *J* = 7 Hz), 1.68 (s, 6H), 4.07 (q, *J*= 7 Hz, 2H), 6.32 (d, *J*= 3.9 Hz, 1H), 7.07 (d, *J* = 3.9 Hz, 1H), 7.15 (d, *J*= 9 Hz, 1H), 7.42 (m, 2H), 12.33 (s, 1H); MS (ESI) *m*/*z* 310 (M-H)⁻.

### EXAMPLE 24

### 4-(1,2-Dihydro-2-thioxospiro[4H-3,1-benzoxazin-4,1-cyclohexan]-6-yl)-2-thiophenecarbonitrile

1-(2-Amino-5-bromo-phenyl) cyclohexanol was prepared, according to the procedure of Example 1 using 2-amino-5-bromobenzoic acid and the Grignard reagent prepared from 1,5-dibromopentane, as a clear oil: ¹H-NMR (DMSO-d₆) δ 7.07 (d, 1H, *J*= 2.3 Hz), 7.03 (dd, 1H, *J =* 8.4, 2.4 Hz), 6.55 (d, 1H, *J* = 8.6 Hz), 5.49 (s, 2H, D₂O exchangeable), 5.00 (s, 1H, D₂O exchangeable), 2.01 (d, 2H, *J* = 1.8 Hz), 1.66-1.77 (m, 2H), 1.44-1.61 (m, 4H), 1.16-1.34 (m, 2H); MS (ESI) *m*/*z* 270/272 ([M+H]⁺, 98%/100%).

6-Bromo-spiro[4H-3, 1-benzoxazine-4, 1'-cyclohexane-2-(1H)-one] was prepared from 1-(2-amino-5-bromo-phenyl) cyclohexanol and carbonyl diimidazole according to the procedure of Example 2. The product was obtained as an off-white solid: mp 208-210 °C; ¹H-NMR (DMSO-d₆) δ 10.26 (s, 1H), 7.45 (d, 1H, *J =* 2.2 Hz), 7.39 (dd, 1H, *J* = 8.2, 2.2 Hz), 6.81 (d, 1H, *J*= 8.3 Hz), 1.90-1.97 (m, 2H), 1. 80-1.85 (m, 5H), 1.25-1.35 (m, 1H); MS (APCl) *m*/*z* 296 ([M+H]⁺, 68%).

Spiro-(4, 1'-cyclohexane-1, 4-dihydro-2-oxo-2H-3,1-benzoxazin-6-yl) boronic acid, prepared according to the procedure of Example 4 using 6-bromo-spiro[4H-3, 1-benzoxazine-4, 1'-cyclohexane]-2-(1H)-one, as an off-white solid: mp 223-225 °C. ¹H-NMR (DMSO-d₆) δ 10.17 (s, 1H, D₂O exchangeable), 7.92 (s, 2H, D₂O exchangeable), 7.67 (S, 1H), 7.63 (dd, 1H, *J* = 8.0, 1.1 Hz), 6.81 (d, 1H, *J* = 7.9 Hz), 1.96(s, 1H), 1.93 (s, 1H), 1.57-1.88 (m, 7H), 1.24-1.34 (m, 1H); MS (ESI) *m*/*z* 262 (M+H)⁺.

4-(1, 2-Dihydro-2-oxospiro[4H-3,1-benzoxazine-4,1-cyclohexan]-6-yl)-2-thiophenecarbonitrile was prepared, according to the procedure of Example 5 from spiro-(4, 1'-cyclohexane-1,4-dihydro-2-oxo-2H-3,1-benzoxazin-6-yl) boronic acid and 3-bromo-5-cyanothiophene, as white crystals: mp 230-232 °C; IR (KBr) 2200 cm⁻¹; ¹H-NMR (DMSO-d₆) δ 10.29 (s, 1H), 8.49 (s, 1H), 8.33 (s, 1H), 7.69-7.63 (m, 2H), 6.93-6.91 (d, 1H, *J* = 8.2 Hz), 1.99-1.87 (m, 4H), 1.73-1.64 (m, 5H), 1.38-1.31 (m, 1H); MS(+)APCI m/z 325 (M+H)⁺; Anal. Calc. For C₁₈H₁₆N₂O₂S1/4H₂O: C, 65.73; H, 5.06; N, 8.52. Found: C, 65.55; H, 5.06; N, 8.22.

The title compound was prepared, according to the procedure of Example 16 using 4-(1,2-dihydro-2-oxospiro[4H-3,1-benzoxazine-4,1-cyclohexan]-6-yl)-2-thiophenecarbonitrile, as a yellow solid: mp 225-227°C; ¹H-NMR (CDCl₃) δ 8.98 (s, 1H), 7.83 (d, 1H, *J*= 1.47 Hz), 7.63 (d, 1H, *J=* 1.47 Hz), 7.46 (dd, 1H, *J=* 8.2, 1.91 Hz), 7.32 (m, 1H), 6.86 (d, 1H, *J*= 8.19 Hz), 2.28-2.29 (m, 2H), 2.06-2.01 (m, 2H), 1.83-1.70 (m, 5H), 1.37-1.3 (m, 1H); MS (ES) *m*/*z* 339 ([M-H]⁻).

### EXAMPLE 25

### 5-(4,4-Dimethyl-2-thioxo-1,4-dihydro-2H-3,1-benzoxaz-6-yl)-2-fluorobenzonitrile

3-(4,4-Dimethyl-2-oxo-1,4-dihydro-2H-benzo[d][1,3]oxazin-6-yl)-4-fluorobenzonitrile was prepared, from (1, 4-dihydro-4,4-dimethyl-2-oxo-2H-3,1-benzoxin-6-yl)boronic acid and 5-bromo-2-fluorobenzonitrile according to the procedure of Example 5, as a white solid: mp 229-230°C; ¹H-NMR (DMSO-d₆) δ 10.4 (s, 1H), 8.15 (dd, 1H, *J* = 7.39, 2.12 Hz), 7.95-7.89 (m, 1H), 7.59-7.48 (m, 3H), 6.99 (d, 1H, *J =* 8.1 Hz), 1. 7 (s, 6H); MS (APCI) *mlz* 297 ([M + H]⁺, 100%); Anal. Calc. For C₁₇H₁₃FN₂O₂: C, 68.91, H, 4.42, N, 9.45. Found: C, 68.74, H, 4.83, N, 9.10.

The title compound was prepared, according to the procedure of Example 16 using 5-(4,4-dimethyl-2-oxo-1,4-dihydro-2H-benzo[d][1,3]oxazin-6-yl)-2-fluorobenzonitrile, as a white solid: mp 258-259°C; ¹H-NMR (DMSO-d₆) δ 12.3 (s, 1H), 8.35-8.32 (m, 1H), 8.15-8.10 (m, 1H), 7.72-7.7 (m, 2H), 7.66-7.60 (m, 1H), 7.13 (d, 1H, *J*= 8.07 Hz), 1.7 (s, 6H); LC/MS (ES) m/z 311 ([M + H]⁺, 100%); Anal. Calcd. For C₁₇H₁₃FN₂OS: C, 64.99, H, 4.24, N, 8.66. Found: C, 64.7, H, 4.09, N, 8.66.

### EXAMPLE 26

### 6-(5-Bromopyridin-3-yl)-4,4-dimethyl-1,4-dihydro-2H-3,1-benzoxazine-2-thione

6-(5-Bromo-pyridin-3-yl)-4,4-dimethyl-1,4-dihydro-benzo[d][1,3]oxazin-2-one, prepared from (1,4-dihydro-4,4-dimethyl-2-oxo-2H-3,1-benzoxin-6-yl)boronic acid and 3,5-dibromopyridine according to the procedure of Example 6, as a white solid: mp 211-212°C; ¹H-NMR (DMSO-d₆) δ 10.4 (s, 1H), 8.92 (d, 1H, *J*= 1.9 Hz), 8.66 (d, 1H, *J=* 2.09 Hz), 8.40 (t, 1H, *J=* 2.02 Hz), 7.72-7.68 (m, 2H), 6.99 (d, 1H, *J=* 8.1 Hz), 1.7 (s, 6H); MS (APCI) *m*/*z* 333([M + H]⁺, 100%), 335([M + H]⁺, 100%); Anal. Calcd. For C₁₅H₁₃BrN₂O₂: C, 54.07, H, 3.93, N, 8.41. Found: C, 54.15, H, 3.89, N, 8.31.

The title compound was prepared, according to the procedure of Example 16 using 6-(5-bromo-pyridin-3-yl)-4,4-dimethyl-1,4-dihydro- benzo[d][1,3]oxazin-2-one, as a white solid: mp 252-253°C; ¹H-NMR (DMSO-d6) δ 12.3 (s, 1H), 8.94 (s, 1H), 8.69 (s, 1H), 8.44 (s, 1H), 7.78-7.76 (m, 2H), 7.14 (d, 1H, J = 8.8 Hz), 1.7 (s, 6H); LC/MS (ES) m/z 347/349 ([M- H]⁻); Anal. Calcd. For C₁₅H₁₃BrN₂OS: C, 51.32, H, 3.79, N, 7.98 Found: C, 50.95, H, 3.56, N, 7.72

### EXAMPLE 27

### 6-(3-Chloro-5-fluorophenyl)-4,4-dimethyl-1,4-dihydro-2H-3,1-benzoxazine-2-thione

6-(3-Chloro-5-fluoro-phenyl)-4,4-dimethyl-1,4-dihydro-benzo[d][1,3]oxazin-2-one was prepared, from (1,4-dihydro-4,4-dimethyl-2-oxo-2H-3,1-benzoxin-6-yl)boronic acid and 1-bromo-3-chloro-5-fluorobenzene according to the procedure of Example 5, as a white solid: mp 193-194 °C; ¹H-NMR (DMSO-d₆) δ 10.4 (s, 1H), 7.67-7.64 (m, 3H), 7.61-7.57 (m, 1H), 7.41-7.37 (m, 1H), 6.96 (d, 1H, *J*= 8.72 Hz), 1.7 (s, 6H); MS (APCI) *m*/*z* 306([M + H]⁺, 100%); Anal. Calc. For C₁₆H₁₃ClFNO₂: C, 62.86, H, 4.29, N, 4.58. Found: C, 62.98, H, 4.1, N, 4.6.

The title compound was prepared, according to the procedure of Example 16, starting with 6-(3-chloro-5-fluoro-phenyl)-4,4-dimethyl-1,4-dihydro-benzo[d][1,3]oxazin-2-one: mp 204-205°C; ¹H-NMR (CDCl₃) δ 10.0 (s, 1H), 7.49 (m, 1H), 7.31 (bs, 2H), 7.15-7.08 (m, 2H), 7.01 (d, 1H, *J=* 8.23 Hz), 1.9 (s, 6H); LC/MS (ES) m/z 320/322 ([M- H]⁻).

### EXAMPLE 28

### 6-(3-Bromo-5-methylphenyl)-4,4-dimethyl-1,4-dihydro-2H-3,1-benzoxazine-2-thione

6-(3-Bromo-5-methyl-phenyl)-4,4-dimethyl-1,4-dihydrobenzo-[d][1,3]oxazin-2-one was prepared, using (4,4-dimethyl-1,4-dihydro-2-oxo-2H-3,1-benzoxazin-6-yl)boronic acid and 3,5-dibromotoluene according to the procedure of Example 5, as a white solid: mp 231-233 °C; ¹H-NMR (DMSO-d₆) δ 10.4 (s, 1H), 7.66 (s, 1H), 7.58-7.56 (m, 2H), 7.50 (s, 1H), 7.37 (s, 1H), 6.95 (d, 1H, *J=* 8.67 Hz), 2.37 (s, 3H), 1.67 (s, 6H); MS (ESI) *m*/*z* 344/346 ([M-H]⁻, 100%); Anal. Calc. For C₁₇H₁₆BrNO₂: C, 58.98, H, 4.66, N, 4.05. Found: C, 58.82, H, 4.62, N, 3.94.

The title compound was prepared according to the procedure of Example 16, using 6-(3-Bromo-5-methyl-phenyl)-4,4-dimethyl-1,4-dihydro-benzo[d][1,3]oxazin-2-one, as a yellow solid: mp 183-184°C; ¹H-NMR (CDCl₃) δ 9.8 (s, 1H), 7.48-7.46 (m, 2H), 7.34-7.25 (m, 4H), 6.97 (d, 1H, *J*= 8.2 Hz), 2.4 (s, 3H), 1.8 (s, 6H); MS (ES) m/z 360/362 ([M- H]⁻).

### EXAMPLE 29

### 6-(3-Bromo-5-trifluoromethoxyphenyl)-4,4-dimethyl-1,4-dihydro-2H-3,1-benzoxazine-2-thione

6-(3-Bromo-5-trifluoromethoxy-phenyl)-4,4-dimethyl-1,4-dihydrobenzo[d][1,3]-oxazin-2-one was prepared, using (4, 4-dimethyl-1,4-dihydro-2-oxo-2H-3,1-benzoxazin-6-yl)boronic acid and 1, 3-dibromo-5-trifluoromethoxybenzene according to the procedure of Example 5, as a white solid: mp 214-216°C; ¹H-NMR (DMSO-d₆) δ 10.4 (s, 1H), 7.99 (s, 1H), 7.73 (s, 1H), 7.68-7.62 (m, 3H), 6.97 (d, 1H, *J=* 8.0 Hz), 1.68 (s, 6H); MS (ESI) *m*/*z* 414 ([M - H]⁻, 100%); Anal. Calc. For C₁₇H₁₃BrF₃NO₃: C, 49.06, H, 3.15, N, 3.37. Found: C, 49.16, H, 3.05, N, 3.30.

The title compound was prepared, according to the procedure of Example 16 starting with 6-(3-Bromo-5-trifluoromethoxy-phenyl)-4,4-dimethyl-1,4-dihydro-benzo[d][1,3]oxazin-2-one, as a yellow solid: mp 192-193°C; ¹H-NMR (CDCl₃) δ 9.4 (s, 1H), 7.61 (s, 1H), 7.49-7.46 (m, 1H), 7.40 (s, 1H), 7.30 (s, 2H), 6. 96 (d, 1H, *J=* 8.22 Hz) 1.9 (s, 6H); MS (ES) m/z 431/433 ([M- H]⁻).

### EXAMPLE 30

### 3-(1,2-Dihydro-2-thioxospiro[4H-3,1-benzoxazine-4,1-cyclohexan]-6-yl)-5-fluorobenzonitrile

3-(1, 2-Dihydro-2-oxospiro[4H-3,1-benzoxazine-4,1-cyclohexan]-6-yl)-5-fluorobenzonitrile was prepared, according to the procedure of Example 5 from spiro-(4, 1'-cyclohexane-1,4-dihydro-2-oxo-2H-3,1-benzoxazin-6-yl) boronic acid and 3-bromo-5-fluorobenzonitrile, as a white powder: mp 250-253 °C; IR (KBr) 2220 cm⁻¹; ¹H-NMR (DMSO-d₆) δ 10.34 (s, 1H), 8.13 (s, 1H), 8.0 (d, 1H, J= 10.6 Hz), 7.80-7.7 (m, 3 H), 6.98-6.95 (d, 1H, *J*= 8.1 Hz), 1.99-1.97 (m, 4H), 1.76-1.65 (m, 6H), 1.37-1.33 (m 1H). MS (EI) *m*/*z* 336 (M⁺); Anal. Calc. For C₂₀H₁₇FN₂O₂H₂O: C, 67.78; H, 5.40; N, 7.90. Found: C, 67.9; H, 4.93; N, 7.67.

The title compound was prepared, according to the procedure of Example 16 using 3-(1,2-Dihydro-2-oxospiro[4H-3,1-benzoxazine-4,1-cyclohexan]-6-yl)-5-fluorobenzonitrile: mp 235-237°C; ¹H-NMR (CDCl₃) δ 10.0 (s, 1H), 7.76-7.69 (m, 2H), 7.50-7.33 (m, 3H), 7.03 (d, 1H, *J=* 8.8 Hz), 2.3-1.26 (m, 10H); MS (ES) *mlz* 351 ([M-H]⁻).

### EXAMPLE 31

### 3-(4,4-Dimethyt-2-thioxo-1,4-dihydro-2H-3,1-benzoxazin-6-yl)-5-methylbenzonitrile

3-(4,4-Dimethyl-2-oxo-1,4-dihydro-2H-benzo[d][1,3]oxazin-6-yl)-5-methylbenzonitrile was prepared, from (4,4-dimethyl-1,4-dihydro-2-oxo-2H-3,1-benzoxazin-6-yl)boronic acid and 3-bromo-5-methylbemonitrile according to the procedure of Example 5, as a white solid: mp 256-258 °C; ¹H-NMR (DMSO-d₆) δ 10.4 (s, 1H), 7.99 (s, 1H), 7.86 (s, 1H), 7.67-7.62 (m, 3H), 6.97 (d, 1H, *J*= 8.11 Hz), 2.42 (s, 3H), 1.68 (s, 6H); MS (APCI) *m*/*z* 293 ([M+H]⁺, 100%); Anal. Calc. For C₁₈H₁₆N₂O₂: C, 73.96, H, 5.52, N, 9.58. Found: C, 73.26, H, 5.46, N, 9.24.

The title compound was prepared, according to the procedure for Example 16 starting with 6-(3-cyano-5-methyl-phenyl)-4.4-dimethyl-1,4-dihydro-benzo[d][1,3]oxazin-2-one: mp 230-231°C, ¹H-NMR (CDCl₃) δ 9.1 (s, 1H), 7.61 (s, 1H), 7.55 (s, 1H), 7.50-7.47 (m, 2H), 7.32-7.31 (m, 1H), 6.91 (d, 1H, *J*= 8.2), 2.5 (s, 3H), 1.8 (s, 6H); MS (ES) *mlz* 307 ([M- H]⁻).

### EXAMPLE 32

### 6-(3,5-Dichlorophenyl)-4,4-dimethyl-1,4-dihydro-2H-3,1-benzoxazine-2-thione

6-(3,5-dichloro-phenyl)-4,4-dimethyl-1,4-dihydrobenzo-[d][1,3]oxazin-2-one was prepared, from 6-bromo-4,4-dimethyl-1,4-dihydro-benzo[d][1,3]oxazin-2-one and 3,5-dichlorophenyl boronic acid according to the procedure of Example 4, as a white solid: mp 245-246 °C; ¹H-NMR (DMSO-d₆) δ 10.4 (s, 1H), 7.77 (m, 2H), 7.67-7.64 (m, 2H), 7.56 (bs, 1H), 6.96 (d, 1H, *J*= 7.98 Hz), 1.7 (s, 6H); MS (EI) m/z 321 ([M+H]⁺, 40%); Anal. Calc. For C₁₆H₁₃C₁₂NO₂: C, 59.32, H, 4.11, N, 4.32. Found: C, 59.13, H, 4.29, N,4.17.

The title compound was prepared, according to the procedure of Example 16 starting with 6-(3,5-Dichloro-phenyl)-4,4-dimethyl-1,4-dihydro-benzo[d][1,3]oxazin-2-one: mp 206-208°C; ¹H-NUR (CDCl₃) δ 9.5 (s, 1H), 7.49-7.45 (m, 1H), 7.40-7.36 (m, 3H), 7.3-7.29 (m, 1H), 6.95 (d, 1H, *J=* 8.23), 1.8 (s, 6H); MS (ES) m/z 336/338 ([M- H]⁻).

### EXAMPLE 33

### 5-(4,4-Dimethyl-1,2-thioxo-1,4-dihydro-2H-3,1-benzoxazin-6-yl)isophthalonitrile

5-(4,4-Dimethyl-2-oxo-1,4-dihydro-2H-benzo[d][1,3]oxazin-6-yl)-isophthalonitrile was prepared from (1,4-dihydro-4,4-dimethyl-2-oxo-2H-3, 1-benzoxin-6-yl)boronic acid and 5-bromoisophthalonitrile according to the procedure Example 5, as a white solid: mp 288-289 °C; ¹H-NMR (DMSO-d₆) δ 10.4 (s, 1H), 8.58 (s, 2H), 8.40 (d, 1H, *J=* 0.77 Hz), 7.80.7.75 (m, 2H), 6.99 (d, 1H, *J=* 8.2 Hz), 1.7 (s, 6H); MS (EI) *m*/*z* 303([M⁺], 20%); Anal. Calc. For C₁₈H₁₃N₃O₂·1.65 H₂O: C, 64.92, H, 4.93, N, 12.62. Found: C, 64.74, H, 4.69, N, 12.32.

The title compound was prepared according to the procedure of Example 16 starting with 5-(4,4-Dimethyl-2-oxo-1,4-dilrydro-2H-benzo[d][1,3]ox,azin-6-yl)-isophthaloriitrile: mp 240-242°C; ¹H-NMR (CDCl₃) δ 9.4 (s, 1H), 8.03 (d, 2H, *J=* 1.25 Hz), 7.92 (s, 1H), 7.50 (dd, 1H, *J=* 8.22, 1.89 Hz), 7.33 (d, 1H, *J=* 1.8 Hz), 7.01 (d, 1H, *J=* 8.24 Hz), 1.84 (s, 6H); MS (ES) *m*/*z* 318 ([M- H]⁻).

### EXAMPLE 34

### 5-(4,4-Dimethyl-2-thioxo-1,4-dihydro-2H-3,1-benzoxazin-6-yl)-2-furonitrile

5-(4,4-Dimethyl-2-oxo-1,4-dihydro-2H-benzo[d][1,3]oxazin-6-yl)-furan-2-carbonitrile was prepared, according to the procedure of Example 5 from 2-bromo-5-cyanofuran (1.0 g, 5.6 mmol) (J. Med. Chem (1997), 40(23), 3804-3819) and (1,4-dihydro-4,4-dimethyl-2-oxo-2H-3,1-benzoxazin-6-yl)boronic acid (1.8 g, 8.18 mmol), as a white solid (0.39 g, 1.45 mmol, 17%): mp 257 - 260°C; ¹H-NMR (DMSO-d₆) δ 10.48 (s, 1H), 7.73 - 7.70 (m, 3H), 7.19 (d, 1 H, *J*= 3.8 Hz), 6.98 (d, 1 H, *J*= 8.9 Hz), 1.66 (s, 6H); MS ((+)-APCI) *mlz* = 269 (M+H)⁺.

The title compound was prepared according to the procedure of Example 16 using 5-(1,4-Dihydro-4,4-dimethyl-2-oxo-2H-3,1-benzoxazin-6-yl)-2-furancarbonitrile: mp 200°C decomposes; ¹H-NMR (CDCl₃) δ 9.1 (s, 1H), 7.63 (dd, 1H, *J*= 8.26, 1.68 Hz), 7.53 (d, 1H, *J=* 1.59 Hz), 7.19 (d, 1H, *J* = 3.7 Hz), 6.89 (d, 1H, *J=* 8.31 Hz), 6.71 (d, 1H, *J=* 3.72 Hz), 1.8 (s, 6H); MS (ES) *m*/*z* 283 ([M-H]⁻)

### EXAMPLE 35

### 4,4-Diethyl-6-(3-nitrophenyl)-1,4-dihydro-2H-3,1-benzoxazine-2-thione

4,4-Diethyl-6-(3-nitrophenyl)-1,4-dihydrobenzo[d][1,3]oxazin-2-one was prepared, from 4,4-diethyl-6-iodo-1,4-dihydrobenzo[d][1,3]oxazin-2-one and 3-nitrophenyl boronic acid according to the procedure of Example 4, as an off-white solid: mp 193-194 °C; ¹H-NMR (CDCl₃) δ 9.19 (s, 1H, D₂O exchangeable), 8.38 (t, 1H, *J =* 1.9 Hz), 8.20 (m, 1H), 7.83 (m, 1H), 7.61 (t, 1H, *J* =8. 0 Hz), 7.50 (dd, 1H, *J* = 8.2, 2.0 Hz), 7.23 (d, 1H, *J =* 1.7 Hz), 6.99 (d, 1H, *J=* 8.3 Hz), 2.09 (q, 4H, *J =* 7.4 Hz), 0.96 (t, 6H*, J =* 8.3 Hz); MS (EI) *mlz* 325 ([M-H]⁻, 100%). Anal. Calc. For C₁₈H₁₈N₂O₄·0.3 H₂O: C, 65.17, H, 5.65, N, 8.44. Found: C, 65.31, H, 5.60, N, 8.10.

The title compound was prepared, according to the procedure of Example 16 starting with 4,4-Diethyl-6-(3-nitro-phenyl)-1,4-dihydro-benzo[d][1,3]oxazin-2-one, as a yellow solid: mp 180-181°C; ¹H-NMR (CDCl₃) δ 9.1 (s, 1H), 8.38 (t, 1H, *J* = 1.94 Hz), 8.25-8.22 (m, 1H), 7.87-7.85 (m, 1H), 7.64 (t, 1H, *J=* 7.99 Hz), 7.55 (dd, 1H, *J=* 8.24, 1.89 Hz), 7.25 (d, 1H, *J* = 1.71 Hz), 6.93 (d, 1H, *J*= 8.25 Hz), 2.2-2.03 (m, 4H), 0.96 (t, 6H, *J* = 7.33 Hz); MS (ES) *m*/*z* 341 ([M- H]⁻).

### EXAMPLE 36

### 6-(3-Chlorophenyl)-4-methyl-4-phenyl-1,4-dihydro-2H-3,1-benzoxazine-2-thione

A mixture of 2-amino-5-bromo-*N*-methoxy-*N*-methylbenzamide (7.78g, 30 mmol), 3-chlorophenyl boronic acid (5.63g, 36 mmol), tetrakis(triphenylphosphine)palladium (0) (1.73g, 1.5 mmol), and sodium carbonate (7.63g, 72 mmol) in a mixture of DME and water (150 mL/30 mL) was degassed to remove the oxygen and heated at 85°C under nitrogen for 3 hours. The reaction mixture was cooled to room temperature and treated with brine (30 mL) and ethyl acetate (100 mL). The organic layer was separated and the aqueous layer was extracted with ethyl acetate (3x40 mL). The combined organic layers were washed with brine and dried with MgSO₄. After removal of the solvent, the residue was purified by a flash chromatography (silica gel, hexane:ethyl acetate/1:1) to give 5-(3-chlorophenyl)-*N*-methoxy-*N*-methylbenzamide as a brown oil (5g, 57%). To a solution of this benzamide (5g, 17.2 mmol) in anhydrous THF was added in a dropwise fashion a solution of methyllithium in ether (1.4M, 28.6 mL, 40 mL) at -78 °C under nitrogen. After stirring for 30 minutes, the reaction mixture was treated with a saturated aqueous ammonium chloride solution (50 mL) at -78 °C. Ethyl acetate (100 mL) was added, organic layer was separated, and aqueous layer was extracted with ethyl acetate (3x20 mL). The combined organic layers were washed (brine) and dried (MgSO₄). After removal of the solvent, the residue was purified by a flash chromatography (silica gel, hexane: ethyl acetate/2:1) to afford 1-(4-amino-3'-chlorobiphenyl-3-yl)-ethanone as a yellow solid (2g, 47%): mp 89-90 °C; ¹H-NMR (CDCl₃) δ 7.89 (d, 1H, *J=* 2.0 Hz), 7.51 (m, 2H), 7.25-7.40 (m, 3H), 6.73 (d, 1H, *J=* 8.6 Hz), 6.38 (br, 2H), 2.65 (s, 3H); MS (EI) *m*/*z* 268([M+Na]⁺, 60%); Anal. Calc. For C₁₄H₁₂ClNO: C, 68.44, H, 4.92, N, 5.70. Found: C, 68.40, H, 4.89, N, 5.61.

6-(3-Chlorophenyl)-4-methyl-4-phenyl-1,4-dihydro-benzo[d][1,3]oxazin-2-one was prepared, from 1-(4-amino-3'-chloro-biphenyl-3-yl)-ethanone (0.2g, 0.82 mmol) and phenylmagnesium bromide followed by treatment with CDI in THF, as a white solid: mp 179-180 °C; ¹H-NMR (CDCl₃) δ 8.27 (s, 1H, D₂O exchangeable), 7.51-7.57 (m, 2H), 7.28-7.45 (m, 9H), 6.92 (d, 1H, *J=* 8.4 Hz), 2.12 (s, 3H); MS (ESI) *m*/*z* 348 ([M-H]⁻, 100%); Anal. Calc. For C₂₁H₁₆ClNO₂: C, 72.10, H, 4.61, N, 4.00. Found: C, 71.72, H, 4.86, N, 3.91.

The title compound was prepared, according to the procedure of Example 16 starting with 6-(3-chlorophenyl)-4-methyl-4-phenyl-1,4-dihydro-benzo[d][1,3]oxazin-2-one, as a white solid: mp 202-203°C; ¹H-NMR (CDCl₃) δ 8.9 (s, 1H), 7.59-7.56 (m, 2H), 7.49-7.30 (m, 9H), 6.91 (d, 1H, *J=* 8.19 Hz), 2.2 (s, 3H); MS (ES) m/z 364 ([M-H]⁻).

### EXAMPLE 37

### 4-Allyl-6-(3-chlorophenyl)-4-methyl-1,4-dihydro-2H-3,1-benzoxazine-2-thione

To a solution of 1-(4-amino-3'-chloro-biphenyl-3-yl)-ethanone (0.2g, 0.82 mmol) in anhydrous THF (10 mL) was added a solution of allylmagnesium bromide in ether (1.0 M, 3 mL, 3 mmol) at 0°C under nitrogen. The reaction solution was slowly warmed to ambient temperature and stirred under nitrogen for 1 hour. A saturated aqueous ammonium chloride solution (10 mL) was added and was followed by addition of ethyl acetate (50 mL). The organic layer was separated and the aqueous layer was extracted with ethyl acetate (3x10 mL). The combined organic layers were washed with brine and dried with MgSO₄. After removal of solvent, the residue was purified by a flash chromatography (silica gel, hexane:ethyl acetate/3:1) to afford an amino carbinol intermediate which was used in next step without further purification.

To a solution of the above amino carbinol in anhydrous THF was added CDI (0.38g, 2.3 mmol) at ambient temperature under nitrogen. The reaction solution was heated at 55 °C for 12 hours and then cooled to room temperature. The solvent was *removed in vacuo* and the residue was purified by a flash chromatography (silica gel, hexane:ethyl acetate/2:1) to yield 4-allyl-6-(3-chlorophenyl)-4-methyl-1,4-dihydro-benzo[d][1,3]oxazin-2-one as a white solid (130 mg, 52%): mp 128-129 °C; ¹H-NMR (CDCl₃) δ 8.68 (s, 1H, D₂O exchangeable), 7.50 (s, 1H), 7.44 (dd, 1H, *J*= 8.2, 1.9 Hz), 7.31-7.40 (m, 3H), 7.25 (d, 1H, *J=* 1.6 Hz), 6.92 (d, 1H, *J=* 8.2 Hz), 5.70-5.85 (m, 1H), 5.17 (m, 2H), 2.76 (m, 2H), 1.79 (s, 3H); MS (ESI) m/z 314 ([M+H]⁺, 40%); Anal. Calc. For C₁₈H₁₆CINO₂: C, 68.90, H, 5.14, N, 4.46. Found: C, 68.90, H, 5.18, N, 4.43.

The title compound was prepared according to the procedure of Example 16 starting with 4-Allyl-6-(3-chloro-phenyl)-4-methyl-1,4-dihydro-benzo[d][1,3]oxazin-2-one: mp 150-151°C; ¹H-NMR (CDCl₃) δ 8.9 (s, 1H), 7.50-7.47 (m, 2H), 7.40-7.35 (m, 3H), 7.27 (s, 1H), 6.87 (d, 1H, *J*= 8.22 Hz), 5.81-5.72 (m, 1H), 5.19-5.13 (m, 2H), 2.78-2.75 (m, 2H), 1.82 (s, 3H); MS (ES) m/z 328 ([M- H]⁻).

### EXAMPLE 38

### 3-Chloro-5-(4,4-dimethyl-2-thioxo-1,4-dihydro-2H-3,1-benzoxazin-6-yl)benzonitrile

3-Chloro-5-(4,4-dimethyl-2-oxo-1,4-dihydro-2H-benzo[d][1,3]oxazin-6-yl)-benzonitrile was prepared, from (1,4-dihydro-4,4-dimethyl-2-oxo-2H-3,1-benzoxin-6-yl)boronic acid and 1-bromo-3-chlorobenzonitrile according to the procedure of Example 5, as a white solid: mp 256-257 °C; ¹H-NMR (DMSO-d₆) δ 10.4 (s, 1H), 8.22 (bs, 1H), 8.15 (bs, 1H), 7.98 (bs, 1H), 7.74-7.71 (m, 2H), 6.97 (d, 1H, *J*= 8.09 Hz), 1.7 (s, 6H); MS (ESI) *m*/*z* 311([M - H]⁻, 100%); Anal. Calc. For C₁₇H₁₃CIN₂O₂: C, 65.29, H, 4.19, N, 8.96. Found: C, 65.25, H, 3.92, N, 8.71.

The title compound was prepared according to the procedure of Example 16 starting with 3-chloro-5-(4,4-dimethyl-2-oxo-1,4-dihydro-2H-benzo[d][1,3]oxazin-6-yl)-benzonitrile: mp 249-251°C; ¹H-NMR (CDCl₃) δ 9.7 (s, 1H), 7.74-7.73 (m, 1H), 7.71-7.70 (m, 1H), 7.64-7.63 (m, 1H), 7.48 (dd, **1H,** *J=* 8.24, 1.86 Hz), 7.3 (d, 1H, *J* = 1.73 Hz), 7.01 (d, 1H, *J*= 8.24 Hz), 1.8 (s, 6H); MS (ES) m/z 327/329 ([M- H]⁻).

### EXAMPLE 39

### 6-(3,5-Difluorophenyl)-4,4-dimethyl-1,4-dihydro-2H-3,1-benzoxazine-2-thione

6-(3,5-difluoro-phenyl)-4,4-dimethyl-1,4-dihydrobenzo-[d] [1,3]oxazin-2-one was prepared, according to the procedure of Example 5 from (4,4-dimethyl-1,4-dihydro-2-oxo-2H-3,1-benzoxazin-6-yl)boronic acid and 1-bromo-3,5-difluorobenzene, as a white solid: mp 218-219 °C; ¹H-NMR (DMSO-d₆) δ 10.4 (s, 1H), 7.67-7.65 (m, 2H), 7.49 (d, 2H, *J*= 7.73 Hz), 7.19 (t, 1H, *J*= 9.29 Hz), 6.96 (d, 1H, *J=* 8.88 Hz), 1.7 (s, 6H); MS (APCI) *mlz* 290 ([M + H]⁺, 100%); Anal. Calc. For C₁₆Hₗ₃F₂NO₂: C, 66.43, H, 4.53, N, 4.84. Found: C, 66.01, H, 4.46, N, 4.67.

The title compound was prepared according to the procedure of Example 16 starting with 6-(3,5-Difluoro-phenyl)-4,4-dimethyl-1,4-dihydro-benzo[d][1,3]oxazin-2-one: mp 224-225°C; ¹H-NMR (CDCl₃) δ 9.7 (s, 1H), 7.48 (dd, 1H, *J* = 8.16, 1.74 Hz), 7.31 (d, 1H, *J=* 1.66 Hz), 7.08-7.03 (m, 2H), 6.98 (d, 1H, *J*= 8.23 Hz), 6.85-6.78 (m, 1H), 1.8 (s, 6H); MS (ES) *m*/*z* 304 ([M- H]⁻).

### EXAMPLE 40

### 6-(3-Fluoro-5-methoxyphenyl)-4,4-dimethyl-1,4-dihydro-2H-3,1-benzoxazine-2-thione

6-(3-Fluoro-5-methoxy-phenyl)-4,4-dimethyl-1,4-dihydro-benzo[d][1,3]oxazin-2-one was prepared, from (1,4-dihydro-4,4-dimethyl-2-oxo-2H-3,1-benzoxin-6-yl)boronic acid and 3-bromo-5-fluoroanisole according to the procedure of Example 5, as a white solid: mp 181-182 °C; ¹H-NMR (DMSO-d₆) δ 10.4 (s, 1H), 7.62-7.59 (m, 2H), 7.13-7.06 (m, 2H), 6.97-6.94 (d, 1H, *J=* 8.89 Hz), 6.80 (dt, 1H, *J* = 10.95, 2.12 Hz), 3.8 (s, 3H), 1.7 (s, 6H); MS (ESI) *m*/*z* 302 ([M + H]⁺, 100%); Anal. Calc. For C₁₇H₁₆FNO₃·0.1 H₂O: C, 67.36, H, 5.39, N, 4.62. Found: C, 67.11, H, 5.44, N, 4.48.

The title compound was prepared, according to the procedure of Example 16 starting with 6-(3-fluoro-5-methoxy-phenyl)-4,4-dimethyl-1,4-dihydro-benzo[d][1,3]oxazin-2-one, as a white solid: mp 170-171°C; ¹H-NMR (CDCl₃) δ 9.2 (s, 1H), 7.48 (dd, 1H, *J* = 8.18, 1.84 Hz), 7.32 (d, 1H, *J =* 1.66 Hz), 6.91 (d, 1H, *J* = 8.23 Hz), 6.84 (d, 1H, *J* = 2.11 Hz), 6.82-6.81 (m, 1H), 6.66-6.61 (m, 1H), 3.9 (s, 3H), 1.8 (s, 6H); MS (ES) m/z 316 ([M- H]⁻).

### EXAMPLE 41

### 3-(4,4-Dimethyl-2-thioxo-1,4-dihydro-2H-3,1-benzoxazin-6-yl)-5-methoxybenzonitrile

A mixture of (4,4-dimethyl-1,4-dihydro-2-oxo-2H-3,1-benzoxazin-6-yl)boronic acid (4.2 g, 19.0 mmol), 3-cyano-5-methoxybenzyltriflate (5.1 g, 19.0 mmol), tetrakis(triphenylphosphine)-palladium (0) (1.1 g, 0.95 mmol), sodium carbonate (4.0 g, 38.0 mmol), lithium bromide (5 g, 57 mmol) in DME (50 mL), and water (25 mL) under a blanket of nitrogen, was stirred for 15 minutes at 50°C. This solution was then was heated at 85 °C for 1 hour. The reaction mixture was cooled to room temperature and ethyl acetate (100 mL) was added. The organic layers were washed twice with aqueous ammonium chloride (100 mL) and once with brine (100 mL), dried over magnesium sulfate and concentrated. Purification via chromatography (silica gel, 40% ethyl acetate/ hexane) gave 3-(4,4-dimethyl-2-oxo-1,4-dihydro-2H-benzo[d][1,3]oxazin-6-yl)-5-methoxy-benzonitrile as a white solid (0.69 g, 53%): mp 254-255 °C; ¹H-NMR (DMSO-d₆) δ 10.4 (s, 1H), 7.84 (s, 1H), 7.67-7.61 (m, 2H), 7.55 (bs, 1H), 7.4 (bs 1H) 6.99 (d, 1H, *J*= 7.94 Hz), 3.88 (s, 3H), 1.67 (s, 6H,); MS (EI) *mlz* 308 ([M + H]⁺, 30%); Anal. Calc. For C₁₈H₁₆N₂O₃: C, 68.13, H, 5.40, N, 8.83. Found: C, 68.03, H, 5.22, N, 8.46.

The title compound was prepared, according to the procedure of Example 16 starting with 3-(4,4-dimethyl-2-oxo-1,4-dihydro-2H-benzo[d][1,3]oxazin-6-yl)-5-methoxy-benzonitrile, as an off-white solid: mp 201-202°C; ¹H-NMR (CDCl₃) δ 9.1 (s, 1H), 7.48 (dd, 1H, *J*= 8.16, 1.8 Hz), 7.41 (s, 1H), 7.31 (d, 1H, *J*= 1.69 Hz), 7.27 (m, 1H), 7.14 (m, 1H), 6.92 (d, 1H,J= 8.2 Hz), 3.9 (s, 3H), 1.8 (s, 6H); MS (ES) m/z 323 ([M- H]⁻).

### EXAMPLE 42

### 6-(3-Fluorophenyl)-4,4-dimethyl-1,4-dihydro-2H-3,1-benzoxazine-2-thione

6-(3-Fluoro-phenyl)-4,4-dimethyl-1,4-dihydro-benzo[d][1,3]oxazin-2-one was prepared, from 6-bromo-4,4-dimethyl-1,4-dihydro-benzo[d][1,3]oxazin-2-one 1-bromo-3-fluorobenzene according to the procedure of Example 4, as a light yellow solid: mp 181-182 °C; ¹H-NMR (DMSO-d₆) δ 10.4 (s, 1H), 7.62-7.44 (m, 5H), 7.16 (t, 1H, *J=* 2.22 Hz), 6.97 (d, 1H, *J=* 8.83), 1.67 (s, 6H); MS (EI) *mlz* 271 ([M + H]⁺, 40%); Anal. Calc. For C₁₆H₁₄FNO₂: C, 69.91, H, 5.3, N, 5.1. Found: C, 70.0, H, 5.32, N, 4.92.

The title compound was prepared, according to the procedure of Example 16 using 6-(3-Fluoro-phenyl)-4,4-dimethyl-1,4-dihydro-benzo[d][1,3]oxazin-2-one, as a white solid: mp 194-195°C; ¹H-NMR (CDCl₃) δ 8.9 (s, 1H), 7.50 (dd, 1H, *J*= 8.15, 1.73 Hz), 7.46-7.38 (m, 1H), 7.34-7.30 (m, 2H), 7.25-7.21 (m, 1H), 7.10-7.04 (m, 1H), 6.89 (d, 1H, *J =* 8.21 Hz), 1. 8 (s, 6H); MS (ES) *m*/*z* 286 ([M- H]⁻).

### EXAMPLE 43

### 6-[3-Fluoro-5-(trifluoromethyl)phenyl]-4,4-dimethyl1,4-dihydro-2H-3,1-benzoxazine-2-thione

6-(3-Fluoro-5-trifluoromethyl-phenyl)-4,4-dimethyl-1,4-dihydro-benzo[d][1,3]oxazin-2-one was prepared from (1,4-dihydro-4,4-dimethyl-2-oxo-2H-3,1-benzoxin-6-yl)boronic acid and 1-bromo-3-fluoro-5-trifluoromethylbenzene according to the procedure of Example 5, as a white solid: mp 207-208 °C; ¹H-NMR (DMSO-d₆) δ 10.4 (s, 1H), 7.94-7.9 (m, 2H), 7.73-7.7 (m, 2H), 7.63 (d, 1H, *J*= 8.58 Hz), 6.99 (d, 1H, *J=* 8.68 Hz), 1.7 (s, 6H); MS (EI) *mlz* 339([M⁺], 60%); Anal. Calc. For C₁₇H₁₃F₄NO₂: C, 60.18, H, 3.86, N, 4.13. Found: C, 59.9, H, 3.99, N, 4.06.

The title compound was prepared, according to the procedure of Example 16 starting with 6-(3-fluoro-5-trifluoromethyl-phenyl)-4,4-dimethyl-1,4-dihydro-benzo[d][1,3]oxazin-2-one, as a white solid: mp 204-206°C; ¹H-NMR (CDCl₃) δ 9.2 (s, 1H), 7.56 (s, 1H), 7.5 (dd, 1H, *J =* 8.14,1.97 Hz), 7.44-7.38 (m, 1H), 7.36-7.30 (m, 2H), 6.92 (d, 1H, *J=* 8.14 Hz), 1.83 (s, 6H); MS (ES) m/z 354 ([M- H]⁻).

### EXAMPLE 44

### 6-(2-Fluorophenyl)-4,4-dimethyl-1,4-dihydro-2H-3,1-benzoxazine-2-thione

6-(2-Fluoro-phenyl)-4,4-dimethyl-1,4-dihydro-benzo[d][1,3]oxazin-2-one was prepared, according to the procedure of Example 5 from (1,4-dihydro-4,4-dimethyl-2-oxo-2H-3,1-benzoxin-6-yl)boronic acid and 1-bromo-2-fluorobenzene, as off-white crystals: mp 164-165 °C; ¹H-NMR(DMSO-d₆) δ 10.33 (s, 1H), 7.56 (m, 1H), 7.25-7.45 (m, 4H), 6.98 (d, 1H, *J =* 8.7 Hz), 1.64 (s, 6H).

The title compound was prepared, according to the procedure of Example 16 starting with 6-(2-fluoro-phenyl)-4,4-dimethyl-1,4-dihydro-benzo[d][1,3]oxazin-2-one, as a white solid: mp 171-173°C; ¹H-NMR (CDCl₃) δ 8.97 (s, 1H), 7.5-7.13 (m, 6H), 6.88 (d, 1H, *J =* 8.14 Hz), 1.80 (s, 6H); MS (ES) m/z 286 ([M- H]⁻).

### EXAMPLE 45

### 6-(3,4-Difluorophenyl)-4,4-dimethyl-1,4-dihydro-2H-3,1-benzoxazine-2-thione

6-(3,4-Difluoro-phenyl)-4,4-dimethyl-1,4-dihydro-benzo [d] [1,3]oxazin-2-one was prepared, according to the procedure of Example 5 from (1,4-dihydro-4,4-dimethyl-2-oxo-2H-3,1-benzoxin-6-yl)boronic acid and 1-bromo-3, 4-difluorobenzene, as off-white crystals: mp 207-208 °C; ¹H-NMR(DMSO-d₆) δ 10.35 (s, 1H), 7.79 (m, 1H), 7.40-7.63 (m, 4H), 6.95 (d, 1H, *J*= 8.9 Hz), 1.62 (s, 6H).

The title compound was prepared, according to the procedure of Example 16 starting with 6-(3,4-difluoro-phenyl)-4,4-dimethyl-1,4-dihydro-benzo[d][1,3]oxazin-2-one, as a yellow solid: mp 209-211°C;¹H-NMR (CDCl₃) δ 8.94 (s, 1H), 7.44 (dd, 1H, *J=* 8.35, 1.98 Hz), 7.36-7.22 (m, 4H), 6.87 (d, 1H, *J=* 8.35 Hz), 1.81 (s, 6H); MS (ES) *m*/*z* 304 ([M- H]⁻).

### EXAMPLE 46

### 6-(4-Fluorophenyl)-4,4-dimethyl-1,4-dihydro-2H-3,1-benzoxazine-2-thione

6-(4-Fluoro-phenyl)-4,4-dimethyl-l ,4-dihydro-benzo[d] [1,3]oxazin-2-one was prepared, according to the procedure of Example 5 from (1,4-dihydro-4,4-dimethyl-2-oxo-2H-3,1-benzoxazin-6-yl)boronic acid and 1-bromo-4-fluorobenzene, as off-white crystals: mp 232-233 °C; ¹H-NMR (DMSO-d₆) δ 10.3 (s, 1H), 7.74 (m, 2H), 7.53 (m, 2H), 7.28 (m, 2H), 6.96 (d, 1H, *J*= 8.9 Hz), 1.63 (s, 6H).

The title compound was prepared, according to the procedure of Example 16 starting with 6-(4-fluoro-phenyl)-4,4-dimethyl-1,4-dihydro-benzo[d][1,3]oxazin-2-one, as a white solid: mp 221-223°C; ¹H-NMR (CDCl₃) δ 8.98 (s, 1H), 7.5-7.44 (m, 3H), 7.5 (m, 1H), 7.17-7.10 (m, 2H), 6.87 (d, 1H, *J=* 8.14 Hz), 1.81 (s, 6H); MS (ES) *mlz* 286 ([M- H]⁻).

### EXAMPLE 47

### 3-(4,4-Dimethyl-2-thioxo-1,4-dihydro-2H-3,1-benzoxazin-6-yl)-4 fluorobenzonitrile

3-(4,4-Dimethyl-2-oxo-1,4-dihydro-2H-benzo[d][1,3]oxazin-6-yl)-4-fluorobenzonitrile was prepared, from (1, 4-dihydro-4,4-dimethyl-2-oxo-2H-3,1-benzoxin-6-yl)boronic acid and 5-bromo-2-fluorobenzonitrile according to the procedure of Example 5, as a white solid: mp 229-230 °C; ¹H-NMR (DMSO-d₆) δ 10.4 (s, 1H), 8.15 (dd, 1H *J*= 7.39, 2.12 Hz), 7.95-7.89 (m, 1H), 7.59-7.48 (m, 3H), 6.99 (d, 1H, *J =* 8.1 Hz), 1. 7 (s, 6H); MS (APCI) *m*/*z* 297 ([M + H]⁺, 100%); Anal. Calc. For C₁₇H₁₃FN₂O₂: C, 68.91, H, 4.42, N, 9.45. Found: C, 68.74, H, 4.83, N, 9.10.

The title compound was prepared, according to the procedure of Example 16 starting with 3-(4,4-dimethyl-2-oxo-1,4-dihydro-2H-benzo[d][1,3]oxazin-6-yl)-4-fluoro-benzonitrile, as a yellow solid: mp 250-251°C; ¹H-NMR (CDCl₃) δ 8.83 (s, 1H), 7.73 (dd, 1H, *J* = 8.34, 2.19 Hz), 7.68-7.64 (m, 1H), 7.48-7.44 (m, 1H), 7.32-7.28 (m, 2H), 6.9 (d, 1H, *J =* 8.34 Hz), 1.81 (s, 6H); MS (ES) *m*/*z* 311 ([M- H]⁻).

### EXAMPLE 48

### 6-(2,3-Difluorophenyl)-4,4-dimethyl-1,4-dihydro-2H-3,1-benzoxazine-2-thione

6-(2,3-Difluoro-phenyl)-4,4-dimethyl-1,4-dihydro-benzo[d][1,3]oxazin-2-one was prepared, according to the procedure of Example 5 from (1,4-dihydro-4,4-dimethyl-2-oxo-2H-3,1-benzoxazin-6-yl)boronic acid and 2,3-difluorobenzyltriflate, as a yellow solid: mp 166-167 °C; ¹H-NMR (DMSO-*d₆*) δ 10.4 (s, 1H), 7.5-7.2 (m, 5H), 7.0 (m, 1H), 1.7 (s, 6H); MS (EI) m/z 289 ([M+H]⁺); Anal. Calc. For C₁₆H₁₃F₂NO₂: C, 66.43, H, 4.53, N, 4.84. Found: C, 66.15, H, 4.37, N, 4.64.

The title compound was prepared, according to the procedure of Example 16 starting with 6-(2,3-difluoro-phenyl)-4,4-dimethyl-1,4-dihydro-benzo[d][1,3]oxazin-2-one, as a white solid: mp 197-198°C; ¹H-NMR (CDCl₃) δ 9.02 (s, 1H), 7.49-7.45 (m, 1H), 7.34 (s, 1H), 7.2-7.13 (m, 3H), 6.9 (d, 1H, *J*= 8.14 Hz), 1.80 (s, 6H); MS (ES) *m*/*z* 304 ([M- H]⁻).

### EXAMPLE 49

### 3-(8-Bromo-4,4-dimethyl-2-thioxo-1,4-dihydro-2H-3,1-benzoxazin-6-yl)-5-fluorobenzonitrile

To a mixture of 3-(4,4-dimethyl-2-oxo-1,4-dihydro-2H-3,1-benzoxazin-6-yl)-5-fluorobenzonitrile (0.5g, 1.7 mmol) and sodium acetate (0.2g, 2.4 mmol) in acetic acid (5 mL) was added, at room temperature under nitrogen, bromine (0.12 mL, 2.34 mmol). The reaction mixture was stirred for 20 hours and then poured into ice water (30 mL). The precipitate was collected on a filter and washed with water (3x5 mL) to yield 3-(8-bromo-4,4-dimethyl-2-oxo-1,4-dihydro-2H-3,1-benzoxazin-6-yl)-5-fluorobenzonitrile as an off-white solid (0.48g, 75%): mp 216-217 °C;; ¹H-NMR (DMSO-*d*₆) δ 9.78 (s, 1H), 8.18 (t, 1H, *J* = 1.6 Hz), 8.02-8.08 (m, 2H), 7.81 (m, 1H), 7.75 (d, 1H, *J=* 1.8 Hz), 1.66 (s, 6H). MS (ESI) *mlz* 373, 375 [M - H]⁻.

The title compound was prepared according to the procedure of Example 16 starting with 3-(8-bromo-4,4-dimethyl-2-oxo-1,4-dihydro-2H-3,1-benzoxazin-6-yl)-5-fluorobenzonitrile: mp 219-220°C; ¹H-NMR (CDCl₃) δ 9.05 (s, 1H), 7.70 (d, 1H, *J =* 1.98 Hz), 7.6 (m, 1H), 7.48-7.44 (m, 1H), 7.4-7.36 (m, 1H), 7.26 (m, 1H), 1.80 (s, 6H); MS (ES) m/z 304 ([M- H]⁻).

### EXAMPLE 50

### 4,4-Dimethyl-6-(3-nitrophenyl)-1,4-dihydro-2H-3,1-benzoxazine-2-thione

4,4-Dimethyl-6-(3-nitrophenyl)-1,4-dihydrobenzo[d][1,3]oxazin-2-one was prepared, from 6-iodo-4,4-dimethyl-1,4-dihydro-benzo[d][1,3]oxazin-2-one and 3-nitrophenyl boronic acid according to the procedure of Example 4, as a yellowish solid: mp 244-245 °C; ¹H-NMR (DMSO-d₆) δ 10.38 (s, 1H, D₂O exchangeable), 8.47 (s, 1H), 8.14-8.20 (m, 2H), 7.70-7.76 (m, 3H), 7.01 (d, 1H,J= 8.1 Hz), 1.68 (s, 6H); MS (EI) m/z 297([M-H]⁻, 100%); Anal. Calc. For C₁₆H₁₄N₂O₄: C, 64.42, H, 4.73, N, 9.39. Found: C, 63.93, H, 4.91, N, 8.71.

The title compound was prepared, according to the procedure of Example 16 starting with 4,4-dimethyl-6-(3-nitro-phenyl)-1,4-dihydro-benzo[d][1,3]oxazin-2-one, as a yellow solid: mp 224-226°C; ¹H-NMR (CDCl₃) δ 8.89 (s, 1H), 8.40 (s, 1H), 8.26-8.22 (m, 1H), 7.88-7.86 (m, 1H), 7.64 (t, 1H, *J*= 7.97 Hz), 7.57 (dd, 1H, *J*=8.21, 1.3 Hz), 7.40 (m, 1H), 6.94 (d, 1H, *J=* 8.22 Hz),1.80 (s, 6H); MS (ES) *m*/*z* 313 ([M-H]⁻).

### EXAMPLE 51

### 6-(3-Chlorophenyl)-4,4-diethyl-1,4-dihydro-2H-3,1-benzoxazine-2-thione

6-(3-Chlorophenyl)-4,4-diethyl-1,4-dihydrobenzo[d][1,3]oxazin-2-one was prepared, from 4,4-diethyl-6-iodo-1,4-dihydrobenzo[d][1,3]oxazin-2-one and 3-chlorophenyl boronic acid according to the procedure of Example 4, as a white solid: mp 150-151 °C; ¹H-NMR (CDCl₃) δ 8.52 (s, 1H, D₂O exchangeable), 7.50 (s, 1H), 7.31-7.44 (m, 4H), 7.16 (d, 1H, *J=* 1.5 Hz), 6.89 (d, 1H, *J=* 8.2 Hz), 2.03 (m, 4H), 0.94 (t, 6H, *J =* 7.4 Hz); MS (EI) *m*/*z* 315 (M⁺, 53%). Anal. Calc. For C₁₈H₁₈ClNO₂: C, 68.46, H, 5.75, N, 4.44. Found: C, 68.16, H, 5.81, N, 4.32.

The title compound was prepared, according to the procedure of Example 16 starting with 6-(3-chloro-phenyl)-4,4-diethyl-1,4-dihydro-benzo[d][1,3]oxazin-2-one, as a white solid: mp 148-150°C; ¹H-NMR (CDCl₃) δ 9.27 (s, 1H), 7.50-7.45 (m, 2H), 7.40-7.34 (m, 3H), 7.17 (d, 1H**,** *J* = 1.64 Hz), 6.94 (d, 1H, *J* = 8.22 Hz), 2.18-2.01 (m, 4H), 0.94 (t, 6H, *J* = 7.33 Hz); MS (ES) m/z 303/332 ([M- H]⁻).

### EXAMPLE 52

### 6-(3-Methoxyphenyl)-4,4-dimethyl-1,4-dihydro-2H-3,1-benzoxazine-2-thione

6-(3-Methoxy-phenyl)-4,4-dimethyl-1,4-dihydro-benzo [d] [1,3]oxazin-2-one was prepared, according to the procedure of Example 4 from 6-bromo-4,4-dimethyl-1,4-dihydro-benzo[d][1,3]oxazin-2-one and 3-methoxyphenyl boronic acid, as a yellow solid: mp 164-165 °C; ¹H-NMR (DMSO-d₆) δ 10.3 (s, 1H), 7.56 (m, 2H), 7.36 (t, 1 H, *J*= 7.89 Hz), 7.20 (m, 2H), 6.96 (d, 1H,J= 8.88 Hz), 6.91 (dd, 1H, *J=* 8.13, 2.35 Hz), 3.8 (s, 3H), 1.7 (s, 6H); MS (ESI) m/z 284 ([M+H]⁺, 30%); Anal. Calc. For C₁₇H₁₇NO₃: C, 72.07, H, 6.05, N, 4.94. Found: C, 70.58, H, 5.73, N, 4.67.

The title compound was prepared, according to the procedure of Example 16 starting with 6-(3-Methoxy-phenyl)-4,4-dimethyl-1,4-dihydro-benzo[d][1,3]oxazin-2-one, as a white solid: mp 142-143°C; ¹H-NMR (CDCl₃) δ 8.96 (s, 1H), 7.51 (dd, 1H, *J* = 8.2, 1.84 Hz), 7.40-7.35 (m, 2H), 7.13-7.10 (m, 1H), 7.05 (t, 1H, *J =* 2.21 Hz), 6.90 (dd, 1H, *J=* 8.09, 2.46 Hz), 6.87 (d, 1H, *J=* 8.2 Hz), 3.87 (s, 3H), 1.8 (s, 6H); MS (ES) *m*/*z* 298 ([M- H]⁻).

### EXAMPLE 53

### 6-(2-Chlorophenyl)-4,4-dimethyl-1,4-dihydro-2H-3,1-benzozazine-2-thione

6-(2-Chloro-phenyl)-4,4-dimethyl-1,4-dihydro-benzo[d][1,3]oxazin-2-one was prepared, according to the procedure of Example 4 from 6-bromo-4,4-dimethyl-1,4-dihydro-benzo[d][1,3]oxazin-2-one and 2-chlorophenyl boronic acid, as a white solid: mp 181-182 °C; MS (ESI) *m*/*z* 288 ([M+H]⁺, 70%); Anal. Calc. For C₁₆H₁₄ClNO₂: C, 66.79, H, 4.90, N, 4.87. Found: C, 66.78, H, 4.82, N, 4.55.

The title compound was prepared, according to the procedure of Example 16 starting with 6-(2-chloro-phenyl)-4,4-dimethyl-1,4-dihydro-benzo[d][1,3]oxazin-2-one, as a white solid: mp 171-172°C; ¹H-NMR (CDCl₃) δ 8.95 (s, 1H), 7.51-7.47 (m, 1H), 7.40-7.27 (m, 5H), 6.87 (d, 1H, *J*= 8.14 Hz), 1.79 (s, 6H); MS (ES) *m*/*z* 302/304 ([M- H]⁻).

### EXAMPLE 54

### 4-Benzyl-6-(3-chlorophenyl)-4-methyl-1,4-dihydro-2H-3,1-benzoxazine-2-thione

A mixture of 1-(4-amino-3-chloro-biphenyl-3-yl)-1-benzyl-ethanol (prepared from 1-(4-amino-3'-chloro-biphenyl-3-yl)-ethanone and benzylmagnesium bromide according to procedure described previously, 0.14 g, 0.42 mmol) and triphosgene (0.04 g, 0.14 mmol) in dry THF (10 mL) was stirred under a blanket of nitrogen for 10 minutes. THF was removed and the residue purified via flash chromatography (silica gel, 35% ethyl acetate/hexane) to give 4-benzyl-6-(3-chloro-phenyl)-4-methyl-1,4-dihydro-benzo[d][1,3]oxazin-2-one (0.045 g, 30%) as an off-white solid: mp 187-188 °C; ¹H-NMR (DMSO-d₆) δ 10.1 (s, 1H), 7.70 (t, 1H, *J=* 2.3 Hz), 7.6 (d, 1H, *J=* 8.0 Hz), 7.58-7.53 (m, 2H), 7.46 (t, 1H, *J=* 8.0 Hz), 7.38 (d, 1H, *J =* 8.0 Hz), 7.22-7.17 (m, 3H), 7.06-7.0 (m, 2H), 6.84 (d, 1H, *J=* 9.14 Hz), 3.24 (d, 1H, *J=* 14.3 Hz), 3.06 (d, 1H, *J =* 14.3 Hz), 1.68 (s, 3H); MS (ESI) *mlz* 364 ([M+H]⁺, 100%); Anal. Calc. For C₂₂H₁₈CINO₂: C, 72.63; H, 4.99; N, 3.85. Found: C, 71.82; H, 5.09; N,3.58.

The title compound was prepared, according to the procedure of Example 16 starting with 4-benzyl-6-(3-chloro-phenyl)-4-methyl-1,4-dihydro-benzo[d][1,3]oxazin-2-one, as a white solid: ¹H-NMR (CDCl₃) δ 9.09 (s, 1H), 7.63 (dd, 1H, *J=* 8.03, 1.83 Hz), 7.38-7.22 (m, 7H), 7.04-6.97 (m, 3H), 6.83 (d, 1H, *J=* 8.22 Hz), 3.22 (s, 2H), 1.86 (s, 3H); MS (ES) m/z 378/380 ([M- H]⁻).

### EXAMPLE 55

### 6-(3-Bromo-5-fluorophenyl)-4,4-dimethyl-1,4-dihydro-2H-3,1-benzoxazine-2-thione

6-(3-Bromo-5-fluorophenyl)-4,4-dimethyl-1,4-dihydrobenzo[d][1,3]oxazin-2-one was prepared, from (1,4-dihydro-4,4-dimethyl-2-oxo-2H-3,1-benzoxazin-6-yl)boronic acid and 1,3-dibromo-5-fluorobenzene following the procedure of Example 5. as a white solid: mp 182-183 °C; ¹H-NMR (DMSO-d₆) δ 10.36 (s, 1H, D₂O exchangeable), 7.78 (s, 1H), 7.58-7.65 (m, 3H), 7.49 (dd, 1H, *J*= 8.3, 1.8 Hz), 6.96 (d, 1H, *J=* 8.5 Hz), 1.69 (s, 6H); ¹⁹F-NMR (DMSO-d₆) □ -112.46 (m, 1F); MS (CI) *mlz* 352 ([M+H]⁺, 78%), 350 ([M+H]⁺, 75%); Anal. Calc. For C₁₆H₁₃BrFNO₂: C, 54.88, H, 3.74, N, 4.00. Found: C, 54.83, H, 3.82, N, 3.95.

The title compound was prepared, according to the procedure of Example 16 starting with 6-(3-bromo-5-fluoro-phenyl)-4,4-dimethyl-1,4-dihydro-benzo[d][1,3]oxazin-2-one, as a yellow solid: mp 221-222°C; ¹H-NMR (CDCl₃) δ 9.28 (s, 1H), 7.49-7.45 (m, 2H), 7.30 (d, 1H, *J=* 1.71 Hz), 7.24 (t, 1H, *J*= 2.07 Hz), 7.17 (dt, 1H, *J*= 9.54, 1.99 Hz), 6.93 (d, 1H, *J-*= 8.25 Hz), 1.8 (s, 6H); MS (ES) *mlz* 364/366 ([M- H]⁻).

### EXAMPLE 56

### 5-(4,4-Dimethyl-2-thioxo-1,4-dihydro-2H-3,1-benzoxazin-6-yl) thiophene-2-carbonitrile

5-(4,4-Dimethyl-2-oxo-1,4-dihydro-2H-benzo[d][1,3]oxazin-6-yl)-thiophene-2-carbonitrile was prepared, according to the procedure of Example 5 using 5-bromo-2-thiophenecarbonitrile and (1,4-dihydro-4,4-dimethyl-2-oxo-2H-3,1-benzoxazin-6-yl)boronic acid, as an off-white solid: mp 264-266 °C; ¹H-NMR (DMSO-d₆) δ 10.3 (s, 1H), 7.97 (d, 1H, *J=* 7.9 Hz), 7.60-7.66 (m, 3H), 6.96 (d, 1H, *J=* 8.1 Hz), 1.65 (s, 6H); MS (APCI) *m*/*z* 285 (M+H)⁺, 302 (M+NH₄)⁺. Anal. Calc. For C₁₅H₁₂N₂O₂S: C, 63.36; H, 4.25; N, 9.85. Found: C, 63.01; H, 4.36; N, 9.39.

The title compound was prepared, according to the procedure of Example 16 using 5-(4,4-dimethyl-2-oxo-1,4-dihydro-2H-benzo[d][1,3]oxazin-6-yl)-thiophene-2-carbonitrile, as a yellow solid: mp 242-244 °C; ¹H-NMR (CDCl₃) δ 9.05 (s, 1H), 7.61 (d, 1H, *J=* 3.89 Hz), 7.54 (dd, 1H, *J* = 8.23, 1.56 Hz), 7.35 (m, 1H), 7.24 (d, 1H, *J =* 3.89 Hz), 6.88 (d, 1H, *J* -= 8.26 Hz), 1.8 (s, 6H); MS (ES) *m*/*z* 299 ([M- H]⁻).

### EXAMPLE 57

### 3-Fluoro-5-(8-fluoro-4,4-dimethyl-2-thioxo-1,4-dihydro-2H-3,1-benzoxazin-6-yl)benzonitrile

*N*-(*tert*-Butoxycarbonylamino)-3-fluorobenzoic acid (Takagishi et al. Synlett 4, 360-2 (1992); mp 159-161 °C) was deprotected using trifluoroacetic acid to give o-amino benzoic acid, which was then treated with methylmagnesium bromide to afford *o*-amino dimethyl carbinol. The o-amino dimethyl carbinol (2.23 g, 13.2 mmol) was treated with 1,1-carbonyldiinfidizole (2.8 g, 17.2 mmol) in THF (20 mL) at 50 °C for 12 hours. The solution was then cooled to room temperature and ethyl acetate (100 mL) was added. The organic layer was washed with 10% aqueous HCl solution (2x25 mL), dried over MgSO₄ and concentrated. The residue was purified via chromatography (silica gel, 10% ethyl acetate/hexane) to give 8-fluoro-4,4-dimethyl-dihydro-benzo[d][1,3]oxazin-2-one as a white solid (1.3 g, 50%): mp 127-128 °C; ¹H-NMR (DMSO-d₆) δ 10.4 (s, 1H), 7.22-7.12 (m, 2H), 7.07-7.00 (m, 2H), 1.6 (s, 6H); MS (APCI) *m*/*z* 196 ([M + H]⁺, 100%); Anal. Calc. For C₁₀H₁₀FNO₂: C, 61.53, H, 5.16, N, 7.18. Found: C, 61.27, H, 5.37, N, 7.02.

8-Fluoro-(1 ,4-dihydro-4,4-dimethyl-2-oxo-2H-3, 1-benzoxin-6-yl)boronic acid was prepared from 6-bromo-8-Fluoro-4,4-dimethyl-dihydro-benzo[d][1,3]oxazin-2-one using the procedure of Example 4.

3-Fluoro-5-(8-fluoro-4,4-dimethyl-2-oxo-1,4-dihydro-2H-benzo[d][1,3]oxazin-6-yl)-benzonitrile was prepared, from 8-fluoro-(1,4-dihydro-4,4-dimethyl-2-oxo-2H-3,1-benzoxin-6-yl)boronic acid and 5-bromo-3-fluorobenzonitrile according to the procedure of example 5, as a white solid: mp 256-257 °C; ¹H-NMR (DMSO-d₆) δ 10. 5 (s, 1H), 8.20 (bs, 1H), 8.06 (dt, 1H, *J* = 10.48, 2.16 Hz), 7.85-7.82 (m, 1H), 7.77 (dd, 1H, *J=* 11.89, 1.81 Hz), 7.63 (s, 1H), 1.7 (s, 6H); MS (EI) *mlz* 314([M⁺], 60%).

The title compound was prepared, according to the procedure of Example 16 using 3-Fluoro-5-(8-fluoro-4,4-dimethyl-2-oxo-1,4-dihydro-2H-benzo[d][1,3]oxazin-6-yl)-benzonitrile, as a yellow solid: ¹H-NMR (CDCl₃) δ 8.91 (s, 1H), 7.61 (d, 1H), 7.47 (dt, 1H, *J*= 9.25, 2.0 Hz), 7.39 (m, 1H), 7.33-7.29 (m, 1H), 7.13 (s, 1H), 1.8 (s, 6H).

### EXAMPLE 58

### 3-(1,2-Dihydro-2-thioxospiro[4H-3,1-benzoxazine-4,1-cyclohexan]-6-yl)benzonitirile

3-(1,2-Dihydro-2-oxospiro[4H-3,1-benzoxazine-4,1-cyclohexan]-6-yl)-benzonitrile was prepared, according to Procedure B from spiro-(4, 1'-cyclohexane-1,4-dihydro-2-oxo-2H-3,1-benzoxazin-6-yl) boronic acid and 3-bromobenzonitrile, as a tan powder: mp 245-247 °C; ¹H-NMR(DMSO-d₆) δ 10.31 (s, 1H), 8.21 (s, 1H), 8.02 (d, 1H, *J =* 8.0 Hz), 7.78 (d, 1H, *J =* 7.7 Hz), 7.68-7.61 (m, 3H), 6.97 (d, 1H, *J* = 8.2 Hz), 1.98-1.96 (m, 4H), 1.75-1.64 (m, 5H), 1.40-1.32 (m, 1H); MS (EI) m/z 318[M⁺]; Anal. Calc. For C₂₀H₁₈N₂O₂·1/2 H₂O: C 73.38; H, 5.85; N, 8.56. Found: C, 73.86; H, 5. 81; N, 8.22.

The title compound was prepared, according to the procedure of Example 16 starting with 3-(1,2-dihydro-2-oxospiro[4H-3,1-benzoxazine-4,1-cyclohexan]-6-yl)benzonitrile, as a white solid: mp 222-224°C; ¹H-NMR (CDCl₃) δ 9.08 (s, 1H), 7.86-7.81 (m, 1H), 7.77(dt, 1H, *J=* 7.79, 1.35 Hz), 7.67-7.64 (m, 1H), 7.58 (d, 1H, *J* = 7.77 Hz), 7.48 (dd, 1H, *J=* 8.2*,* 1.93 Hz), 7.35 (d, 1H, *J=* 1.78 Hz), 6.91 (d, IH,J = 8.2 Hz), 2.30-2.26 (m, 2H), 2.07-1.98 (m, 2H), 1.90-1.70 (m, 4H), 1,39-1.24 (m, 2H); MS (ES) m/z 333 ([M-H]⁻).

### EXAMPLE 59

### 5-(1,2-Dihydro-2-thioxospiro]4H-3,1-benzoxazine-4,1-cyclohexan)-6-yl)-4-methyl-2-thiophenecarbonitrile

5-(1, 2-Dihydro-2-oxospiro[4H-3, 1-benzoxazine-4,1-cyclohexan]-6-yl)-4-methyl-2-thiophenecarbonitrile was prepared, according to Procedure B from spiro-(4, 1'-cyclohexane-1,4-dihydro-2-oxo-2H-3,1-benzoxazin-6-yl) boronic acid and 2-bromo-3-methyl-5-cyanothiophene, as a white powder: mp 200-203 °C; ¹H-NMR (DMSO-d₆) δ 10.4 (s, 1H), 7.85 (s, 1H), 7.43-7.40 (m, 2H), 7.0 (d, 1H, *J*= 8.8 Hz), 2.27 (s,3 H), 2.00-1.62 (m, 9H), 1.42-1.23 (m, 1H); MS(EI) *m*/*z* 338 (M⁺); Anal. Calc. For C₁₉H₁₈N₂O₂S: C, 67.43; H, 5.36, N, 8.28. Found: C, 67.12; H, 5.45; N, 8.05

The title compound was prepared, according to the procedure for Example 16 starting with 5-(1,2-Dihydro-2-oxospiro[4H-3,1-benzoxazine-4,1-cyclohexan]-6-yl)-4-methyl-2-thiophenecarbonitrile, as a yellow solid: mp 199-201°C; ¹H-NMR (CDCl₃) δ 8.92 (s, 1H), 7.5 (s, 1H), 7.36 (dd, 1H, *J=* 8.17, 1.9 Hz), 7.23 (d, 1H, *J=* 1.7 Hz), 6.87 (d, 1H, *J*= 8.18 Hz), 2.3 (s, 3H), 2.05-1.70 (m, 7H), 1.36-1.25 (m, 3H); MS (ES) m/z 353 ([M-H]⁻).

### EXAMPLE 60

### 5-(1,2-Dihydro-2-thioxospiro[4H-3,1-benzoxazine-4,1-cyclohexan]-6-yl)-2-thiophenecarbonitrile

5-(1, 2-Dihydro-2-oxospiro[4H-3,1-benzoxazine-4,1-cyclohexan]-6-yl)-2-thiophenecarbonitrile was prepared, according to Procedure B from spiro-(4, 1'-cyclohexane-1,4-dihydro-2-oxo-2H-3,1-benzoxazin-6-yl) boronic acid and 2-bromo-5-cyanothiophene, as a tan powder: mp 243-245 °C; ¹H-NMR (DMSO-d₆) δ 10.41(s, 1H), 7.98-7.97 (d, 1H, *J =* 3.9 Hz), 7.67-7.60 (m, 3H), 6.97-6.94 (d, 1H, *J =* 8.3 Hz), 1.98-1.92 (m, 4H), 1.74-1.64 (m, 5H), 1.45-1.21 (m, 1H); MS (EI) *m*/*z* 324 (M⁺). Anal. Calc. For C₁₈H₁₆N₂O₂S 1/2 H₂O: C, 65.08; H, 5.04; N, 8.18. Found: C, 64.84; H, 5.09; N,8.40.

The title compound was prepared, according to the procedure for Example 16 starting with 5-(1,2-Dihydro-2-oxospiro[4H-3,1-benzoxazine-4,1-cyclohexan]-6-yl)-2-thiophenecarbonitrile, as a yellow solid: mp 232-233 °C; ¹H-NMR (CDCl₃) δ 8.90 (s, 1H), 7.6 (d, 1H, *J =* 3.93 Hz), 7.36 (d, 1H, *J*= 1.8 Hz), 7.24-7.20 (m, 1H), 6.85 (d, 1H, *J*= 8.25 Hz), 2.28-2.23 (m, 2H), 2.11-1.96 (m, 2H), 1.90-1.70 (m, 5H), 1.38-1.33 (m 2H); MS (ES) *m*/*z* 339 ([M-H]⁻).

### EXAMPLE 61

### 6-(3-Chloro-4-fluorophenyl)-4,4-dimethyl-1,4-dihydro-2H-3,1-benzoxazine-2-thione

6-(3-Chloro-4-fluoro-phenyl)-4,4-dimethyl-1,4-dihydro-benzo[d][1,3]oxazin-2-one was prepared, from 6-bromo-4,4-dimethyl-1,4-dihydro-benzo[d][1,3]oxazin-2-one and 1-bromo-3-chloro-4-fluorobenzene according to Procedure A, as a white solid: mp 211-212 °C; ¹H-NMR (DMSO-d₆) δ 10.4 (s, 1H), 7.92 (dd, 1H, *J*= 7.13, 2.19 Hz), 7.71-7.66 (m, 1H), 7.60-7.57 (m, 2H), 7.49 (t, 1H, *J* = 8.95 Hz), 6.96 (d, 1H, *J* = 8.01 Hz), 1.67 (s, 6H); MS (EI) *m*/*z* 305 ([M + H]⁺, 20%); Anal. Calc. For C₁₆H₁₃ClFNO₂: C, 62.86, H, 4.29, N, 4.58. Found: C, 62.52, H, 4.45, N, 4.42.

The title compound was prepared, according to the procedure for Example 16 starting with 6-(3-Chloro-4-fluoro-phenyl)-4,4-dimethyl-1,4-dihydro-benzo[d][1,3]oxazin-2-one, as a white solid: mp 196-197 °C;'H-NMR (CDCl₃) δ 9.29 (s, 1H), 7.55 (dd, 1H, *J = 6.89, 2.28* Hz), 7.45 (dd, 1H, *J*= 8.21, 1.91 Hz), 7.41-7.27 (m, 1H), 7.28-7.27 (m, 1H), 7.22 (t, 1H,J= 8.66 Hz), 6.92 (d, 1H, *J =* 8.22 Hz), 1. 81 (s, 6H); MS (ES) m/z 320 ([M-H]⁻).

### EXAMPLE 62

### 5-(4,4-Dimethyl-2-thioxo-1,4-dihydro-2H-3,1-benzozazin-6-yl)-4-propylthiophene-2-carbonitrile

5-(4,4-Dimethyl-2-oxo-1,4-dihydro-2H-benzo[d][1,3]oxazin-6-yl)-4-*n*-propyl-thiophene-2-carbonitrile was prepared according to Procedure B from spiro-(4, 1'-cyclohexane-1,4-dihydro-2-oxo-2H-3,1-benzoxazin-6-yl) boronic acid and 2-bromo-3-*n-*propyl-5-thiophenecarbonitrile. White crystals: mp 160-162 °C; IR (KBr) 2220 cm⁻¹; ¹H-NMR (DMSO-d₆) δ 10.47 (s, 1H), 7.93 (s, 1H), 7.38-7.36 (m, 2H), 7.01 (d, 1H, *J=* 8.7 Hz), 2.59-2.48 (m, 2H), 1.64-1.51 (m, 2H), 0.85 (t, 3H, *J=* 7.3 Hz). MS(-ESI) *m*/*z* [M-H]⁻ 325; Anal. Calc. For C₁₈H₁₈N₂O₂S·3/4H₂O: C, 63.60; H, 5.78, N, 8.24. Found: C, 63.48; H, 5.59; N, 8.04.

The title compound was prepared according to the procedure for Example 16 starting with 5-(4,4-Dimethyl-2-oxo-1,4-dihydro-2H-benzo[d][1,3]oxazin-6-yl)-4-propyl-thiophene-2-carbonitrile. A yellow solid: mp 174-175°C; ¹H-NMR (CDCl₃) δ 9.43 (s, 1H), 7.49 (s, 1H), 7.35 (dd, 1H, *J =* 8.17, 1.8 Hz), 7.19 (d, 1H, *J =* 1. 62 Hz), 6.95 (d, 1H, *J=* 8.18 Hz), 2.56 (t, 2H, *J =* 7.53 Hz), 1.79 (s, 6H), 1.60 (m, 2H, *J* = 7.56 Hz), 0.92 (t, 3H, *J* =7.3 Hz); MS (ES) m/z 341 ([M-H]⁻)

### EXAMPLE 63

### 4-(4,4-Dimethyl-2-thioxo-1,4-dihydro-2H-3,1-benzoxazin-6-yl)-2-furonitrile

4-(4,4-Dimethyl-2-oxo-1,4-dihydro-2H-benzo[d][1,3]oxazin-6-yl)-furan-2-carbonitrile was prepared from (1,4-dihydro-4,4-dimethyl-2-oxo-2H-3,1-benzoxazin-6-yl)boronic acid and 4-bromo-2-furancarbonitrile according to Procedure B. Off-white solid: mp 255-256 °C. ¹H-NMR (DMSO-d₆) δ 10.32 (s, 1H, D₂O exchangeable), 8.57 (s, 1H), 8.15 (s, 1H), 7.61 (s, 1H), 7.55 (dd, 1H, *J*= 8.3, 1.5 Hz), 6.92 (d, 1H, *J=* 8.2 Hz), 1.65 (s, 6H); MS (ESI) *m*/*z* 269(M+H, 72%). Anal. Calc. For C₁₅H₁₂N₂O₃: C, 67.16, H, 4.51, N, 10.44. Found: C, 67.14, H, 4.59, N, 10.07.

The title compound was prepared according to the procedure for Example 16 starting with 4-(4,4-dimethyl-2-oxo-1,4-dihydro-2H-benzo[d][1,3]oxazin-6-yl)-furan-2-carbonitrile. An off-white solid: mp 214-216°C; ¹H-NMR (CDCl₃) δ 8.93 (s, 1H), 7.83 (s, 1H), 7.39 (dd, 1H,*J*= 8.2, 1.87 Hz), 7.35 (s, 1H), 7.22-7.21 (m, 1H), 6.86 (d, 1H, *J* = 8.2 Hz), 1.79 (s, 6H); MS (ES) m/z 283 ([M-H]⁻).

### EXAMPLE 64

### 4-Butyl-5-(4,4-dimethyl-2-thioxo-1,4-dihydro-2H-3,1-benzoxazin-6-yl)thiophene-2-carbonitrile

5-(4,4-Dimethyl-2-oxo-1,4-dihydro-2H-benzo[d][1,3]oxazin-6-yl)-4-*n*-butyl-thiophene-2-carbonitrile was prepared according to Procedure B from spiro-(4, 1'-cyclohexane-1,4-dihydro-2-oxo-2H-3,1-benzoxazin-6-yl) boronic acid and 2-bromo-3-n-butyl-5-thiophenecarbonitrile. White crystals: mp 167-168 °C; ¹H-NMR (DMSO-d₆) δ 10.46 (s, 1H), 7.93 (s, 1H), 7.38-7.36 (m, 2H), 7.01 (d, 1H, *J*= 8.7 Hz), 2.59 (t, 2H, *J* = 8.1 Hz), 1.63 (s, 6H), 1.58-1.51 (m, 2H), 1.48-1.17 (m, 2H), 0.82 (t, 3H, *J*= 7.4 Hz). MS(-ESI) m/z [M-H]⁻ 339. Anal. Calc. For C₁₉H₂₀N₂O₂S·1/4 H₂O: C, 66.16; H, 5.99; N, 8.12. Found: C, 66.33; H, 5.92; N, 7.85.

The title compound was prepared according to the procedure for Example 16 starting with 4-Butyl-5-(4,4-dimethyl-2-oxo-1,4-dihydro-2H-benzo[d][1,3]oxazin-6-yl)-thiophene-2-carbonitrile. A yellow solid: mp 174-175°C;¹H-NMR (CDCl₃) δ 9.58 (s, 1H), 7.50 (s, 1H), 7.35 (dd, 1H, *J =* 8.19, 1. 84 Hz), 7.19 (d, 1H, *J =* 1. 7 Hz), 6. 96 (d, 1H, *J=* 8.1,8 Hz), 2.58 (t, 2H, *J=* 7.61 Hz), 1.80 (s, 6H), 1.61- 1.54 (m, 2H), 1.35-1.25 (m, 2H), 0.88 (t, 3H, *J=* 7.29 Hz): MS (ES) m/z 355 ([M-H]⁻).

### EXAMPLE 65

### 6-(3-Bromophenyl)-4,4-dimethyl-1,4-dihydro-2H-3,1-benzoxazine-2-thione

6-(3-Bromo-phenyl)-4,4-dimethyl-1,4-dihydro-benzo[d][1,3]oxazin-2-one was prepared from (1,4-dihydro-4,4-dimethyl-2-oxo-2H-3,1-benzoxin-6-yl)boronic acid and 1,3-dibromobenzene according to procedure B. A white solid: mp 174-175 °C; ¹H-NMR (DMSO-d₆) δ 10.35 (s, 1H), 7.88 (bs, 1H), 7.68 (d, 1H, *J*= 7.5 Hz), 7.6-7.51 (m, 3H), 7.4 (t, 1H, *J=* 7.5 Hz), 6.97 (d, 1H, *J=* 8.57 Hz), 1.64 (s, 6H); MS (EI) *m*/*z* 331([M⁺], 60%), 333([M⁺], 60%); Anal. Calc. For C₁₆H₁₄BrNO₂: C, 57.85, H, 4.25, N, 4.22. Found: C, 57.7, H, 4.36, N, 4.09.

A mixture of 6-(3-bromo-phenyl)-4,4-dimethyl-1,4-dihydro-benzo[d][1,3]oxazin-2-one (3 g, 9.04 mmol) and Lawesson's Reagent (1.83 g, 4.51 mmol) in toluene (30 mL) was heated to 110°C for 24 hours. The reaction was cooled, the toluene removed *in vacuo* and the residue purified via flash chromatography (silica gel, 20% ethyl acetate/hexane) to give 6-(3-bromophenyl)-4,4-dimethyl-1,4-dihydro-2H-3,1-benzoxazine-2-thione (1.93 g, 61%) as a yellow solid: mp 191-193°C; ¹H-NMR (DMSO-d6) δ 12.3 (s, 1H), 7.92 (s, 1H), 7.72-7.65 (m, 3H), 7.57-7.54 (m, 1H); 7.45-7.39 (m, 1H), 7.14-7.11 (m, 1H), 1.7 (s, 6H); MS (ES) m/z 347 ([M-H]⁻, 100%); Anal. Calc. For C₁₆H₁₄BrNOS: C, 55.18, H, 4.05, N, 4.02 Found: C, 55.17, H, 3.93, N, 3.97

### EXAMPLE 66

### 2-(4,4-Dimethyl-2-thioxo-1,4-dihydro-2H-3,1-benzoxazin-6-yl)thiophene-3-carbonitrile

2-(4,4-Dimethyl-2-oxo-1,4-dihydro-2H-benzo[d][1,3]oxazin-6-yl)-thiophene-3-carbonitrile was prepared according to procedure B from (1,4-dihydro-4,4-dimethyl-2-oxo-2H-3,1-benzoxin-6-yl)boronic acid and 2-bromo-3-thiophenecarbonitrile. An off-white solid: mp 200-202 °C; ¹H-NMR (DMSO-d₆) δ 10.49 (s, 1H), 7.75(m, 1H),7.63(d, 1H, *J =* 2.2 Hz), 7.59 (m, 1H), 7.50 (m, 1H), 7.02 (d, 1H, *J* = 8.1 Hz), 1.63(s, 6H); MS(-ESI) m/z 283 (M-H).

The title compound was prepared according to the procedure for Example 16 starting with 2-(4,4-Dimethyl-2-oxo-1,4-dihydro-2H-benzo[d][1,3]oxazin-6-yl)-thiophene-3-carbonitrile. A yellow solid: mp 194-195°C; ¹H-NMR (CDCl₃) δ 9.56 (s, 1H), 7.67-7.62 (m, 2H), 7.35 (d, 1H, *J=* 5.39 Hz), 7.30 (d, 1H, *J=* 5.33 Hz), 6.98 (d, 1H, *J =* 8.18 Hz), 1.80 (s, 6H); MS (ES) m/z 299 ([M-H]⁻).

## Claims

1. A compound selected from the group consisting of (I), (II), (III), (IV), and (V):
(I) a compound of the formula: 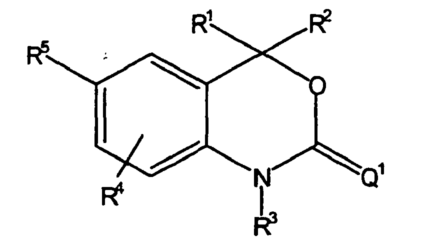 wherein:
R¹ and R² are independent substituents selected from the group consisting of H, C₁ to C₆ alkyl, substituted C₁ to C₆ alkyl, C₂ to C₆ alkenyl, substituted C₂ to C₆ alkenyl, C₂ to C₆ alkynyl, substituted C₂ to C₆ alkynyl, C₃ to C₈ cycloalkyl, substituted C₃ to C₈ cycloalkyl, aryl, substituted aryl, heterocyclic, substituted heterocyclic, COR^{A}, and NR^{B}COR^{A};
or R¹ and R² are fused to form a ring selected from the group consisting of a), b) and c), each ring being optionally substituted by from 1 to 3 substituents selected from the group consisting of H and C₁ to C₃ alkyl:
a) a carbon-based 3 to 8 membered saturated spirocyclic ring;
b) a carbon-based 3 to 8 membered spirocyclic ring having in its backbone one or more carbon-carbon double bonds; and
c) a 3 to 8 membered spirocyclic ring containing in its backbone one to three heteroatoms selected from the group consisting of O, S and N;
R^{A} is selected from the group consisting of H, C₁ to C₃ alkyl, substituted C₁ to C₃ alkyl, aryl, substituted aryl, C₁ to C₃ alkoxy, substituted C₁ to C₃ alkoxy, amino, C₁ to C₃ aminoalkyl, and substituted C₁ to C₃ aminoalkyl;
R^{B} is H, C₁ to C₃ alkyl, or substituted C₁ to C₃ alkyl;
R³ is H, OH, NH₂, C₁ to C₆ alkyl, substituted C₁ to C₆ alkyl, C₃ to C₆ alkenyl, substituted C₃ to C₆ alkenyl, alkynyl, substituted alkynyl, or COR^{c};
R^{C} is selected from the group consisting of H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, and substituted C₁ to C₃ aminoalkyl;
R⁴ is selected from the group consisting ofH, halogen, CN, NO₂, C₁ to C₆ alkyl, substituted C₁ to C₆ alkyl, C₁ to C₆ alkoxy, substituted C₁ to C₆ alkoxy, C₁ to C₆ aminoalkyl, and substituted C₁ to C₆ aminoalkyl;
R⁵ is selected from the goup consisting of (i) and (ii):
(i) a substituted benzene ring containing the substituents X, Y and Z as shown below: 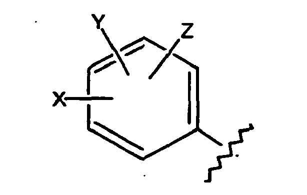 X is selected from the group consisting of H, halogen, CN, C₁ to C₃ alkyl, substituted C₁ to C₃ alkyl, C₁ to C₃ alkoxy, substituted C₁ to C₃ alkoxy, C₁ to C₃ thioalkoxy, substituted C₁ to C₃ thioalkoxy, C₁ to C₃ aminoalkyl, substituted C₁ to C₃ aminoalkyl, NO₂, C₁ to C₃ perfluoroalkyl, 5 or 6 membered heterocyclic ring containing in its backbone 1 to 3 heteroatoms, COR^{D}, OCOR^{D}, and NR^{E}COR^{D};
R^{D} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
R^{E} is H, C₁ to C₃ alkyl, or substituted C₁ to C₃ alkyl;
Y and Z are independent substituents selected from the group consisting of H, halogen, CN, NO₂, C₁ to C₃ alkoxy, C₁ to C₃ alkyl, and C₁ to C₃ thioalkoxy;
wherein not all of X, Y, and Z are H; and
(ii) a five or six membered heterocyclic ring having in its backbone 1, 2, or 3 ring heteroatoms selected from the group consisting of O, S, S(O), S(O₂) and NR⁶ and containing one or two independent substituents selected from the group consisting of H, halogen, CN, NO₂, C₁ to C₃ alkyl, C₁ to C₃ alkoxy, C₁ to C₃ aminoalkyl, COR^{F}, and NR^{G}COR^{F};
R^{F} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
R^{G} is H, C₁ to C₃ alkyl, or substituted C₁ to C₃ alkyl;
R⁶ is H or C₁ to C₃ alkyl;
Q¹ is S, NR⁷, or CR⁸R⁹;
R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, SO₂CF₃, OR¹¹ and NR¹¹R¹²;
R⁸ and R⁹ are independent substituents selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂ CN, and CO₂R¹⁰;
R¹⁰ is C₁ to C₃ alkyl;
or CR⁸R⁹ is a six membered ring as shown by the structure below: 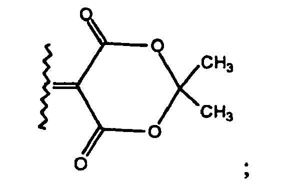 R¹¹ and R¹² are independently selected from the group consisting of H, C₁ to C₆ alkyl, substituted C₁ to C₆ alkyl, aryl, substituted aryl, heteroaryl, substituted heteroaryl, acyl and sulfonyl;
(II) a compound of the formula: 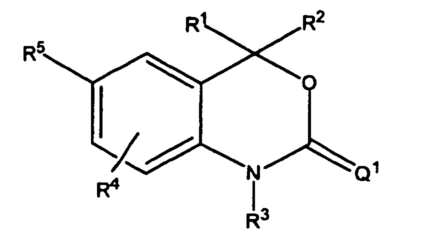 wherein:
R¹ is.H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, COR^{A}, or NR^{B}COR^{A};
R² is H, C₁ to C₆ alkyl, substituted C₁ to C₆ alkyl, C₂ to C₆ alkenyl, substituted C₂ to C₆ alkenyl, C₃ to C₈ cycloalkyl, substituted C₃ to C₈ cycloalkyl, aryl, substituted aryl, heterocyclic, substituted heterocyclic, COR^{A}, or NR^{B}COR^{A};
or R¹ and R² are fused to form a spirocyclic ring selected from the group consisting of a), b) and c), each ring being optionally substituted by from 1 to 3 substituents selected from the group consisting of H and C₁ to C₃ alkyl:
a) a carbon based 3 to 8 membered saturated spirocyclic ring;
b) a carbon-based 3 to 8 membered spirocyclic ring having in its backbone one or more carbon-carbon double bonds; and
c) a 3 to 8 membered spirocyclic ring containing in its backbone one to three heteroatoms selected from the group consisting of O, S and N;
R^{A} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
R^{B} is H, C₁ to C₃ alkyl, or substituted C₁ to C₃ alkyl;
R³ is H, OH, NH₂, C₁ to C₆ alkyl, substituted C₁ to C₆ alkyl, C₃ to C₆ alkenyl, substituted C₃ to C₆ alkenyl, alkynyl, substituted alkynyl, or COR^{C};
R^{C} is H, C₁ to C₄ alkyl, substituted C₁ to C₄ alkyl, aryl, substituted aryl, C₁ to C₄ alkoxy, substituted C₁ to C₄ alkoxy, C₁ to C₄ aminoalkyl, or substituted C₁ to C₄ aminoalkyl;
R⁴ is H, halogen, CN, NO₂, C₁ to C₆ alkyl, substituted C₁ to C₆ alkyl, C₁ to C₆ alkoxy, substituted C₁ to C₆ alkoxy, C₁ to C₆ aminoalkyl, or substituted C₁ to C₆ aminoalkyl;
R⁵ is (i) or (ii):
(i) a substituted benzene ring containing the substituents X, Y and Z as shown below: 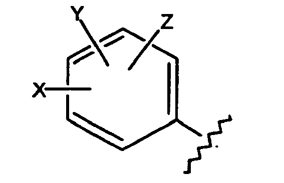 X is selected from the group consisting of H, halogen, CN, C₁ to C₃ alkyl, substituted C₁ to C₃ alkyl, C₁ to C₃ alkoxy, substituted C₁ to C₃ alkoxy, C₁ to C₃ thioalkoxy, substituted C₁ to C₃ thioalkoxy, C₁ to C₃ aminoalkyl, substituted C₁ to C₃ aminoalkyl, NO₂, C₁ to C₃ perfluoroalkyl, 5 membered heterocyclic ring containing in its backbone 1 to 3 heteroatoms, COR^{D}, OCOR^{D}, and NR^{E}COR^{D};
R^{D} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
R^{E} is H, C₁ to C₃ alkyl, or substituted C₁ to C₃ alkyl;
Y and Z are independent substituents selected from the group consisting of H, halogen, CN, NO₂, C₁ to C₃ alkoxy, C₁ to C₃ alkyl, and C₁ to C₃ thioalkoxy;
wherein not all of X, Y, and Z are H; or
(ii) a five or six membered ring having in its backbone 1, 2, or 3 heteroatoms selected from the group consisting of O, S, SO, SO₂ and NR⁶ and containing one or two independent substituents selected from the group consisting of H, halogen, CN, NO₂, C₁ to C₃ alkyl, and C₁ to C₃ alkoxy,
R⁶ is H or C₁ to C₃ alkyl;
Q¹ is S, NR⁷, or CR⁸R⁹;
R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃;
R⁸ and R⁹ are independent substituents selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂, CN, and CO₂R¹⁰;
R¹⁰ is C₁ to C₃ alkyl;
or CR⁸R⁹ is a six membered ring as shown by the structure below: 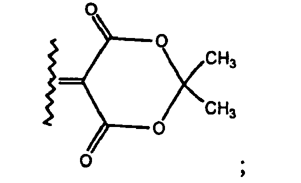
(III) a compound of the formula: 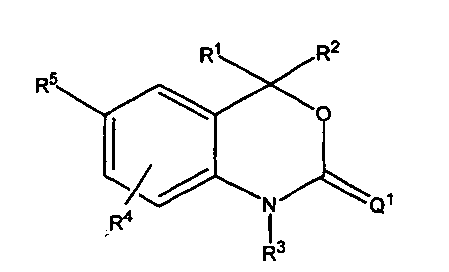 wherein:
R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
R³ is H, OH, NH₂, C₁ to C₆ alkyl, substituted C₁ to C₆ alkyl, or COR^{c};
R^{C} is H, C₁ to C₄ alkyl, or C₁ to C₃ alkoxy;
R⁴ is H, halogen, NO₂, C₁ to C₃ alkyl, or substituted C₁ to C₃ alkyl;
R⁵ is (i) or (ii):
(i) a substituted benzene ring of the structure: 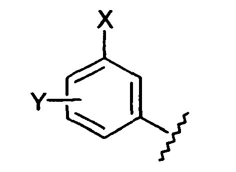 wherein:
X is selected from the group consisting of halogen, CN, C₁ to C₃ alkoxy, C₁ to C₃ alkyl, NO₂, C₁ to C₃ perfluoroalkyl, 5 membered heterocyclic ring containing in its backbone 1 to 3 heteroatoms, and C₁ to C₃ thioalkoxy;
Y is selected from the group consisting of H, halogen, CN, NO₂, C₁ to C₃ alkoxy, C₁ to C₄ alkyl, and C₁ to C₃ thioalkoxy; or
(ii) a five-membered ring of the structure: 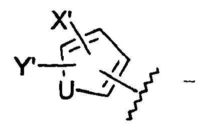 wherein:
X' is selected from the group consisting of halogen, CN, C₁ to C₃ alkoxy, C₁ to C₃ alkyl, and NO₂;
Y' is selected from the group consisting of H, halogen, CN, NO₂, C₁ to C₃ alkoxy, C₁ to C₄ alkyl, and C₁ to C₃ thioalkoxy;
U is O, S, or NR⁶;
R⁶ is H, C₁ to C₃ alkyl, or C₁ to C₄ CO₂alkyl;
Q¹ is S, NR⁷, or CR⁸R⁹;
R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃;
R⁸ and R⁹ are independent substituents selected from the group consisting of H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, NO₂, CN, and CO₂R¹⁰;
R¹⁰ is C₁ to C₃ alkyl;
or CR⁸R⁹ is a six membered ring as shown by the structure below 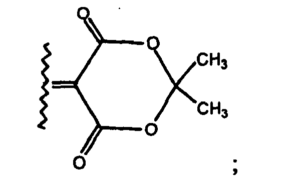
(IV) a compound of the formula: 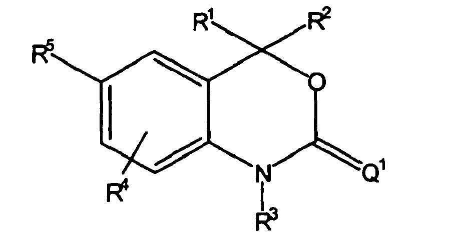 wherein:
R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
R³ is H;
R⁴ is H, halogen, NO₂, C₁ to C₃ alkyl, or substituted C₁ to C₃ alkyl;
R⁵ is the 6-membered ring of the structure: 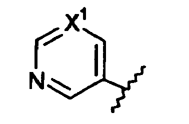 wherein:
X¹ is N; and
Q¹ is S, NR⁷, or CR⁸R⁹; and
with the proviso for each of compounds (I) to (IV) that when Q¹ is S and R³ is H; both R¹ and R² are not H;
with the proviso for each of compounds (I) to (IV) that when Q¹ is S; R³ is H; R⁵ is a benzene ring (i) substituted with one or two substituents or R⁵ is the 5 or 6 membered ring (ii) having in its backbone 1, 2, or 3 heteroatoms selected from the group consisting of O, S, and NR⁶, and one of R¹ or R² is C₂ to C₄ alkenyl, substituted C₂ to C₄ alkenyl, C₂ to C₄ alkynyl, or substituted C₂ to C₄ alkynyl, the other of R² or R¹ is not C₁ to C₆ alkyl, substituted C₁ to C₆ alkyl, C₃ to C₅ cycloalkyl, substituted C₃ to C₅ cycloalkyl, C₂ to C₄ alkenyl, substituted C₂ to C₄ alkenyl, C₂ to C₄ alkynyl, nor substituted C₂ to C₄ alkynyl;
with the proviso for each of compounds (I) to (IV) that when Q¹ is S; R³ is H; R⁵ is a benzene ring (i) substituted with one or two substituents or R⁵ is the 5 or 6 membered ring (ii) having in its backbone 1, 2, or 3 heteroatoms selected from the group consisting of O, S, and NR⁶, and one of R¹ or R² is C₁ to C₆ alkyl or substituted C₁ to C₆ alkyl, the other of R² or R¹ is not C₃ to C₈ cycloalkyl nor substituted C₃ to C₈ cycloalkyl;
with the proviso that for each of compounds (I) to (IV) the term "substituted alkyl" does not include perfluorinated alkyl;
(V) 6-[3-Fluoro-5-(trifluoromethyl)phenyl]-4,4-dimethyl-1,4-dihydro-2H-3,1-benzoxazine-2-thione;
or a pharmaceutically acceptable salt thereof.
(VI) 6-(3-Bromo-6-trifluoromethoxyphenyl)-4,4-dimethyl-1,4-dihydro-2H-3,1-benzoxazine-2-thione.

2. A compound according to claim 1, wherein:
R¹ is H, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, COR^{A}, or NR^{B}COR^{A};
R² is H, C₁ to C₆ alkyl, substituted C₁ to C₆ alkyl, C₂ to C₆ alkenyl, substituted C₂ to C₆ alkenyl, C₃ to C₈ cycloalkyl, substituted C₃ to C₈ cycloalkyl, aryl, substituted aryl, heterocyclic, substituted heterocyclic, COR^{A}, or NR^{B}COR^{A};
or R¹ and R² are fused to form a ring selected from the group consisting of a), b) and c):
a) a carbon-based 3 to 6 membered saturated spirocyclic ring;
b) a carbon-based 3 to 6 membered spirocyclic ring having in its backbone one or more carbon-carbon double bonds; and
c) a 3 to 6 membered spirocyclic ring containing in its backbone one to three heteroatoms;
R^{A} is H, C₁ to C₃ alkyl, substituted C₁ to C₃ alkyl, aryl, substituted aryl, C₁ to C₃ alkoxy, substituted C₁ to C₃ alkoxy, C₁ to C₃ aminoalkyl, or substituted C₁ to C₃ aminoalkyl;
R⁵ is a five or six membered ring, wherein said one or two independent substituents are selected from the group consisting of H, halogen, CN, NO₂, C₁ to C₃ alkyl, and C₁ to C₃ alkoxy;
R₇ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃.

3. A compound according to claim 1, wherein:
R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
R³ is H, OH, NH₂, C₁ to C₆ alkyl, substituted C₁ to C₆ alkyl, or COR^{C};
R^{C} is H, C₁ to C₃ alkyl, or C₁ to C₃ alkoxy;
R⁴ is H, halogen, NO₂, C₁ to C₃ alkyl, or substituted C₁ to C₃ alkyl;
R⁵ is a substituted benzene ring of the structure: .. 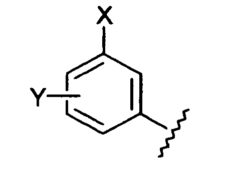 wherein:
X is selected from the group consisting of halogen, CN, C₁ to C₃ alkoxy, C₁ to C₃ alkyl, NO₂, C₁ to C₃ perfluoroalkyl, 5 membered heterocyclic ring containing in its backbone 1 to 3 heteroatoms, and C₁ to C₃ thioalkoxy;
Y is selected from the group consisting of H, halogen, CN, NO₂, C₁ to C₃ alkoxy, C₁ to C₄ alkyl, and C₁ to C₃ thioalkoxy;
R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃.

4. A compound according to claim 1, wherein:
R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
R³ is H;
R^{C} is H, C₁ to C₃ alkyl, or C₁ to C₃ alkoxy;
R⁴ is H, halogen, NO₂, C₁ to C₃ alkyl, or substituted C₁ to C₃ alkyl;
R⁵ is a five-membered ring of the structure: 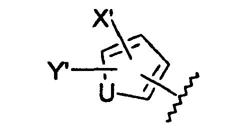
wherein:
X' is selected from the group consisting of halogen, CN, C₁ to C₃ alkoxy, C₁ to C₃ alkyl, NO₂, C₁ to C₃ perfluoroalkyl, 5 membered heterocyclic ring containing in its backbone 1 to 3 heteroatoms, and C₁ to C₃ thioalkoxy;
Y' is selected from the group consisting of H, halogen, CN, NO₂, C₁ to C₃ alkoxy, C₁ to C₃ alkyl, and C₁ to C₃ thioalkoxy;
U is O, S, or NR⁶;
R⁷ is selected from the group consisting of CN, C₁ to C₆ alkyl, substituted C₁ to C₆ alkyl, C₃ to C₈ cycloalkyl, substituted C₃ to C₈ cycloalkyl, aryl, substituted aryl, heterocyclic, substituted heterocyclic, and SO₂CF₃.

5. A compound according to claim 1, wherein:
R¹ and R² and are independently selected from the group consisting of C₁ to C₃ alkyl and substituted C₁ to C₃ alkyl, or R¹ and R² are fused to form a carbon-based 3 to 6 membered saturated spirocyclic ring;
R³ is H;
R⁴ is H, halogen, NO₂, C₁ to C₃ alkyl, or substituted C₁ to C₃ alkyl ;
R⁵ is a 6-membered ring of the structure: 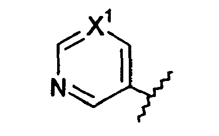
wherein:
X¹ is CX²;
X² is halogen, CN, or NO₂.

6. A compound according to claim 1, which is 6-(3-Chlorophenyl)4,4- dimethyl-1,4-dihydro-benzo[d][1,3]oxazin-2-thione, 4-(4,4-Dimethyl-2-thioxo-1,4-dihydro-2H-benzo[d][1,3]oxazin-6-yl)-thiophene-2-carbonitrile, 3-(4,4-Dimethyl-2-thioxo-1,4-dihydro-2H-benzo[d][1,3]oxazin-6-yl)-5-fluorobenzonitrile, 3-(4,4-Dimethyl-2-thioxo-1,4-dihydro-2H-benzo[d][1,3]oxazin-6-yl)-benzonitrile,5-(4,4-Dimethyl-2-thioxo-1,4-dihydro-2H-3,1-benzoxazin-6-yl)-1-methyl-1H-pyrrole-2-carbonitrile, 6-(3-fluorophenyl)-4-methyl-1,4-dihydro-2H-3,1- benzoxazine-2-thione, 5-(4,4-Dimethyl-2-thioxo-1,4-dihydro-2H-3,1-benzoxazin-6-yl)-4-methylthiophene-2-carbonitrile, 5-(4,4-Dirnethyl-2-thioxo-1,4-dihydro-2H-3,1-benzoxazin-6-yl)-1H-pyrrole-2-carbonitrile, [6-(4,4-dimethyl-2thioxo-1,4-dihydro-2H-3,1-benzoxazin-6-yl)pyridin-2-yl]acetonitrile, 5-(4,4-dimethyl-2-thioxo-1,4-dihydro-2H-3,1-benzoxazin-6-yl)-1H-pyrrole-2-carbothiamide, 5-(4,4-Dimethyl-2-thioxo-1,4-dihydro-2H-benzo[d][1,3]oxazin-6-yl)thiophene-3-carbonitrile, 5-(4,4-dimethyl-2-thioxo-1,4-dihydro-2H-3,1-benzoxazin-6-yl)-1-ethyl-1H-pyrrole-2-carbonitnle, 4-(1,2-Dihydro-2-thioxospiro[4H-3,1-benzoxazin-4,1-cyclohexan]-6-yl)-2-thiophenecarbonitrile,5-(4,4-Dimethyl-2-thioxo-1,4-dihydro-2H-3, 1-benzoxazin-6-yl)-2-fluorobenzonitrile, 6-(5-Bromopyridin-3-yl)-4,4-dimethyl-1,4-dihydro-2H-3,1 -benzoxazine-2-thionc, 6-(3-Chloro-5-fluorophenyl)-4,4-dimethyl-1,4-dihydro-2H-3,1-benzoxazine-2-thione, 6-(3-Bromo-5-methylphenyl)-4,4-dimethyl-1,4-dihydro-2H-3,1-benzoxazine-2-thione, 3-(1,2-Dihydro-2-thioxospiro[4H-3,1-benzoxazine-4,1-cyclohexan]-6-yl)-5-fluorobenzonitrile, 3-(4,4-Dimethyl-2-thioxo-1,4-dihydro-2H-3,1-benzoxazin-6-yl)-5-methylbenzonitrile, 6-(3,5-Dichlorophenyl)-4,4-dimethyl-1,4-dihydro-2H-3,1-benzoxazine-2-thione, 5-(4,4-Dimethyl-1,2-thioxo-1,4-dihydro-2H-3,1-benzoxazin-6-yl)isophthalonitrile, 5-(4,4-Dimethyl-2-thioxo-1,4-dihydro-2H-3,1-benzoxazin-6-yl)-2-furonitrile, 4,4-Diethyl-6-(3-nitrophenyl)-1,4-dihydro-2H-3,1-benzoxazine-2-thione, 6-(3-Chlorophenyl)-4-methyl-4-phenyl-1,4-dihydro-2H-3,1-benzoxazine-2-thione, 4-Allyl-6-(3-chlorophenyl)-4-methyl-1,4-dihydro-2H-3,1-benzoxazine-2-thione, 3-Chloro-5-(4,4-dimethyl-2-thioxo-1,4-dihydro-2H-3,1-benzoxazin-6-yl)benzonitrile, 6-(3,5-Difluorophenyl)-4,4-dimethyl-1,4-dihydro-2H-3,1-benzoxazine-2-thione, 6-(3-Fluoro-5-methoxyphenyl)-4,4-dimethyl-1,4-dihydro-2H-3,1-benzoxazine-2-thione, 3-(4,4-Dimethyl-2-thioxo-1,4-dihydro-2H-3,1-benzoxazin-6-yl)-5-methoxybenzonitrile, 6-(3-Fluorophenyl)-4,4-dimethyl-1,4-dihydro-2H-3,1-benzoxazine-2-thione, 6-(2-Fluorophenyl)-4,4-dimethyl-1,4-dihydro-2H-3,1-benzoxazine-2-thione, 6-(3,4-Difluorophenyl)-4,4-dimethyl-1,4-dihydro-2H-3,1-benzoxazine-2-thione, 6-(4-Fluorophenyl)-4,4-dimethyl-1,4-dihydro-2H-3,1-benzoxazine-2-thione, 3-(4,4-Dimethyl-2-thioxo-1,4-dihydro-2H-3,1-benzoxazin-6-yl)-4-fluorobenzonitrile, 6-(2,3-Difluorophenyl)-4,4-dimethyl-1,4-dihydro-2H-3,1-benzoxazine-2-thione, 3-(8-Bromo-4,4-dimethyl-2-thioxo-1,4-dihydro-2H-3,1-benzoxazm-6-yl)-5-fluorobenzonitrile, 4,4-Dimethyl-6-(3-nitrophenyl)-1,4-dihydro-2H-3,1-benzoxazine-2-thione, 6-(3-Chlorophenyl)-4,4-diethyl-1,4-dihydro-2H-3,1-benzoxazine-2-thione, 6-(3-Methoxyphenyl)-4,4-dimethyl-1,4-dihydro-2H-3,1-benzoxazine-2-thione, 6-(2-Chlorophenyl)4,4-dimethyl-1,4-dihydro-2H-3,1-benzoxazine-2-thione, 4-Benzyl-6-(3-chlorophenyl)-4-methyl-1,4-dihydro-2H-3,1-benzoxazine-2-thione, 6-(3-Bromo-5-fluorophenyl)-4,4-dimethyl-1,4-dihydro-2H-3,1-benzoxazine-2-thione, 5-(4,4-Dimethyl-2-thioxo-1,4-dihydro-2H-3,1-benzoxazin-6-yl) thiophene-2-carbonitrile, 3-Fluoro-5-(8-fluoro-4,4-dimethyl-2-thioxo-1,4-dihydro-2H-3,1-benzoxazin-6-yl)benzonitrile, 3-(1,2-Dihydro-2-thioxospiro[4H-3,1-benzoxazine-4,1-cyclohexan]-6-yl)benzonitrile, 5-(1,2-Dihydro-2-thioxospiro[4H-3,1-benzoxazine-4,1 -cyclohexan]-6-yl)-4-methyl-2-thiophenecarbonitrile, 5-(1,2-Dihydro-2-thioxospiro[4H-3,1-benzoxazine-4,1-cyclohexan]-6-yl)-2-thiophenecarbonitrile, 6-(3-Chloro-4-fluorophenyl)-4,4-dimethyl-1,4-dihydro-2H-3,1-benzoxazine-2-thione, 5-(4,4-Dimethyl-2-thioxo-1,4-dihydro-2H-3,1-benzoxazin-6-yl)-4-propylthiophene-2-carbonitrile, 4-(4,4-Dimethyl-2-thioxo-1,4-dihydro-2H-3,1-benzoxazin-6-yl)-2-furonitrile, 6-(3-Bromophenyl)-4,4-dimethyl-1,4-dihydro-2H-3,1-benzoxazine-2-thione, or 2-(4,4-Dimethyl-2-thioxo-1,4-dihydro-2H-3,1-benzoxazin-6-yl)thiophene-3-carbonitrile, or a pharmaceutically acceptable salt thereof.

7. A compound according to claim 1, which is 4-Butyl-5-(4,4-dimethyl-2-thioxo-1,4-dihydro-2H-3,1-benzoxazin-6-yl)thiophene-2-carbonitrile, tert-Butyl 2-cyano-5-(4,4-dimethyl-2-thioxo-1,4-dihydro- 2H-3,1-benzoxazin-6-yl)-1H-pyrrole-1-carboxylate, or a pharmaceutically acceptable salt thereof.

8. A pharmaceutical composition comprising a pharmaceutically effective amount of a compound of according to any of claims 1 to 7 and a pharmaceutically acceptable carrier or excipient.

9. Use of a compound according to any of claims 1 to 7, or said acceptable salt thereof, in preparing a medicament.

10. Use of a compound according to any of claims 1 to 7, or said acceptable salt, in preparing a medicament useful for administration to a mammalian subject as a contraceptive.

11. Use of a compound according to any of claims 1 to7, or said acceptable salt, in preparing a medicament useful for administration to mammalian subject for treating carcinomas or adenocarcinomas of the endometrium ovary, breast, colon, or prostate.

12. Use of a compound according to any of claims 1 to 7, or said acceptable salt, in preparing a medicament useful for administration to mammalian subject for treating dysfunctional bleeding, uterine leiomyomata, endometriosis, polycystic ovary syndrome, or fibroids:

13. Use of a compound according to any of claims 1 to 7, or said acceptable salt, in preparing a medicament useful for administration to mammalian subject for hormone replacement therapy.

## Patentansprüche

1. Verbindung ausgewählt aus der Gruppe bestehend aus (I), (II), (III), (IV) und (V):
(I) eine Verbindung der Formel 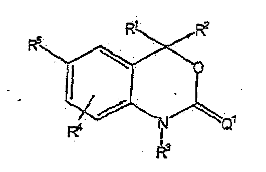 wobei:
R¹ und R² unabhängig voneinander Substituenten sind, ausgewählt aus der Gruppe bestehend aus H, C₁ bis C₆ Alkyl, substituiertem C₁ bis C₆ Alkyl, C₂ bis C₆ Alkenyl, substituiertem C₂ bis C₆ Alkenyl, C₂ bis C₆ Alkinyl, substituiertem C₂ bis C₆ Alkinyl, C₃ bis C₈ Cycloalykl, substituiertem C₃ bis C₈ Cycloalkyl, Aryl, substituiertem Aryl, Heterozyklus, substituiertem Heterozyklus, COR^{A} und NR^{B}COR^{A}:
oder R¹ und R² sind miteinander verbunden, um einen Ring auszubilden, ausgewählt aus der Gruppe bestehend aus a), b) und c), jeder Ring ist gegebenenfalls substituiert mit ein bis drei Substituenten ausgewählt aus der Gruppe bestehend aus H und C₁ bis C₃ Alkyl:
a) ein auf Kohlenstoff basierender 3 bis 8 gliedriger, gesättigter spirocyclischer Ring;
b) ein auf Kohlenstoff basierender 3 bis 8 gliedriger spirocyclischer Ring, der in einem seinen Grundgerüst ein oder mehrere Kohlenstoff-Kohlenstoff Doppelpindungen aufweist; und
c) ein 3 bis 8 gliedriger spirocyclischer Ring, der in seinem Grundgerüst 1 bis 3 Heteroatome ausgewählt aus der Gruppe bestehend aus O, S und N enthält;
R^{A} ist ausgewählt der Gruppe bestehend aus H, C1 bis C3 Alkyl, substituiertes C₁ bis C₃ Alkyl, Aryl, substituiertes Aryl, C₁ bis C₃ Alkoxy, substituiertes C₁ bis C₃ Alkoxy, Amino, C₁ bis C₃ Aminoalkyl und substituiertes C₁ bis C₃ Aminoalkyl; R^{B} ist H, C₁ bis C₃ Alkyl oder substituiertes C₁ bis C₃ Alkyl;
R³ ist H, OH, NH₂, C₁ bis C₆ Alkyl, substituiertem C₁ bis C₆ Alkyl, C₃ bis C₆ Alkenyl, substituiertem C₃ bis C₆ Alkenyl, Alkinyl, substituiertem Alkinyl oder COR^{c} ;
R^{c} ist ausgewählt aus der Gruppe bestehend aus H, C₁ bis C₃ Alkyl, substituiertem C₁ bis C₃ Alkyl, Aryl, substituiertem Aryl, C₁ bis C₃ Alkoxy, substituiertem C₁ bis C₃ Alkoxy, C₁ bis C₃ Aminoalkyl und substituiertem C₁ bis C₃ Aminoalkyl ;
R⁴ ist ausgewählt aus der Gruppe bestehend aus H, Halogen, CN, NO₂, C₁ bis C₆ Alkyl, substituiertem C₁ bis C₅ Alkyl, C₁ bis C₆ Alkoxy, substituiertem C₁ bis C₆ Alkoxy, C₁ bis C₆ Aminoalkyl, und substituiertem C₁ bis C₆ Aminoalkyl ;
R⁵ ist ausgewählt aus der Gruppe bestehend aus (i) und (ii) :
(i) ein substituierter Benzolring enthaltend die Substituenten X₁ Y und Z, wie unten dargestellt: 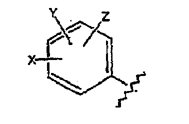 X ist ausgewählt aus der Gruppe bestehend aus H, Halogen, CN, C₁ bis C₃ Alkyl, substituiertem C₁ bis C₃ Alkyl, C₁ bis C₃ Alkoxy, substituiertem C₁ bis C₃ Alkoxy, C₁ bis C₃ Thioalkoxy, substituiertem C₁ bis C₃ Thioalkoxy, C₁ bis C₃ Aminoalkyl, substituiertem C₁ bis C₃ Aminoalkyl, NO₂, C₁ bis C₃ Perfluoralkyl, 5- oder 6-gfiedriger Heterozyklus, der in seinem Grundgerüst 1 bis 3 Heteroatome enthält, COR^{D}, OCOR^{D}, und NR^{E}COR^{D};
R^{D} ist H, C₁ bis C₃ Alkyl, substituiertes C₁ bis C₃ Alkyl, Aryl, substituiertes Aryl, C₁ bis C₃ Alkoxy, substituiertes C₁ bis C₃ Alkoxy, C₁ bis C₃ Aminoalkyl oder substituiertes C₁ bis C₃ Aminoalkyl;
R^{E} ist H, C₁ bis C₃ Alkyl oder substituiertes C₁ bis C₃ Alkyl;
Y und Z sind unabhängige Substituenten ausgewählt aus der Gruppe bestehend aus H, Halogen, CN, NO₂, C₁ bis C₃ Alkoxy, C₁ bis C₃ Alkyl und C₁ bis C₃ Thioalkoxy; wobei X, Y, Z nicht alle H sind, und
(ii) ein 5- oder 6-gliedriger heterozyklischer Ring, der in seinem Grundgerüst 1, 2 oder 3 Ringheteroatome aufweist, ausgewählt aus der Gruppe bestehend aus O, S, S(O), S(O₂) und NR⁶ und 1 oder 2 unabhängige Substituenten enthält, ausgewählt aus der Gruppe bestehend aus H, Halogen, CN, NO₂, C₁ bis C₃ Alkyl, C₁ bis C₃ Alkoxy, C₁ bis C₃ Aminoalkyl, COR^{F} und NR^{G}COR^{F};
R^{F} ist H, C₁ bis C₃ Alkyl, substituiertes C₁ bis C₃ Alkyl, Aryl, substituiertes Aryl, C₁ bis C₃ Alkoxy, substituiertes C₁ bis C₃ Alkoxy, C₁ bis C₃ Aminoalkyl oder substituiertes C₁ bis C₃ Aminoalkyl;
R^{G} ist H, C₁ bis C₃ Alkyl oder substituiertes C₁ bis C₃ Alkyl;
R⁶ ist H oder C₁ bis C₃ Alkyl;
Q¹ ist S, NR⁷ oder CR⁸R⁹;
R⁷ ist ausgewählt aus der Gruppe bestehend aus CN, C₁ bis C₆ Alkyl, substituiertem C₁ bis C₆ Alkyl, C₃ bis C₈ Cycloalkyl, substituiertem C₃ bis C₆ Cycloalkyl, Aryl, substituiertem Aryl, Heterozyklus, substituiertem Heterozyklus, SO₂CF₃, OR¹¹ und NR¹¹R¹²,
R⁸ und R⁹ sind unabhängig voneinander Substituenten ausgewählt aus der Gruppe bestehend aus H, C₁ bis C₆ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus, substituiertem Heterozyklus, NO₂, CN, und CO₂R¹⁰; R¹⁰ ist C₁ bis C₃ Alkyl;
oder CR⁸R⁹ ist ein 6-gliedriger Ring, mit der unten gezeigten Struktur: 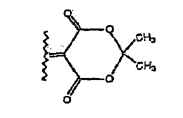 R¹¹ und R¹² sind unabhängig ausgewählt aus der Gruppe bestehend aus H, C₁ bis C₆ Alkyl, substituiertem C₁ bis C₆ Alkyl, Aryl, substituiertem Aryl, Heteroaryl, substituiertem Heteroaryl, Acyl und Sufonyl;
(II) eine Verbindung der Formel: 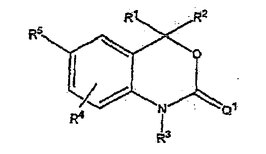 wobei R¹ ist H, C₁ bis C₆ Alkyl, substituiertes C₁ bis C₆ Alkyl, C₃ bis C₈ Cycloalkyl, substituiertes C₃ bis C₈ Cycloalkyl, Aryl, substituiertes Aryl, Heterozyklus, substituierter Heterozyklus, COR^{A}, oder NR^{B}COR^{A};
R² ist H, C₁ bis C₆ Alkyl, substituiertes C₁ bis C₆ Alkyl, C₂ bis C₆ Alkenyl, substituiertes C₂ bis C₆ Alkenyl, C₃ bis C₈ Cycloalkyl, substituiertes C₃ bis C₈ Cycloalkyl, Aryl, substituiertes Aryl, Heterozyklus, substituierter Heterozyklus, COR^{A} oder Nr^{B}COR^{A};
oder R¹ und R² bilden zusammen einen spirocyclischen Ring ausgewählt aus der Gruppe bestehend aus a), b) und c), jeder Ring ist gegebenenfalls substituiert mit 1 bis 3 Substiuenten ausgewählt aus der Gruppe bestehend aus H und C₁ bis C₃ Alkyl:
a) ein auf Kohlenstoff basierender 3- bis 8-gliedriger, gesättigter spirocyclischer Ring;
b) ein auf Kohlenstoff basierender 3- bis 8-gliedriger spirocyclischer Ring der in seinem Grundgerüst ein oder mehrere Kohlenstoff-KohlenstoffDoppelbindungen aufweist; und
c) einen 3- bis 8-gliedrigen spirocyclischer Ring, der in seinem Grundgerüst 1 bis 3 Heteroatome ausgewählt aus der Gruppe bestehend O, S und N aufweist;
R^{A} ist H, C₁ bis C₃ Alkyl, substituiertes C₁ bis C₃ Alkyl, Aryl, substituiertes Aryl, C₁ bis C₃ Alkoxy, substituiertes C₁ bis C₃ Alkoxy, C₁ bis C₃ Aminoalkyl, oder substituiertes C₁ bis C₃ Aminoalkyl;
R^{B} ist H, C₁ bis C₃ Alkyl, oder substituiertes C₁ bis C₃ Alkyl;
R³ ist H, OH, NH₂, C₁ bis C₆ Alkyl, substituiertes C₁ bis C₆ Alkyl, C₃ bis C₈ Alkenyl, substituiertes C₃ bis C₆ Alkenyl, Alkinyl, substituiertes Alkinyl oder COR^{c};
R^{c} ist H, C₁ bis C₄ Alkyl, substituiertes C₁ bis C₄ Alkyl, Aryl, substituiertes Aryl, C₁ bis C₄ Alkoxy, substituiertes C₁ bis C₄ Alkoxy, C₁ bis C₄ Aminoalkyl, oder substituiertes C₁ bis C₄ Aminoalkyl;
R⁴ ist H, Halogen, CN, NO₂, C₁ bis C₆ Alkyl, substituiertes C₁ bis C₆ Alkyl, C₁ bis C₆ Alkoxy, substituiertes C₁ bis C₆ Alkoxy, C₁ bis C₆ Aminoalkyl, oder substituiertes C₁ bis C₆ Aminoalkyl;
R⁵ is (i) oder (ii):
(i) ein substituierter Benzolring enthaltend die Substituenten X, Y und Z, wie unten dargestellt: 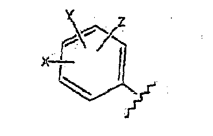 X ist ausgewählt aus der Gruppe bestehend aus H, Halogen, CM, C₁ bis C₃ Alkyl, substituiertem C₁ bis C₃ Alkyl, C₁ bis C₃ Alkoxy, substituiertem C₁ bis C₃ Alkoxy, C₁ bis C₃ Thioalkoxy, substituiertem C₁ bis C₃ Thioalkoxy, C₁ bis C₃ Aminoalkyl, substituiertem C₁ bis C₃ Aminoalkyl, NO₂, C₁ bis C₃ Perfluoralkyl, 5-gliedrigen Heterozyklusring in seinem Grundgerüst enthaltend 1 bis 3 Heteroatome, COR^{D}, OCOR^{D} und NR^{E}COR^{D};
R^{D} ist H, C₁ bis C₃ Alkyl, substituiertes C₁ bis C₃ Alkyl, Aryl, substituiertes Aryl, C₁ bis C₃ Alkoxy, substituiertes C₁ bis C₃ Alkoxy, C₁ bis C₃ Aminoalkyl, oder substituiertes C₁ bis C₃ Aminoalkyl;
R^{E} ist H, C₁ bis C₃ Alkyl, oder substituiertes C₁ bis C₃ Alkyl;
Y und Z sind unabhängig voneinander Substituenten ausgewählt aus der Gruppe bestehend aus H, Halogen, CN, NO₂, C₁ bis C₃ Alkoxy, C₁ bis C₃ Alkyl, und C₁ bis C₃ Thioalkoxy;
wobei X, Y und Z nicht alle H sind, oder
(ii) ein 5- oder 6-gliedriger Ring, der in seiner Grundstruktur 1, 2 oder 3 Heteroatome aufweist, ausgewählt aus der Gruppe bestehend aus O, S, SO, SO₂ und NR⁶ und der 1 oder 2 unabhängige Substituenten enthält, ausgewählt aus der Gruppe bestehend aus H, Halogen, CN, NO₂, C₁ bis C₃ Alkyl, und C₁ bis C₃ Alkoxy;
R⁵ ist H oder C₁ bis C₃ Alkyl;
Q¹ ist S, NR⁷ oder CR⁸R⁹;
R⁷ ist ausgewählt aus der Gruppe bestehend aus CN, C₁ bis C₆ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus; substituiertem Heterozyklus und SO₂CF₃;
R⁸ und R⁹ sind unabhängig voneinander Substituenten ausgewählt aus der Gruppe bestehend aus H, C₁ bis C₆ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus; substituiertem Heterozyklus, NO₂, CN und CO₂R¹⁰;
R¹⁰ ist C₁ bis C₃ Alkyl;
oder CR⁸R⁹ ist ein 6-gliedriger Ring, wie er durch die unten aufgeführte Struktur dargestellt ist: 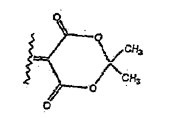
(III) Eine Verbindung der Formel: 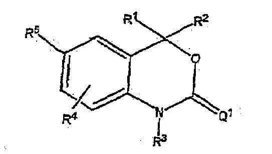 wobei R¹ und R² unabhängig voneinander ausgewählt sind aus der Gruppe bestehend aus C₁ bis C₃ Alkyl und substituiertem C₁ bis C₃ Alkyl oder R¹ und R² bilden zusammen einen drei- bis sechsgliedrigen gesättigten spirocyclischen Ring auf Kohlenstoffbasis;
R³ ist H, OH, NH₂, C₁ bis C₆Alkyl, substituiertes C₁ bis C₆ Alkyl oder COR^{C};
R^{c} ist H, C₁ bis C₄ Alkyl oder C₁ bis C₃ Alkoxy;
R⁴ ist H, Halogen, NO₂, C₁ bis C₃ Alkyl oder substituiertes C₁ bis C₃ Alkyl,
R⁵ ist (i) oder (ii):
(i) ein substituierter Benzolring, der Struktur: 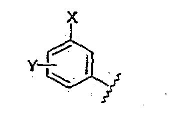 wobei
X ausgewählt ist aus der Gruppe bestehend aus Halogen, CN, C₁ bis C₃ Alkoxy, C₁ bis C₃ Alkyl, NO₂, C₁ bis C₃ Perfluoralkyl, 5-gliedriger Heterozyklusring, der in seinem Grundgerüst 1 bis 2 Heteroatome enthält, und C₁ bis C₃ Thioalkoxy;
Y ist ausgewählt aus der Gruppe bestehend aus H, Halogen, CN, NO₂, C₁ bis C₃ Alkoxy, C₁ bis C₄ Alkyl und C₁ bis C₃ Thioalkoxy oder
(ii) ein 5-gliedriger Ring der Struktur 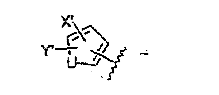 wobei
X' ausgewählt ist der Gruppe bestehend aus Halogen, CN, C₁ bis C₃ Alkoxy, C₁ bis C₃ Alkyl und NO₂;
Y' ist ausgewählt aus der Gruppe bestehend aus H, Halogen, CN, NO₂, C₁ bis C₃ Alkoxy, C₁ bis C₄ Alkyl und C₁ bis C₃ Thioalkoxy;
U ist O, S, oder NR⁶;
R⁶ ist H, C₁ bis C₃ Alkyl oder C₁ bis C₄ CO₂ Alkyl;
Q¹ ist S, NR⁷ oder CR⁸R⁹;
R⁷ ist ausgewählt aus der Gruppe bestehend aus CN, C₁ bis C₆ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus, substituiertem Heterozyklus und SO₂CF3;
R⁸ und R⁹ sind unabhängig voneinander Substituenten ausgewählt aus der Gruppe bestehend aus H, C₁ bis C₆ Alkyl, substituiertem C₁ bis C₆ Alkyl, C₃ bis C₈ Cykloalkyl, substituiertem C₃ bis C₈ Cykloalkyl, Aryl, substituiertem Aryl, Heterozyklus, substituiertem Heterozyklus, NO₂, CN und CO₂R¹⁰;
R¹⁰ ist C₁ bis C₃ Alkyl;
oder CR⁸R⁹ ist ein 6-gliedriger Ring mit der unten dargestellten Struktur 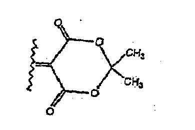
(IV) Eine Verbindung der Formel 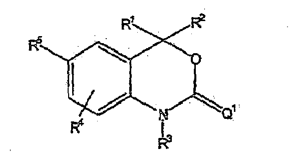 wobei
R¹ und R² unabhängig voneinander ausgewählt sind aus der Gruppe bestehend aus C₁ bis C₃ Alkyl und substituiertem C₁ bis C₃ Alkyl oder R¹ und R² bilden zusammen einen 3- bis 6-gliedrigen, gesättigten spirocyclischen Ring auf Kohlenstoffbasis ;
R³ ist H;
R⁴ ist H; Halogen, NO₂, C₁ bis C₃ Alkyl oder substituiertes C₁ bis C₃ Alkyl;
R⁵ ist ein 6-gliedriger Ring der Struktur : 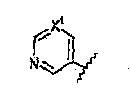 wobei
X¹ ist N; und
Q¹ ist S, NR⁷ oder CR⁸R⁹, und
unter der Voraussetzung, dass für jede der Verbindungen (I) bis (IV) gilt, dass wenn Q¹ ist S und R³ ist H, sowohl R¹ als auch R² nicht H sind;
unter der Voraussetzung, dass für jede der Verbindungen (I) bis (IV) gilt, dass wenn Q¹ ist S; R³ H ist; R⁵ ist ein Benzolring (i) substituiert mit ein oder zwei Substituenten oder R⁵ ist der 5- oder 6-gliedriger Ring (ii) mit 1, 2 oder 3 Heteroatomen ausgewählt aus der Gruppe O, S und NR⁶ in seinem Grundgerüst und einer von R¹ oder R² ist C₂ bis C₄ Alkenyl, substituiertes C₂ bis C₄ Alkenyl, C₂ bis C₄ Alkinyl oder substituiertes C₂ bis C₄ Alkinyl, der andere von R² oder R¹ nicht C₁ bis C₆ Alkyl, substituiertes C₁ bis C₆ Alkyl, C₃ bis C₅ Cycloalkyl, substituiertes C₃ bis C₅ Cycloalkyl, C₂ bis C₄ Alkenyl, substituiertes C₂ bis C₄ Alkenyl, C₂ bis C₄ Alkinyl oder substituiertes C₂ bis C₄ Alkinyl ist;
unter der Voraussetzung für jede der Verbindungen (I) bis (IV), dass wenn Q¹ ist S, R³ ist H; R⁵ ein Benzolring (i) substituiert mit ein oder zwei Substituenten oder R⁵ ist der 5- oder 6-gliedrige Ring (ii), der in seinem Grundgerüst 1, 2 oder 3 Heteroatome ausgewählt aus der Gruppe bestehend aus O, S und NR⁶ aufweist, und einer von R¹ oder R² C₁ bis C₆ Alkyl oder substituiertes C₁ bis C₆ Alkyl ist, der andere von R² oder R¹ nicht C₃ bis C₈ Cycloalkyl oder substituiertes C₃ bis C₈ Cycloalkyl ist;
unter der Voraussetzung, dass für jede der Verbindungen (I) bis (IV) der Ausdruck "substituiertes Alkyl" nicht Perfluoralkyl einschließt;
(V) 6-[3-Fluor-5-(trifluormethyl)phenyl]-4,4-dimethyl-1,4-dihydro-2H-3,1-benzoxazin-2-thion;
(VI) 6-(3-Brom-5-trifluormethoxyphenyl)-4,4-dimethyl-1,4-dihydro-2H-3,1-benzoxazin-2-thion; oder einem pharmazeutisch annehmbaren Salz davon.

2. Eine Verbindung gemäß Anspruch 1, wobei R¹ ist H, C₁ bis C₆Alkyl, substituiertes C₁ bis C₆ Alkyl, C₃ bis C₈ Cycloalkyl, substituiertes C₃ bis C₈ Cycloakyl, Aryl, substituiertes Aryl, Heterozyklus, substituierter Heterozyklus, COR^{A} oder NR^{B} COR^{A};
oder R1 und R2 bilden einen Ring ausgewählt aus der Gruppe bestehend aus a), b) und c):
a) ein auf Kohlenstoff basierender drei- bis sechsgliedriger gesättigter spirocyclischer Ring;
b) ein auf Kohlenstoff basierender drei- bis sechsgliedriger spirocyclischer Ring der in seiner Grundstruktur ein oder mehrere Kohfenstoff-Kohfenstoffverbindungen aufweist; und
c) ein 3- bis 6-gliedriger spirocyclischer Ring enthaltend in seiner Grundstruktur ein bis drei Heteroatome;
R^{A} ist H, C₁ bis C₃ Alkyl, substituiertes C₁ bis C₃ Alkyl, Aryl, substituiertes Aryl, C₁ bis C₃ Alkoxy, substituiertes C₁ bis C₃ Alkoxy, C₁ bis C₃ Aminoalkyl oder substituiertes C₁ bis C₃ Aminoalkyl;
R⁵ ist ein 5- oder 6-gliedriger Ring, wobei die 1 oder 2 unabhängig voneinander vorliegenden Substituenten ausgewählt sind aus der Gruppe bestehend aus H, Halogen, CN, NO₂, C₁ bis C₃ Alkyl und C₁ bis C₃ Alkoxy;
R⁷ ist ausgewählt aus der Gruppe bestehend aus CN, C₁ bis C₆ Alkyl, substituiertem C₁ bis C₆ Alkyl, C₃ bis C₈ Cykloalkyl, substituiertem C₃ bis C₈ Cycloalkyl, Aryl, substituiertem Aryl, Heterozyklus, substituiertem Heterozyklus und SO₂CF₃

3. Eine Verbindung gemäß Anspruch 1; wobei:
R¹ und R² unabhängig voneinander ausgewählt sind aus der Gruppe bestehend aus C₁ bis C₃ Alkyl und substituiertem C₁ bis C₃ Alkyl oder R¹ und R² bilden zusammen einen auf Kohlenstoff basierenden 3- bis 6-gliedrigen gesättigten spirocyclischer Ring:
R³ ist H, OH, NH₂, C₁ bis C₅ Alkyl, substituiertes C₁ bis C₆ Alkyl oder COR^{c};
R^{c} ist H, C₁ bis C₃ Alkyl oder C₁ bis C₃ Alkoxy;
R₄ ist H, halogen, NO₂, C₁ bis C₃ Alkyl oder substituiertes C₁ bis C₃ Alkyl;
R⁵ ist ein substituierter Benzolring der Struktur: 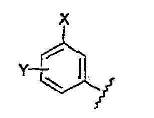
X ist ausgewählt aus der Gruppe bestehend aus Halogen, CN, C₁ bis C₃ Alkoxy, C₁ bis C₃ Alkyl, NO₂, C₁ bis C₃ Perfluoralkyl, fünfgliedrigem Heterozyklusring enthaltend in seiner Grundstruktur 1 bis 3 Heteroatome, und C₁ bis C₃ Thioalkoxy;
Y ist ausgewählt aus der Gruppe bestehend aus H, Halogen, CN, NO₂, C₁ bis C₃ Alkoxy, C₁ bis C₄ Alkyl und C₁ bis C₃ Thioalkoxy;
R⁷ ist ausgewählt aus der Gruppe bestehend aus CN, C₁ bis C⁶ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus, substituiertem Heterozyklus und SO₂CF₃.

4. Eine Verbindungen gemäß Anspruch 1, wobei:
R¹ und R² unabhängig ausgewählt sind aus der Gruppe bestehend aus C₁ bis C₃ Alkyl und substituiertem C₁ bis C₃ Alkyl, oder R¹ und R² bilden zusammen einen auf Kohlenstoff basierenden 3- -bis 6-gliedrigen gesättigten spirocyclischem Ring;
R³ ist H;
R^{c} ist H, C₁ bis C₃ Alkyl oder C₁ bis C₃ Alkoxy;
R⁴ ist H, Halogen, NO₂, C₁ bis C₃ Alkyl, substituiertes C₁ bis C₃ Alkyl;
R⁵ ist ein 5-gliedriger Ring der Struktur: 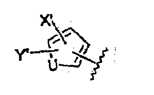
wobei:
X' ist ausgewählt aus der Gruppe bestehend aus Halogen, CN, C₁ bis C₃ Alkoxy, C₁ bis C₃ Alkyl, NO₂, C₁ bis C₃ Perfluoralkyl, fünfgliedrigem Heterozyklusring, der in seinem Grundgerüst 1 bis 3 Heteroatome enthält, und C₁ bis C₃ Thioalkoxy;
Y' ist ausgewählt aus der Gruppe bestehend aus H, Halogen, CN, NO₂, C₁ bis C₃ Alkoxy, C₁ bis C₃ Alkyl und C₁ bis C₃Thioalkoxy;
U ist O, S oder NR⁶;
R⁷ ist ausgewählt aus der Gruppe bestehend aus CN, C₁ bis C₆ Alkyl, substituiertem C₁ bis C₆ Alkyl, C₃ bis C₈ Cycloalkyl, substituiertem C₃ bis C₈ Cycloalkyl, Aryl, substituiertem Aryl, Heterozyklus, substituiertem Heterozyklus und SO₂CF₃.

5. Eine Verbindung gemäß Anspruch 1, wobei:
R¹ und R² unabhängig voneinander ausgewählt sind aus der Gruppe bestehend aus C₁ bis C₃ Alkyl und substituiertem C₁ bis C₃ Alkyl oder R¹ und R² bilden zusammen einen auf Kohlenstoff basierenden 3- bis 6-gliedrigen spirocyclischem Ring:
R³ ist H;
R⁴ ist H, Halogen, NO₂, C₁ bis C₃ Alkyl oder substituiertes C₁ bis C₃ Alkyl;
R⁵ ist ein 6-gliedriger Ring der Struktur: 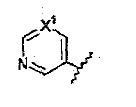
wobei:
X¹ CX² ist;
X² ist Halogen, CN oder NO₂.

6. Eine Verbindung gemäß Anspruch 1:
6-(3-Chlorophenyl)-4,4- dimethyl-1,4-dihydro-benzo[d] [1,3]oxazin-2-thion,4-(4,4-Dimethyl-2-thioxo-1,4-dihydro-2H-benzo[d][1,3]oxazin-6-yl)-thiophen-2-carbonitril, 3-(4,4-Dimethyl-2-thioxo-1,4-dihydro-2H-benzo[d][1,3]oxazin-6-yl)-5-fluorobenzonitril, 3-(4,4-Dimethyl-2-thioxo-1,4-dihydro 2H-benzo [d][1,3] oxazin-6-yl)-benzonitril, 5-(4,4-Dimethyl-2-thioxo-1,4-dihydro-2H-3,1,1-benzoxazin-6-yl)-1-methyl-1H-pyrrol-2-carbonitril, 6-(3-Fluorophenyl)-4-methyl-9,4-dihydro-2H-3,1-benzoxazin-2-thion, 5-(4,4-Dimethyl,2-thioxo, 1,4-dihydro-2H-3,1-benzoxazin-6-yl)-4-methylthiophen-2-carbonitril, 5-(4,4-Dimethyl-2 thioxo-1,4-dihydro-2H-3,1-benzoxazin-6-yl)-1H pyrrol-2-carbonitril, [6-(4,4-Dimethyl-2-thioxo-1,4-dihydro-2H-3,1-benzoxazin-6-yl) pyridin-2-yl] acetonitril, 5-(4,4-Dimethyl-2-thioxo-1,4-dihydro-2H-3,1-benzoxazin-6-yl)-1H-pyrrol-2-carbothiamid, 5-(4,4-Dimethyl,2-thioxo-1,4-dihydro-2H-benzo [d] [1,3] oxazin-6-yl) thiophen-3-carbonitril,5-(4,4-Dimethyl, 2-thioxo-1,4-dihydro-2H-3,1-benzoxazin-6-yl)-1-ethyl-1H-pirol-2-carbonitril, 4-(1,2-Dihydro-2-thioxospiro [4H-3,1-benzoxazin-4,1-cyclohexan]-6-yl)-2-thiophenecarbonitril, 5-(4,4-Dimethyl-2-thioxo-1,4-dihydro-2H-3,1-benzoxazin-6-yl)-2-fluorobenzonitril, 6-(5-Bromopyridin-3-yl)-4,4-dimethyl-1,4-dihydro-2H-3,1-benzoxazin-2-thion, 6-(3-Chloro-5-fluorophenyl)-4,4 dimethyl-1,4-dihydro-2H-3,1-benzoxazin-2-thion, 6-(3-Bromo-5-methylphenyl)-4,4-dimethyl-1,4-dihydro-2H-3,1-benzoxazin-2-thion -3-(1,2-Dihydro-2-thioxospiro[4H-3,1-benzoxazin-4,1-cyclohexan]-6-yl)-5-fluorobenzonitril, 3-(4,4-Dimethyl-2-thioxo-1,4-dihydro-2H-3,1-benzoxazin-6-yl)-5-methylbenzonitril, 6-(3,5-Dichlorophenyl)-4,4-dimethyl-1,4-dihydro-2H-3,1-benzoxazin-2-thion, 5-(4,4-Dimethyl-1,2-thioxo-1,4-dihydro-2H-3,1-benzoxazin-6-yl)isophthalonitril, 5(4,4-Dimethyl-2-thioxo-1,4-dihydro-2H-3,1-benzoxazin-6-yl)-2-furonitril, 4,4-Diethyl-6-(3-Nitrophenyl)-1,4-dihydro-2H-3,1-benzoxazin-2-thion, 6-(3-Chlorophenyl)-4-methyl-4-phenyl-1,4-dihydro-2H-3,1-benzoxazin-2-thion, 4-Allyl-6-(3-chlorophenyl)-4-methyl-1,4-dihydro-2H-3, 1-benzoxazin-2-thion, 3-Chloro-5-(4,4-Dimethyl-2-thioxo-1,4-dihydro-2H-3,1-benzoxazin-6-yl) benzonitril, 6-(3,5-Difluorophenyl)-4,4-dimethyl-1,4-dihydro-2H-3,1-benzoxazin-2-thion, 6-(3-Fluoro-5-methoxyphenyl)-4,4-dimethyl-1,4-dihydro-2H-3,1-benzoxazin-2-thion, 3-(4,4-Dimethyl-2-thioxo-1,4-dihydro-2H-3,1-benzoxazin-6-yl)-5-methoxybenzonitril, 6-(3-Fluorophenyl)-4,4-dimethyl-1,4-dihydro-2H-3,1-benzoxazin-2-thion, 6-(2-Fluorophenyl)-4,4-dimethyl-1,4-dihydro-2H-3,1-benzoxazin -2-thion, 6-(3,4-Difluorophenyl)-4,4-dimethyl-1,4-dihydro-2H-3,1-benzoxazin-2-thion, 6-(4-Fluorophenyl)-4,4-dimethyl-1,4-dihydro-2H-3,1-benzoxazin-2-thion, 3-(4,4-Dimethyl-2-thioxo-1,4-dihydro-2H-3,1-benzoxazin-6-yl)-4-fluorobenzonitril, 6-(2,3-Difluorophenyl)-4,4-dimethyl-1,4-dihydro-2H-3,1-benzoxazin-2-thion, 3-(8-Bromo-4,4-dimethyl-2-thioxo-1,4-dihydro-2H-3,1-benzoxazin-6-yl)-5-fluorobenzonitril, 4,4-Dimethyl-6-(3-nitrophenyl)-1,4-dihydro-2H-3, 1-Benzoxazin-2-thion, 6-(3-chlorophenyl)-4,4-diethyl-1,4-dihydro-2H-3,1-benzoxazin-2-thion, 6-(3-Methoxyphenyl)-4,4-dimethyl-1,4-dihydro-2H-3,1-benzoxazin-2-thion, 6-(2-Chlorophenyl)-4,4-dimethyl-1,4-dihydro-2H-3,1-benzoxazin-2-thion, 4-Benzyl-6-(3-Chlorophenyl)-4-methyl-1,4-dihydro-2H-3,1-benzoxazin-2-thion, 6-(3-Bromo-5-fluorophenyl)-4,4-dimethyl-1,4-dihydro-2H-3,1-benzoxazin-2-thion, 5-(4,4-Dimethyl-2-thioxo-1,4-dihydro-2H-3,1-benzoxazin-6-yl) thiophen-2-carbonitril, 3-Fluoro-5-(8-Fluoro-4,4-dimethyl-2-thioxo-1,4-dihydro-2H-3,1-benzoxazin-6-yl) benzonitril, 3-(1,2-Dihydro-2-thioxospiro[4H-3,1-benzoxazin-4,1-cyclohexan]-6-yl) benzonitril, 5-(1,2-Dihydro-2-thioxospiro[4H-3,1-benzoxazin-4,1-cyclohexan]-6-yl)-4-methyl-2-thiophencarbonitril, 5-(1,2-Dihydro-2-fhioxospiro[4H-3,1-benzoxazin-4,1-cyclohexan]-6-yl)-2-thiophenecarbonitril, 6-(3-Chloro-4-fluorophenyl)-4,4-dimefhyl-1,4-dihydrö-2H-3.1 -benzoxazin-2-thion, 5-(4,4-Dimethyl-2-thioxo-1,4-dihydro-2H-3, 1 -benzoxazin-6-yl)-4-propylthiophen-2-carbonitril, 4-(4,4-Dimethyl-2-thioxo-1,4-dihydro-2H-3,1-benzoxazin-6-yl)-2-furonitril, 6-(3-Bromophenyl)-4,4-dimethyl-1,4-dihydro-2H-3,1-benzoxazin-2-thion oder 2-(4,4-Dimethyl-2-thioxo-1,4-dihydro-2H-3,1-benzaxazin-5-yl)thiophen-3-carbonitril oder ein pharmazeutisch annehmbares Salz davon.

7. Eine Verbindung gemäß Anspruch 1, die 4-Butyl-5-(4,4-Dimethyl-2-thioxo-1,4 dihydro-2H-3,1-benzoxazin-6-yl)thiphen-2-carbonitril, tert-Butyl- 2-cyano-5-(4,4-dimethyl-2-thioxo-1,4-dihydro-2H-3,1-benzoxazin-6-yl)-1H-pyrrol-1-carboxylat oder ein pharmazeutisch annehmbares Salz davon ist.

8. Eine pharmazeutische Zusammensetzung umfassend eine pharmazeutisch wirksame Menge einer Verbindung gemäß einem der Ansprüche 1 bis 7 und einem pharmazeutisch annehmbaren Träger oder Excipienten.

9. Verwendung einer Verbindung gemäß einem der Ansprüche 1 bis 7 oder dessen annehmbaren Salzes davon zur Herstellung eines Medikamentes.

10. Verwendung einer Verbindung gemäß einem der Ansprüche 1 bis 7 oder dessen annehmbaren Salzes zur Herstellung eines Medikamentes nützlich zur Verabreichung an einem Säugetier als Verhütungsmittel.

11. Verwendung einer Verbindung gemäß einem der Ansprüche 1 bis 7 oder dessen annehmbaren Salzes zur Herstellung eines Medikamentes nützlich zur Verabreichung bei einem Säugetier zur Behandlung oder Karzinomen oder Adenokarzinomen des Endometriums, Eierstocks, Brust, Kolon oder Prostata.

12. Verwendung einer Verbindung gemäß einem der Ansprüche 1 bis 7 oder dessen annehmbaren Salzes zur Herstellung eines Medikamentes nützlich zur Verabreichung bei einem Säugetier zur Behandlung von funktionsgestörten Blutungen, Uterusleiomyom, Endometriose, Syndrom der polyrystischen Ovarien oder Fibrombildungen.

13. Verwendung einer Verbindung gemäß einem der Ansprüche 1 bis 7 oder dessen annehmbaren Salzes zur Herstellung eines Medikamentes nützlich zur Verabreichung bei einem Säugetier zur Hormonersatztherapie.

## Revendications

1. Composé sélectionné dans le groupe consistant en (I), (II), (III), (IV) et (V) :
(I) un composé de formule : 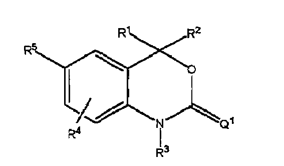 dans laquelle
R¹ et R² sont des substituants indépendants sélectionnés dans le groupe consistant en H, un groupe alkyle en C₁-C₆, un groupe alkyle en C₁-C₆ substitué, un groupe alcényle en C₂-C₆, un groupe alcényle en C₂-C₆ substitué, un groupe alcynyle en C₂-C₆, un groupe alcynyle en C₂-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, COR^{A} et NR^{B}COR^{A} ;
ou R¹ et R² sont fusionnés pour former un cycle sélectionné dans le groupe consistant en a), b) et c), chaque cycle étant éventuellement substitué par 1 à 3 substituants sélectionnés dans le groupe consistant en H et un groupe alkyle en C₁-C₃ :
a) un cycle spirocyclique saturé de 3 à 8 éléments à base d'atome de carbone ;
b) un cycle spirocyclique de 3 à 8 éléments à base d'atome de carbone ayant dans son squelette une ou plusieurs doubles liaisons carbone-carbone ; et
c) un cycle spirocyclique de 3 à 8 éléments contenant dans son squelette un à trois hétéroatomes sélectionnés dans le groupe consistant en O, S et N ;
R^{A} est sélectionné dans le groupe consistant en H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe amino, un groupe aminoalkyle en C₁-C₃, et un groupe aminoalkyle en C₁-C₃ substitué ;
R^{B} est H, un groupe alkyle en C₁-C₃ ou un groupe alkyle en C₁-C₃ substitué ;
R³ est H, OH, NH₂, un groupe alkyle en C₁-C₆, un groupe alkyle en C₁-C₆ substitué, un groupe alcényle en C₃-C₆, un groupe alcényle en C₃-C₆ substitué, un groupe alcynyle, un groupe alcynyle substitué ou COR^{c};
R^{c} est sélectionné dans le groupe consistant en H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, et un groupe aminoalkyle en C₁-C₃ substitué ;
R⁴ est sélectionné dans le groupe consistant en H, un atome d'halogène, CN, NO₂, un groupe alkyle en C₁-C₆, un groupe alkyle en C₁-C₆ substitué, un groupe alcoxy en C₁-C₆, un groupe alcoxy en C₁-C₆ substitué, un groupe aminoalkyle en C₁-C₆, et un groupe aminoalkyle en C₁-C₆ substitué ;
R⁵ est sélectionné dans le groupe consistant en (i) et (ii) :
(i) un cycle benzène substitué contenant les substituants X, Y et Z comme illustré ci-dessous : 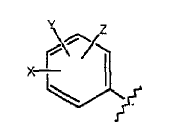 X est sélectionné dans le groupe consistant en H, un atome d'halogène, CN, un groupe alkyle en C₁-C₃, un groupe alkyle en C₁-C₃ substitué, un groupe alcoxy en C₁-C₃, un groupe alcoxy en C₁-C₃ substitué, un groupe thioalcoxy en C₁-C₃, un groupe thioalcoxy en C₁-C₃ substitué, un groupe aminoalkyle en C₁-C₃, un groupe aminoalkyle en C₁-C₃ substitué, NO₂, un groupe perfluoroalkyle en C₁-C₃, un cycle hétérocyclique de 5 ou 6 éléments contenant dans son squelette 1 à 3 hétéroatomes, COR^{D}, OCOR^{D}, et NR^{E}COR^{D};
R^{D} est H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, ou un groupe aminoalkyle en C₁-C₃ substitué ;
R^{E} est H, un groupe alkyle en C₁-C₃ ou un groupe alkyle en C₁-C₃ substitué;
Y et Z sont des substituants indépendants sélectionnés dans le groupe consistant en H, un atome d'halogène, CN, NO₂, un groupe alcoxy en C₁-C₃, un groupe alkyle en C₁-C₃, et un groupe thioalcoxy en C₁-C₃ ;
où pas tous les groupes X, Y et Z sont H ; et
(ii) un cycle hétérocyclique de 5 ou 6 éléments ayant dans son squelette 1, 2 ou 3 hétéroatomes sur le cycle sélectionnés dans le groupe consistant en O, S, S(O), S(O₂) et NR⁶ et contenant un ou deux substituants indépendants sélectionnés dans le groupe consistant en H, un atome d'halogène, CN, NO₂, un groupe alkyle en C₁-C₃, un groupe alcoxy en C₁-C₃, un groupe aminoalkyle en C₁-C₃, COR^{F} et NR^{G}COR^{F} ;
R^{F} est un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, ou un groupe aminoalkyle en C₁-C₃ substitué ;
R^{G} est H, un groupe alkyle en C₁-C₃ ou un groupe alkyle en C₁-C₃ substitué ;
R⁶ est H ou un groupe alkyle en C₁-C₃ ;
Q¹ est S, NR⁷ ou CR⁸R⁹;
R⁷ est sélectionné dans le groupe consistant en CN, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, SO₂CF₃, OR¹¹ et NR¹¹R¹² ;
R⁸ et R⁹ sont des substituants indépendants sélectionnés dans le groupe consistant en H, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, NO₂, CN et CO₂R¹⁰;
R¹⁰ est un groupe alkyle en C₁-C₃ ;
ou CR⁸R⁹ est un cycle de 6 éléments comme illustré par la structure ci-dessous : 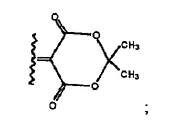 R¹¹ et R¹² sont indépendamment sélectionnés dans le groupe consistant en H, un groupe alkyle en C₁-C₆, un groupe alkyle en C₁-C₆ substitué, un groupe aryle, un groupe aryle substitué, un groupe hétéroaryle, un groupe hétéroaryle substitué, un groupe acyle et un groupe sulfonyle ;
(II) un composé de formule : 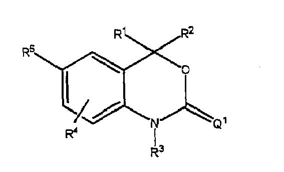 dans laquelle
R¹ est H, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, COR^{A} ou NR^{B}COR^{A} ;
R² est H, un groupe alkyle en C₁-C₆, un groupe alkyle en C₁-C₆ substitué, un groupe alcényle en C₂-C₆, un groupe alcényle en C₂-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, COR^{A} ou NR^{B}COR^{A} ;
ou R¹ et R² sont fusionnés pour former un cycle spirocyclique sélectionné dans le groupe consistant en a), b) et c), chaque cycle étant éventuellement substitué par 1 à 3 substituants sélectionnés dans le groupe consistant en H et un groupe alkyle en C₁-C₃ ;
a) un cycle spirocyclique saturé de 3 à 8 éléments à base d'atome de carbone ;
b) un cycle spirocyclique de 3 à 8 éléments à base d'atome de carbone ayant dans son squelette une ou plusieurs doubles liaisons carbone-carbone ; et
c) un cycle spirocyclique de 3 à 8 éléments contenant dans son squelette un à trois hétéroatomes sélectionnés dans le groupe consistant en O, S et N ;
R^{A} est H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, ou un groupe aminoalkyle en C₁-C₃ substitué ;
R^{B} est H, un groupe alkyle en C₁-C₃ ou un groupe alkyle en C₁-C₃ substitué ;
R³ est H, OH, NH₂, un groupe alkyle en C₁-C₆, un groupe alkyle en C₁-C₆ substitué, un groupe alcényle en C₃-C₆, un groupe alcényle en C₃-C₆ substitué, un groupe alcynyle, un groupe alcynyle substitué ou COR^{c} ;
R^{c} est H, un groupe alkyle en C₁-C₄, un groupe alkyle en C₁-C₄ substitué, un groupe aryle, un groupe aryle substitué, un groupe alcoxy en C₁-C₄, un groupe alcoxy en C₁-C₄ substitué, un groupe aminoalkyle en C₁-C₄, ou un groupe aminoalkyle en C₁-C₄ substitué ;
R⁴ est H, un atome d'halogène, CN, NO₂ un groupe alkyle en C₁-C₆, un groupe alkyle en C₁-C₆ substitué, un groupe alcoxy en C₁-C₆, un groupe alcoxy en C₁-C₆ substitué, un groupe aminoalkyle en C₁-C₆, ou un groupe aminoalkyle en C₁-C₆ substitué ;
R⁵ est (i) ou (ii) :
(i) un cycle benzène substitué contenant les substituants X, Y et Z comme illustré ci-dessous : 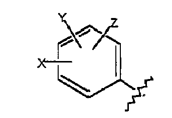 X est sélectionné dans le groupe consistant en H, un atome d'halogène, CN, un groupe alkyle en C₁-C₃, un groupe alkyle en C₁-C₃ substitué, un groupe alcoxy en C₁-C₃, un groupe alcoxy en C₁-C₃ substitué, un groupe thioalcoxy en C₁-C₃, un groupe thioalcoxy en C₁-C₃ substitué, un groupe aminoalkyle en C₁-C₃, un groupe aminoalkyle en C₁-C₃ substitué, NO₂*,* un groupe perfluoroalkyle en C₁-C₃, un cycle hétérocyclique de 5 éléments contenant dans son squelette 1 à 3 hétéroatomes, COR^{D}, OCOR^{D}, et NR^{E}COR^{D} ;
R^{D} est H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, ou un groupe aminoalkyle en C₁-C₃ substitué ;
R^{E} est H, un groupe alkyle en C₁-C₃ ou un groupe alkyle en C₁-C₃ substitué ;
Y et Z sont des substituants indépendants sélectionnés dans le groupe consistant en H, un atome d'halogène, CN, NO₂, un groupe alcoxy en C₁-C₃, un groupe alkyle en C₁-C₃, et un groupe thioalcoxy en C₁-C₃ ;
où pas tous les groupes X, Y et Z sont H ; ou
(ii) un cycle de 5 ou 6 éléments ayant dans son squelette 1, 2 ou 3 hétéroatomes sélectionnés dans le groupe consistant en O, S, SO, SO₂ et NR⁶ et contenant un
ou deux substituants indépendants sélectionnés dans le groupe consistant en H, un atome d'halogène, CN, NO₂, un groupe alkyle en C₁-C₃, et un groupe alcoxy en C₁-C₃ ;
R⁶ est H ou un groupe alkyle en C₁-C₃;
Q¹ est S, NR⁷ ou CR⁸R⁹ ;
R⁷ est sélectionné dans le groupe consistant en CN, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, et SO₂CF₃ ;
R⁸ et R⁹ sont des substituants indépendants sélectionnés dans le groupe consistant en H, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, NO₂, CN et CO₂R¹⁰ ;
R¹⁰ est un groupe alkyle en C₁-C₃ ;
ou CR⁸R⁹ est un cycle de 6 éléments comme illustré par la structure ci-dessous : 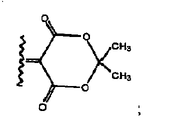
(III) un composé de formule : 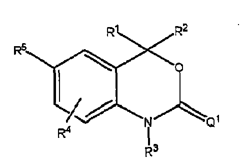 dans laquelle
R¹ et R² sont indépendamment sélectionnés dans le groupe consistant en un groupe alkyle en C₁-C₃ et un groupe alkyle en C₁-C₃ substitué, ou R¹ et R² sont fusionnés pour former un cycle spirocyclique saturé de 3 à 6 éléments à base d'atome de carbone ;
R³ est H, OH, NH₂, un groupe alkyle en C₁-C₆, un groupe alkyle en C₁-C₆ substitué, ou COR^{c};
R^{c} est H, un groupe alkyle en C₁-C₄, ou un groupe alcoxy en C₁-C₃ ;
R⁴ est H, un atome d'halogène, NO₂, un groupe alkyle en C₁-C₃, ou un groupe alkyle en C₁-C₃ substitué ;
R⁵ est (i) ou (ii) :
(i) un cycle benzène substitué de la structure : 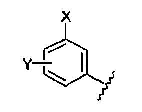 dans laquelle
X est sélectionné dans le groupe consistant en un atome d'halogène, CN, un groupe alcoxy en C₁-C₃, un groupe alkyle en C₁-C₃, NO₂, un groupe perfluoroalkyle en C₁-C₃, un cycle hétérocyclique de 5 éléments contenant dans son squelette 1 à 3 hétéroatomes, et un groupe thioalcoxy en C₁-C₃ ;
Y est sélectionné dans le groupe consistant en H, un atome d'halogène, CN, NO₂, un groupe alcoxy en C₁-C₃, un groupe alkyle en C₁-C₄, et un groupe thioalcoxy en C₁-C₃ ; ou
(ii) un cycle de 5 éléments de la structure : 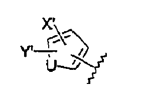
dans laquelle
X' est sélectionné dans le groupe consistant en un atome d'halogène, CN, un groupe alcoxy en C₁-C₃, un groupe alkyle en C₁-C₃, et NO₂;
Y' est sélectionné dans le groupe consistant en H, un atome d'halogène, CN, NO₂, un groupe alcoxy en Ci-C₃, un groupe alkyle en C₁-C₄, et un groupe thioalcoxy en C₁-C₃ ;
U est O, S ou NR⁶ ;
R⁶ est H, un groupe alkyle en C₁-C₃ ou un groupe CO₂-alkyle en C₁-C₄ ;
Q¹ est S, NR⁷ ou CR⁸R⁹ ;
R⁷ est sélectionné dans le groupe consistant en CN, un groupe alkyle en C₁-C₆, un groupe alkyle en C₁-C₆ substitué, un groupe cycloalkyle en C₃-C_{B}, un groupe cycloalkyle en C₃-C₈ substitué, un groupe aryle, un groupe aryle substitué, un groupe hétérocyclique, un groupe hétérocyclique substitué, et SO₂CF₃ ;
R⁸ et R⁹ sont des substituants indépendants sélectionnés dans le groupe consistant en H, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, NO₂, CN et CO₂R¹⁰ ;
R¹⁰ est un groupe alkyle en C₁-C₃ ;
ou CR⁸R⁹ est un cycle de 6 éléments comme illustré par la structure ci-dessous : 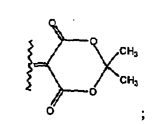
(IV) un composé de formule : 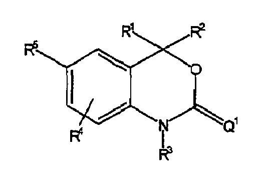 dans laquelle
R¹ et R² sont des substituants indépendants sélectionnés dans le groupe consistant en un groupe alkyle en C₁-C₃ et un groupe alkyle en C₁-C₃ substitué, ou R¹ et R² sont fusionnés pour former un cycle spirocyclique saturé de 3 à 6 éléments à base d'atome de carbone ;
R³ est H ;
R⁴ est H, un atome d'halogène, NO₂, un groupe alkyle en C₁-C₃, ou un groupe alkyle en C₁-C₃ substitué ;
R⁵ est le cycle de 6 éléments de la structure : 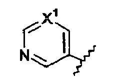 dans laquelle
X¹ est N ; et
Q¹ est S, NR⁷ ou CR⁸R⁹ ; et
à condition que pour chacun des composés (I) à (IV), lorsque Q¹ est S et R³ est H, R¹ et R² ne soient pas tous les deux H ;
à condition que pour chacun des composés (I) à (IV), lorsque Q¹ est S et R³ est H, R⁵ soit un cycle benzène (i) substitué par un ou deux substituant ou R⁵ soit un cycle de 5 ou 6 éléments (ii) ayant dans son squelette, 1, 2, ou 3 hétéroatomes sélectionnés dans le groupe consistant en O, S et NR⁶, et un de R¹ ou R² soit un groupe alcényle en C₂-C₄, un groupe alcényle en C₂-C₄ substitué, un groupe alcynyle en C₂-C₄, ou un groupe alcynyle en C₂-C₄ substitué, l'autre de R¹ ou R² ne soit pas un groupe alkyle en C₁-C₆, un groupe alkyle en C₁-C₆ substitué, un groupe cycloalkyle en C₃-C₅, un groupe cycloalkyle en C₃-C₅ substitué, un groupe alcényle en C₂-C₄, un groupe alcényle en C₂-C₄ substitué, un groupe alcynyle en C₂-C₄, ou un groupe alcynyle en C₂-C₄ substitué ;
à condition que pour chacun des composés (I) à (IV), lorsque Q¹ est S ;R³ est H, R⁵ soit un cycle benzène (i) substitué par un ou deux substituant ou R⁵ soit un cycle de 5 ou 6 éléments (ii) ayant dans son squelette, 1, 2, ou 3 hétéroatomes sélectionnés dans le groupe consistant en O, S et NR⁶, et un de R¹ ou R² soit un groupe alkyle en C₁-C₆ ou un groupe alkyle en C₁-C₆ substitué, l'autre de R¹ ou R² ne soit pas un groupe cycloalkyle en C₃-C₈ ou un groupe cycloalkyle en C₃-C₈ substitué ;
à condition que pour chacun des composés (I) à (IV), le terme « alkyle substitué » n'incluse pas un groupe alkyle perfluoré ;
(V) la 6-[3-fluoro-5-(trifluorométhylphényl]-4,4-diméthyl-1,4-dihydro-2H-3,1-benzoxazine-2-thione ;
ou un sel pharmaceutiquement acceptable de celle-ci ;
(VI) la 6-(3-bromo-5-trifluorométhoxyphényl)-4,4-diméthyl-1,4-dihydro-2H-3,1-bezoxazine-2-thione.

2. Composé selon la revendication 1, dans lequel:
R¹ est H, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, COR^{A} ou NR^{B}COR^{A} ;
R² est H, un groupe alkyle en C₁-C₆, un groupe alkyle en C₁-C₆ substitué, un groupe alcényle en C₂-C₆, un groupe alcényle en C₂-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, COR^{A}
ou NR^{B}COR^{A} ;
ou R¹ et R² sont fusionnés pour former un cycle sélectionné dans le groupe consistant en a), b) et c) :
a) un cycle spirocyclique saturé de 3 à 6 éléments à base d'atome de carbone ;
b) un cycle spirocyclique de 3 à 6 éléments à base d'atome de carbone ayant dans son squelette une ou plusieurs doubles liaisons carbone-carbone ; et
c) un cycle spirocyclique de 3 à 6 éléments contenant dans son squelette un à trois hétéroatomes ;
R^{A} est H, un groupe alkyle en C₁-C₃, un groupe alkyle en C₁-C₃ substitué, un groupe aryle, un groupe aryle substitué, un groupe alcoxy en C₁-C₃, un groupe alcoxy en C₁-C₃ substitué, un groupe aminoalkyle en C₁-C₃, ou un groupe aminoalkyle en C₁-C₃ substitué ;
R⁵ est un cycle de 5 ou 6 éléments, dans lequel ledit ou lesdits deux substituants indépendants sont sélectionnés dans le groupe consistant en H, un atome d'halogène, CN, NO₂, un groupe alkyle en C₁-C₃, et un groupe alcoxy en C₁-C₃ ;
R⁷ est sélectionné dans le groupe consistant en CN, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, et SO₂CF₃.

3. Composé selon la revendication 1, dans lequel:
R¹ et R² sont indépendamment sélectionnés dans le groupe consistant en un groupe alkyle en C₁-C₃ et un groupe alkyle en C₁-C₃ substitué, ou R¹ et R² sont fusionnés pour former un cycle spirocyclique saturé de 3 à 6 éléments à base d'atome de carbone ;
R³ est H, OH, NH₂, un groupe alkyle en C₁-C₆, un groupe alkyle en C₁-C₆ substitué, ou COR^{c} ;
R^{c} est H, un groupe alkyle en C₁-C₃, ou un groupe alcoxy en C₁-C₃ ;
R⁴ est H, un atome d'halogène, NO₂, un groupe alkyle en C₁-C₃, ou un groupe alkyle en C₁-C₃ substitué;
R⁵ est un cycle benzène substitué de la structure : 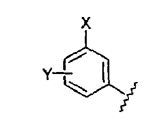
dans laquelle
X est sélectionné dans le groupe consistant en un atome d'halogène, CN, un groupe alcoxy en C₁-C₃, un groupe alkyle en C₁-C₃, NO₂, un groupe perfluoroalkyle en C₁-C₃, un cycle hétérocyclique de 5 éléments contenant dans son squelette 1 à 3 hétéroatomes, et un groupe thioalcoxy en C₁-C₃ ;
Y est sélectionné dans le groupe consistant en H, un atome d'halogène, CN, NO₂, un groupe alcoxy en C₁-C₃, un groupe alkyle en C₁-C₄, et un groupe thioalcoxy en C₁-C₃ ;
R⁷ est sélectionné dans le groupe consistant en CN, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, et SO₂CF₃ ;

4. Composé selon la revendication 1, dans lequel :
R¹ et R² sont indépendamment sélectionnés dans le groupe consistant en un groupe alkyle en C₁-C₃ et un groupe alkyle en C₁-C₃ substitué, ou R¹ et R² sont fusionnés pour former un cycle spirocyclique saturé de 3 à 6 éléments à base d'atome de carbone ;
R³ est H ;
R^{c} est H, un groupe alkyle en C₁-C₃, ou un groupe alcoxy en C₁-C₃ ;
R⁴ est H, un atome d'halogène. NO₂, un groupe alkyle en C₁-C₃, ou un groupe alkyle en C₁-C₃ substitué ;
R⁵ est un cycle de 5 éléments de la structure : 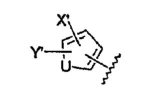
dans laquelle
X' est sélectionné dans le groupe consistant en un atome d'halogène, CN, un groupe alcoxy en C₁-C₃, un groupe alkyle en C₁-C₃, NO₂, un groupe perfluoroalkyle en C₁-C₃, un cycle hétérocyclique de 5 éléments contenant dans son squelette 1 à 3 hétéroatomes, et un groupe thioalcoxy en C₁-C₃ ;
Y' est sélectionné dans le groupe consistant en H, un atome d'halogène, CN, NO₂, un groupe alcoxy en C₁-C₃, un groupe alkyle en C₁-C₃, et un groupe thioalcoxy en C₁-C₃ ;
U est 0, S ou NR⁶ ;
R⁷ est sélectionné dans le groupe consistant en CN, un groupe alkyle en C₁-C₆, un groupe alkyle en C₁-C₆ substitué, un groupe cycloalkyle en C₃-C₈, un groupe cycloalkyle en C₃-C₈ substitué, un groupe aryle, un groupe aryle substitué, un groupe hétérocyclique, un groupe hétérocyclique substitué, et SO₂CF₃.

5. Composé selon la revendication 1, dans lequel :
R¹ et R² sont indépendamment sélectionnés dans le groupe consistant en un groupe alkyle en C₁-C₃ et un groupe alkyle en C₁-C₃ substitué, ou R¹ et R² sont fusionnés pour former un cycle spirocyclique saturé de 3 à 6 éléments à base d'atome de carbone ;
R³ est H ;
R⁴ est H, un atome d'halogène, NO₂, un groupe alkyle en C₁-C₃, ou un groupe alkyle en C₁-C₃ substitué ;
R⁵ est un cycle de 6 éléments de la structure : 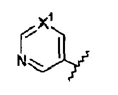
dans laquelle
X¹, est CX² ;
X² est un atome d'halogène, CN, ou NO₂.

6. Composé selon la revendication 1, qui est la 6-(3-chlorophényl)-4,4-diméthyl-1,4-dikxydro-benzo[d] [1,3]oxazin-2-thione, le 4-(4,4-diméthyl-2-thioxo-1,4-dihydro-2H-benzo[d][1,3]oxazin-6-yl)-thiophène-2-cabonitrile, le 3-(4,4-diméthyl-2-thioxo-1,4-dihydro-2H-benzol[d][1,3]oxazin-6-yl)-5-fluorobenzonitrile, le 3-(4,4-diméthyl-2-thioxo-1,4-dihydro-2H-benzo[d][1,3]-oxazin-6-yl)-benzonitrile, le 5-(4,4-diméthyl-2-thioxo-1,4-dihydro-2H-3,1-benzoxazin-6-yl)-1-méthyl-1H-pyrrole-2-carbonitrile, la 6-(3-fluorophényl)-4-méthyl-1,4-dihydro-2H-3,1-benzoxazin-2-thione, le 5-(4,4-diméthyl-2-thioxo-1,4-dihydro-2H-3,1-benzoxazin-6-yl)-4-méthylthiophène-2-carbonitrile, le 5-(4,4-diméthyl-2-thioxo-1,4-dihydro-2H-3,1-benzoxazin-6-yl)-1H-pyrrole-2-carbonitrile, le [6-(4,4-diméthyl-2-thioxo-1,4-dihydro-2H-3,1-benzoxazin-6-yl)pyridin-2-yl]acétonitrile, le 5-(4,4-diméthyl-2-thioxo-1,4-dihydro-2H-3,1-benzoxazin-6-yl)-1H-pyrrole-2-carbothiamide, le 5-(4,4-diméthyl-2-thioxo-1,4-dihydro-2H-benzo[d][1,3]oxazin-6-yl)-thiophène-3-carbonitrile, le 5-(4,4-diméthyl-2-thioxo-1,4-dihydro-2H-3,1-benzoxazin-6-yl)-1-éthyl-1H-pyrrole-2-carbonitrile, le 4-(1,2-dihydro-2-thioxospiro[4H-3,1-benzoxazin-4,1-cyclohexan]-6-yl)-2-thiophènecarbonitrile, le 5-(4,4-diméthyl-2-thioxo-1,4-dihydro-2H-3,1-benzo-xazin-6-yl)-2-fluorobenzonitrile, la 6-(5-bromopyridin-3-yl)-4.4-diméthyl-1,4-dihydro-2H-3,1-benzoxazine-2-thione, la 6-(3-chloro-5-fluorophényl)-4,4-diméthyl-1,4-dihydro-2H-3,1-benzoxazine-2-thione, la 6-(3-bromo-5-méthylphényl)-4,4-diméthyl-1,4-dihydro-2H-3,1-benzoxazine-2-thione, le 3-(1,2-dihydro-2-thioxospiro[4H-3,1-benzoxazin-4,1-cyclohexan]-6-yl)-5-fluorobenzonitrile, le 3-(4,4-diméthyl-2-thioxo-1,4-dihydro-2H-3,1-benzo-xazin-6-yl)-5-méthylbenzonitrile, la 6-(3,5-dichlorophényl)-4,4-diméthyl-1,4-dihydro-2H-3,1-benzoxazine-2-thione, le 5-(4,4-diméthyl-1,2-thioxo-1,4-dihydro-2H-3,1-benzoxazin-6-yl)isophtalonitrile, le 5-(4,4-diméthyl-2-thioxo-1,4-dihydro-2H-3,1-benzoxazin-6-yl)-2-furonitrile, la 4,4-diéthyl-6-(3-nitrophényl)-1,4-dihydro-2H-3,1-benzoxazine-2-thione, la 6-(3-chlorophényl)-4-méthyl-4-phényl-1,4-dihydro-2H-3,1-benzoxazine-2-thione, la 4-allyl-6-(3-chlorophényl)-4-méthyl-1,4-dihydro-2H-3,1-benzoxazine-2-thione, le 3-chloro-5-(4,4-diméthyl-2-thioxo-1,4-dihydro-2H-3,1-benzoxazin-6-yl) benzonitrile, la 6-(3,5-difluorophényl)-4,4-diméthyl-1,4-dihydro-2H-3,1-benzoxazine-2-thione, la 6-(3-fluoro-5-méthoxyphényl)-4,4-diméthyl-1,4-dihydro-2H-3,1-benzoxazine-2-thione, le 3-(4,4-diméthyl-2-thioxo-1,4-dihydro-2H-3,1-benzoxazin-6-yl)-5-méthoxybenzonitrile, la 6-(3-fluorophényl)-4,4-diméthyl-1,4-dihydro-2H-3,1-benzoxazine-2-thione, la 6-(2-fluorophényl)-4,4-diméthyl-1,4-dihydro-2H-3,1-benzoxazine-2-thione, la 6-(3,4-difluorophényl)-4,4-diméthyl-1,4-dihydra-2H-3,1-benzoxazine-2-thione, la 6-(4-fluorophényl)-4,4-diméthyl-1,4-dihydro-2H-3,1-benzoxazine-2-thione, le 3-(4,4-diméthyl-2-thioxo-1,4-dihydro-2H-3,1-benzoxazin-6-yl)-4-fluorobenzonitrile, la 6-(2,3-difluoraphényl)-4,4-climéthyl-1,4-dihydro-2H-3,1-benzoxazine-2-thione, le 3-(8-bromo-4,4-diméthyl-2-thioxo-1,4-dihydro-2H-3,1-benzoxazin-6-yl)-5-fluorobenzonitrile, la 4,4-diméthyl-6-(3-nitrophényl)-1,4-dihydro-2H-3,1-benzoxazine-2-thione, la 6-(3-chlorophényl)-4,4-diéthyl-1,4-dihydro-2H-3,1-benzoxazine-2-thione, la 6-(3-méthoxyphényl)-4,4-diméthyl-1,4-dihydro-2H-3,1-benzoxazine-2-thione, 1a 6-(2-chlorophényl)-4,4-diméthyl-1,4-dihydro-2H-3,1-benzoxazine-2-thione, la 4-benzyl-6-(3-chlorophényl)-4-méthyl-1,4-dihydro-2H-3,1-benzoxazine-2-thione, la 6-(3-bromo-5-fluorophényl)-4,4-diméthyl-1,4-dihydro-2H-3,1-benzoxazine-2-thione, le 5-(4,4-diméthyl-2-thioxo-1,4-dihydro-2H-3,1-benzoxazin-6-yl)thiophène-2-carbonitrile, le 3-fluoro-5-(8-fluoro-4,4-diméthyl-2-thioxo-1,4-dihydro-2H-3,1-benzoxazin-6-yl)benzonitrile, le 3-(1,2-dihydro-2-thioxospiro[4H-3,1-benzoxazin-4,1-cyclohexan]-6-yl)benzonitrile, le 5-(1,2-dihydro-2-thioxospiro[4H-3,1-benzo-xazin-4,1-cyclohexan]-6-yl)-4-méthyl-2-thiophène-carbonitrile, le 5-(1,2-dihydro-2-thioxo-spiro[4H-3,1-benzoxazin-4,1-cyclohexan]-6-yl)-2-thiophènecarbonitrile, la 6-(3-chloro-4-fluorophényl)-4,4-diméthyl-1,4-dihydro-2H-3,1-benzoxazine-2-thione, le 5-(4,4-diméthyl-2-thioxo-1,4-dihydro-2H-3,1-benzoxazin-6-yl)-4-propylthiophène-2-carbonitrile, le 4-(4,4-diméthyl-2-thioxo-1,4-dihydro-2H-3,1-benzoxazin-6-yl)-2-furonitrile, la 6-(3-bromophényl)-4,4-diméthyl-1,4-dihydro-2H-3,1-benzoxazine-2-thione, ou le 2-(4,4-diméthyl-2-thioxo-1,4-dihydro-2H-3,1-benzoxazin-6-yl)-thiophène-3-carbonitrile, ou des sels pharmaceutiquement acceptables de ceux-ci.

7. Composé selon la revendication 1, qui est le 4-butyl-5-(4,4-diméthyl-2-thioxo-1,4-dihydro-2H-3,1-benzoxazin-6-yl)thiophène-2-carbonitrile, le 2-cyano-5-(4,4-diméthyl-2-thioxo-1,4-dihydro-2H-3,1-benzoxazin-6-yl)-1H-pyrrole-1-carboxylate de tert-butyle, ou des sels pharmaceutiquement acceptables de ceux-ci.

8. Composition pharmaceutique comprenant une quantité pharmaceutiquement efficace d'un composé selon l'une quelconque des revendications 1 à 7 et d'un support ou d'un excipient pharmaceutiquement acceptable.

9. Utilisation d'un composé selon l'une quelconque des revendications 1 à 7, ou dudit sel acceptable de celui-ci, pour la préparation d'un médicament.

10. Utilisation d'un composé selon l'une quelconque des revendications 1 à 7, ou dudit sel acceptable de celui-ci, pour la préparation d'un médicament utile pour l'administration à un sujet mammifère en tant que contraceptif.

11. Utilisation d'un composé selon l'une quelconque des revendications 1 à 7, ou dudit sel acceptable de celui-ci, pour la préparation d'un médicament utile pour l'administration à un sujet mammifère pour le traitement de carcinomes ou d'adénocarcinomes des ovaires de l'endomètre, du sein, du colon ou de la prostate.

12. Utilisation d'un composé selon l'une quelconque des revendications 1 à 7, ou dudit sel acceptable de celui-ci, pour la préparation d'un médicament utile pour l'administration à un sujet mammifère pour le traitement d'un saignement dysfonctionnel, d'un léiomyome utérin, d'une endométriose, d'un syndrome des ovaires polykystiques ou de fibromes.

13. Utilisation d'un composé selon l'une quelconque des revendications 1 à 7, ou dudit sel acceptable de celui-ci, pour la préparation d'un médicament utile pour l'administration à un sujet mammifère pour une hormonothérapie substitutive.
